(19) Europäisches Patentamt
European Patent Office
Office européen des brevets

(11) Veröffentlichungsnummer: **0 020 304**
**A1**

(12) # EUROPÄISCHE PATENTANMELDUNG

(21) Anmeldenummer: **80810172.9**

(22) Anmeldetag: **23.05.80**

(51) Int. Cl.³: **C 07 D 521/00,** A 61 K 31/395 // C07D233/46, C07D277/40, C07D211/16, C07D295/20, (C07D521/00, 279/12, 265/30, 263/28, 277/48, 277/18, 285/12, 263/48, 233/88, 417/12, 233/44, 241/06, 271/10, 261/04)

(30) Priorität: **29.05.79 CH 4994/79**
**29.05.79 CH 4995/79**

(43) Veröffentlichungstag der Anmeldung: **10.12.80**
**Patentblatt 80/25**

(84) Benannte Vertragsstaaten: **AT BE CH DE FR GB IT LI LU NL SE**

(71) Anmelder: **CIBA-GEIGY AG, Patentabteilung Postfach, CH-4002 Basel (CH)**

(72) Erfinder: **Dave, Krishna G., Dr., CIBA-GEIGY Research Centre Housing Colony, Aarey Road Goregaon Bombay 400063 (IN)**
Erfinder: **George, Thomas, Dr., CIBA-GEIGY Research Centre Housing Colony, Aarey Road Goregaon Bombay 400063 (IN)**
Erfinder: **Viswanathan, Narayana I., Dr., CIBA-GEIGY Research Centre Housing Colony, Aarey Road Goregaon Bombay 400063 (IN)**
Erfinder: **Ilvespää, Atso, Dr., Spitzwaldstrasse 154, CH-4123 Allschwil (CH)**
Erfinder: **Frei, Jörg, Dr., Buechring 36, CH-4434 Hölstein (CH)**
Erfinder: **Schweizer, Ernst, Dr., Hollenweg 59, CH-4144 Arlesheim (CH)**

(54) **Guanidine, Verfahren zur Herstellung, pharmazeutische Präparate enthaltend solche Verbindungen und ihre Verwendung.**

(57) Die Erfindung betrifft neue Guanidinderivate, insbesondere substituierte Guanidine der Formel

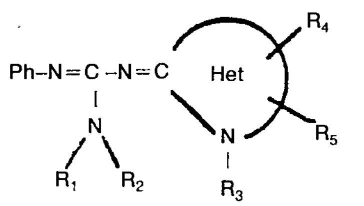

(I)

mit hypoglykämischen Wirksamkeiten zur oralen Behandlung von Hyperglykämie bei Säugetieren, insbesondere von Diabetes mellitus.

EP 0 020 304 A1

0020304

CIBA-GEIGY AG
Basel (Schweiz)

4-12312/12313/+

Guanidine, Verfahren zur Herstellung, pharmazeutische Präparate enthaltend solche Verbindungen und ihre Verwendung.

Die Erfindung betrifft neue Guanidinderivate, ferner Verfahren zu deren Herstellung sowie pharmazeutische Präparate, die diese neuen Verbindungen enthalten, wie auch ihre Verwendung.

Die Erfindung betrifft neue Guanidinderivate, insbesondere heterocyclisch substituierte Guanidine der Formel

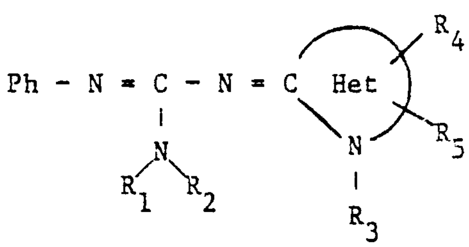

(I)

worin $R_1$ ein gegebenenfalls substituierter aliphatischer oder cycloaliphatischer Kohlenwasserstoffrest, $R_2$ Wasserstoff, ein gegebenenfalls substituierter aliphatischer oder cycloaliphatischer Kohlenwasserstoffrest ist oder $R_1$ und $R_2$ zusammengenommen einen gegebenenfalls substituierten bivalenten Kohlenwasserstoffrest aliphatischen Charakters, in welchem die Kohlenstoffatome der Kette durch ein Heteroatom unterbrochen sein können, $R_3$ Wasserstoff, Niederalkyl, $R_4$ Wasserstoff, Niederalkyl, Niederalkoxy, Niederalkylthio, Niederalkylamino, Diniederalkylamino, Halogen, Trifluormethyl oder gegebenenfalls substituiertes Phenyl, oder an einem beidseitig einfach gebundenen Kohlenstoffatom auch Oxo, $R_5$ Wasserstoff oder Niederalkyl, Het einen Heteroalkylenrest mit 3 Kettengliedern, der die Gruppe C-N zu einem gegebenenfalls ungesättigten heterocyclischen Fünfring mit 2 bis 3 Heteroatomen aus der Gruppe Sauerstoff, Schwefel

oder Stickstoff im Ring ergänzt, bzw. Het einen Heteroalkylenrest mit 4-6 Kettengliedern, der die Gruppe C-N zu einem heterocyclischen Sechs- bis Achtring, der neben dem Stickstoffatom noch ein Heteroatom aus der Gruppe Sauerstoff, Schwefel oder Stickstoff im Ring enthält, ergänzt, und Ph einen gegebenenfalls substituierten Phenylrest bedeuten, ihre tautomeren Verbindungen und Salze.

Im Zusammenhang mit der vorliegenden Beschreibung enthalten die mit "nieder" bezeichneten Reste und Verbindungen vorzugsweise bis zu 7, in erster Linie bis zu 4 Kohlenstoffatome.

Ein aliphatischer Kohlenwasserstoffrest $R_1$ oder $R_2$, der gegebenenfalls substituiert sein kann, ist in erster Linie ein Alkyl- sowie ein Alkenyl- oder Alkinylrest, insbesondere ein Niederalkyl- sowie Niederalkenyl- oder Niederalkinylrest. Substituenten von aliphatischen Kohlenwasserstoffresten sind z.B. freie, veresterte oder verätherte Hydroxygruppen, wie Niederalkanoyloxy-, Niederalkoxy- oder Niederalkenyloxygruppen, oder Halogenatome, sowie gegebenenfalls veresterte Carboxygruppen, wie Niederalkoxycarbonyl.

Vorstehend wie nachstehend können die Allgemeinbegriffe folgende Bedeutung haben:

Niederalkylgruppen sind z.B. vorzugsweise Methyl- sowie Aethyl-, n-Propyl-, Isopropyl-, n-Butyl-, Isobutyl-, sek.-Butyl-, tert.-Butyl-, n-Pentyl-, Isopentyl-, Neopentyl-, n-Hexyl-, Isohexyl- oder n-Heptylgruppen; Niederalkenylgruppen sind z.B. die Allyl- oder die 2-Methylallylgruppe, und Niederalkinylgruppen vorzugsweise Propargylgruppen. Substituierte Niederalkylgruppen sind beispielsweise die Trifluormethylgruppe oder eine gegebenenfalls veresterte Carboxymethylgruppe, wie z.B. eine Niederalkoxycarbonylmethyl-, z.B. Methoxycarbonylmethylgruppe.

Niederalkoxy ist z.B. Methoxy, Aethoxy, n-Propoxy, Isopropoxy, n-Butoxy oder n-Pentyloxy, und Niederalkenyloxy z.B. Vinyloxy oder Allyloxy.
Niederalkanoyloxy ist z.B. Acetoxy oder Propionyloxy.

Halogenatome sind in erster Linie Fluor-, Chlor- oder Bromatome, können aber auch Jodatome sein.

Ein cycloaliphatischer Kohlenwasserstoffrest ist in erster Linie ein mono-, sowie polycyclischer Cycloalkylrest mit z.B. bis zu 12 inkl., vorzugsweise 3 bis 10 Ringkohlenstoffatomen.

Eine Cycloalkylgruppe ist z.B. eine Cyclopropyl-, Cyclobutyl-, Cyclopentyl-, Cyclohexyl-, Cycloheptyl- oder Adamantylgruppe.

Ein gegebenenfalls substituierter Phenylrest kann durch einen, zwei oder drei gleiche oder verschiedene Substituenten substituiert sein. Solche Substituenten sind z.B. Kohlenwasserstoffreste, wie niederaliphatische Kohlenwasserstoffreste, z.B. Niederalkyl, substituiertes Niederalkyl wie z.B. Trifluormethyl, gegebenenfalls funktionell abgewandeltes Hydroxy oder Mercapto, wie veräthertes Hydroxy, z.B. Niederalkoxy, Niederalkenyloxy oder Niederalkylendioxy, ferner Niederalkylthio, oder Halogen, Nitro, Amino einschliesslich substituiertes Amino, z.B. Niederalkylamino oder Di-niederalkylamino, gegebenenfalls funktionell abgewandeltes Carboxy, wie verestertes Carboxy, z.B. Niederalkoxycarbonyl.

Niederalkylthio ist insbesondere Methylthio, ferner auch Aethylthio, Isopropylthio, n-Propylthio oder auch gerades oder verzweigtes Butylthio. Niederalkylamino oder Diniederalkylamino ist z.B. Methylamino, Dimethylamino, Aethylamino, Diäthylamino, n-Propylamino, Di-n-propylamino, Isopropylamino, Di-isopropylamino oder n-Butylamino oder Di-n-butylamino.

Die beiden Substituenten $R_1$ und $R_2$ zusammengenommen können einen gegebenenfalls substituierten bivalenten aliphatischen Kohlenwasserstoffrest mit 4-7 Kohlenstoffatomen in der Kette bedeuten. Die Gruppe $-NR_1R_2$ ist beispielsweise ein gerades, verzweigtes oder durch Phenyl substituiertes Niederalkylenamino, in der die Niederalkylenkette beispielsweise durch ein Heteroatom, wie z.B. Sauerstoff, Schwefel oder gegebenenfalls durch Niederalkyl, Phenyl oder Niederalkoxycarbonyl substituierten Stickstoff, unterbrochen sein kann, und stellt beispielsweise als Niederalkylenamino Pyrrolidino, 2,5-Dimethylpyrrolidino, Piperidino, 2-Methylpiperidino, 4-Phenylpiperidino, Hexahydroazepino oder Octahydroazocino, als Oxaniederalkylenamino beispielsweise Morpholino, 2,6-Dimethylmorpholino als Thianiederalkylenamino beispielsweise Thiomorpholino oder als Azaniederalkylenamino z.B. Piperazino, N-Methyl-, N-Phenyl- oder N-Niederalkoxycarbonylpiperazino dar.

Der Heteroalkylenrest Het mit 3 Kettengliedern, der die Gruppe C-N zusammen zu einem gegebenenfalls ungesättigten heterocyclischen Fünfring mit 2-3 Heteroatomen aus der Gruppe Sauerstoff, Schwefel oder Stickstoff im Ring ergänzt, bildet mit C-N zusammen beispielsweise einen Imidazolin-, Imidazolidin-, Oxazolin-, Oxazolidin-, Thiazolin-, Thiazolidin-, Isoxazolin-, Isoxazolidin-, Isothiazolin-, Isothiazolidin-, 1,2,4-Oxadiazolin-, 1,2,4-Oxadiazolidin-, 1,3,4-Oxadiazolin-, 1,3,4-Oxadiazolidin, 1,2,4-Thiazolin-, 1,2,4-Thiazolidin-, 1,3,4-Thiazolin-, 1,3,4-Thiazolidin-, Pyrazolin-, Pyrazolidin-, 1,2,3-Triazolin-, 1,2,3-Triazolidin-, 1,2,4-Triazolin- oder auch 1,2,4-Triazolidinring.

Insbesondere zu nennen sind der 4-Imidazolin-, Imidazolidin-, 4-Oxazolin-, Oxazolidin-, 4-Thiazolin-, Thiazolidin-, 4-(1,3,4)-Thiadiazolin-, 4-(1,3,4)-Oxadiazolin-, Isoxazolin- oder auch der Isoxazolidinring.

Der Heteroalkylenrest Het mit 4-6 Kettengliedern, der die Gruppe C-N zu einem heterocyclischen Sechs- bis Achtring, der neben dem Stickstoffatom noch ein Heteroatom aus der Gruppe Sauerstoff, Schwefel oder Stickstoff im Ring enthält, ergänzt, bildet beispielsweise einen Morpholin-, Thiomorpholin-, 2H-3,4,5,6-Tetrahydro-1,3-thiazin-, 2H-3,4,5,6-Tetrahydropyrimidin-, Piperazin-, Hexahydro-1,3-thiazepin-, Hexahydro-1,4-thiazepin-, Hexahydro-1,3-thiazocin- oder auch Hexahydro-1,4-thiazocinring. Die beiden letzteren Ringsysteme können in 2- oder 4- bzw. 3- oder 5-Stellung gebunden sein.

Insbesondere zu nennen sind der Morpholin-, Thiomorpholin-, Piperazin-, 2H-3,4,5,6-Tetrahydro-1,3-thiazin- oder auch Hexahydro-1,4-thiazepinring.

Die neuen Verbindungen der allgemeinen Formel I und ihre Additionssalze mit anorganischen oder organischen Säuren besitzen wertvolle pharmakologische Eigenschaften, insbesondere hypoglykämische Wirksamkeit, wie sich an Stoffwechsel-normalen Ratten nach oraler Verabreichung von Dosen ab 3 mg/kg sowie auch an Ratten, die durch Injektion von Streptozotocin in eine Diabetes-ähnliche Stoffwechsellage versetzt wurden [vgl. A. Junod et al., Proc. Soc. Exp. Biol. Med. 126, 201-205 (1967)], nachweisen lässt. Die Senkung des Blutzuckerspiegels ist nicht von einer Hyperlactatämie begleitet. Die pharmakologischen Befunde charakterisieren die neuen Verbindungen der allgemeinen Formel I und ihre pharmazeutisch annehmbaren Säureadditionssalze als Antidiabetica, die zur oralen Behandlung von Hyperglykämie bei Säugetieren, insbesondere von Diabetes mellitus, verwendet werden können.

Besonders zu erwähnen sind Verbindungen der Formel I

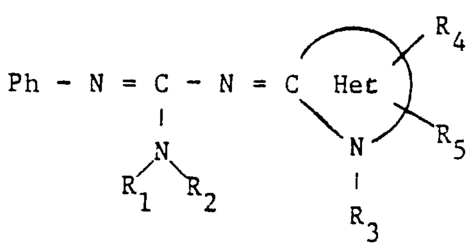

(I)

worin $R_1$ ein gegebenenfalls substituierter aliphatischer oder cycloaliphatischer Kohlenwasserstoffrest, $R_2$ Wasserstoff oder ein gegebenenfalls substituierter aliphatischer Kohlenwasserstoffrest ist oder $R_1$ und $R_2$ zusammengenommen einen gegebenenfalls substituierten bivalenten Kohlenwasserstoffrest aliphatischen Charakters, in welchem die Kohlenstoffatome der Kette durch ein Heteroatom unterbrochen sein

können, $R_3$ Wasserstoff, Niederalkyl, $R_4$ Wasserstoff, Niederalkyl, Niederalkoxy, Niederalkylthio, Niederalkylamino, Diniederalkylamino, Halogen, Trifluormethyl oder gegebenenfalls substituiertes Phenyl, oder an einem beidseitig einfach gebundenen Kohlenstoffatom auch Oxo, $R_5$ Wasserstoff oder Niederalkyl, Het einen Heteroalkylenrest mit 3 Kettengliedern, der die Gruppe C-N zu einem gegebenenfalls ungesättigten heterocyclischen Fünfring mit 2 bis 3 Heteroatomen aus der Gruppe Sauerstoff, Schwefel oder Stickstoff im Ring ergänzt, und Ph einen gegebenenfalls substituierten Phenylrest bedeuten, ihre tautomeren Verbindungen und Salze.

Die Erfindung betrifft in erster Linie Verbindungen der Formel I, in der $R_1$ Niederalkyl oder Cycloalkyl, $R_2$ Wasserstoff oder Niederalkyl oder beide zusammengenommen eine Niederalkylenkette, die gegebenenfalls durch ein Sauerstoff-, Schwefel- oder gegebenenfalls durch z.B. Niederalkyl oder Phenyl substituiertes Stickstoffatom unterbrochen sein kann, $R_3$ Wasserstoff oder Niederalkyl, $R_4$ Wasserstoff, Niederalkyl, Niederalkoxy, Niederalkylthio, Niederalkylamino, Diniederalkylamino, Halogen, Trifluormethyl, oder an einem beidseitig einfach gebundenen Kohlenstoffatom auch Oxo, oder gegebenenfalls durch Niederalkyl, Niederalkoxy oder Halogen substituiertes Phenyl, $R_5$ Wasserstoff oder Niederalkyl, und Het einen Heteroalkylenrest mit 3 Kettengliedern, der die Gruppe C-N zu einem gesättigten oder einfach ungesättigten heterocyclischen Fünfring mit 2-3 Heteroatomen aus der Gruppe Sauerstoff, Schwefel oder Stickstoff im Ring ergänzt, und Ph einen gegebenenfalls substituierten Phenylrest bedeuten, als auch ihre tautomeren Verbindungen und Salze.

Insbesondere betrifft die Erfindung diejenigen Verbindungen der Formel I, in der $R_1$ Niederalkyl oder Cycloalkyl, $R_2$ Wasserstoff oder Niederalkyl oder beide zusammengenommen eine Niederalkylenkette, die gegebenenfalls durch ein Sauerstoff- oder Schwefelatom oder gegebenenfalls durch z.B. Niederalkyl oder Phenyl substituiertes Stickstoffatom unterbrochen sein kann, z.B. Niederalkylenamino, z.B. Pyrrolidino, 2,5-Dimethylpyrrolidino, Piperidino, 2-Methylpiperidino, Hexahydroazepino

oder Octahydroazocino, Oxaniederalkylenamino, z.B. Morpholino, Thianiederalkylenamino, z.B. Thiomorpholino oder Azaniederalkylenamino, z.B. Piperazino, N-Methyl- oder N-Phenylpiperazino bedeuten, $R_3$ Wasserstoff oder Niederalkyl, $R_4$ Wasserstoff, Niederalkyl, Niederalkoxy, Niederalkylthio, Niederalkylamino, Diniederalkylamino, Halogen, Trifluormethyl, oder an einem beidseitig einfach gebundenen Kohlenstoffatom auch Oxo, oder gegebenenfalls durch Niederalkyl, Niederalkoxy oder Halogen substituiertes Phenyl, $R_5$ Wasserstoff oder Niederalkyl, und Het einen zweiwertigen Heteroalkylenrest mit 3-Kettengliedern, der die Gruppe C-N zu einem gesättigten oder einfach ungesättigten und beispielsweise ein Imidazolin-, Imidazolidin-, Oxazolin-, Oxazolidin-, Thiazolin-, Thiazolidin-, Isoxazolin-, Isoxazolidin-, Isothiazolin-, Isothiazolidin-, Oxadiazolin-, Oxadiazolidin-, Thiadiazolin-, Thiadiazolidin-, Pyrazolin-, Pyrazolidin- oder auch Triazolinring sein kann und Ph einen gegebenenfalls durch Niederalkyl, Niederalkoxy oder Halogen substituierten Phenylrest bedeuten, als auch ihre tautomeren Verbindungen und Salze.

Von besonderem Interesse sind Verbindungen der Formel I, in der $R_1$ Niederalkyl oder Cycloalkyl, $R_2$ Wasserstoff oder Niederalkyl oder die Gruppe $-NR_1R_2$ beispielsweise Niederalkylenamino, in der die Niederalkylenkette gegebenenfalls durch ein Sauerstoff oder Schwefelatom unterbrochen sein kann, bedeutet und beispielsweise Pyrrolidino, 2,5-Dimethylpyrrolidino, Piperidino, 2-Methylpiperidino oder auch Morpholino oder Thiomorpholino sein kann, $R_3$ Wasserstoff oder Niederalkyl, $R_4$ Wasserstoff, Niederalkyl, Niederalkoxy, Niederalkylthio, Halogen, Trifluormethyl oder an einem beidseitig einfach gebundenen Kohlenstoffatom auch Oxo, oder gegebenenfalls durch Niederalkyl, Niederalkoxy, oder Halogen substituiertes Phenyl, $R_5$ Wasserstoff oder Niederalkyl, und Het als zweiwertiger Rest mit der Gruppe C-N zusammen einen gegebenenfalls einfach ungesättigten heterocyclischen Fünfring, wie z.B. Imidazolin-, Imidazolidin-, Oxazolin-, Oxazolidin-, Thiazolin-, Thiazolidin-, Oxadiazolin-, Oxadiazolidin-, Triazolin-, Thiadiazolin- oder Thiadiazolidinring bedeutet, und Ph einen gegebenenfalls durch Niederalkyl, Niederalkoxy oder Halogen substituierten Phenylrest bedeutet, als auch ihre tautomeren Verbindungen und Salze.

Von ganz besonderem Interesse sind Verbindungen der Formel I, in der $R_1$ Niederalkyl, z.B. Methyl oder Aethyl, $R_2$ Wasserstoff oder Niederalkyl, z.B. Methyl oder Aethyl bedeutet oder die Gruppe $-NR_1R_2$ als gegebenenfalls durch ein Sauerstoff- oder Schwefelatom unterbrochenes Niederalkylenemino, beispielsweise als Pyrrolidino, Piperidino, Morpholino oder Thiomorpholino vorliegt, $R_3$ Wasserstoff oder Niederalkyl, z.B. Methyl oder Aethyl, $R_4$ Wasserstoff, Niederalkyl, z.B. Methyl oder Aethyl, Niederalkoxy, z.B. Methoxy oder Aethoxy, Niederalkylthio, z.B. Methylthio oder Aethylthio, Halogen, z.B. Chlor oder Brom, Trifluormethyl oder gegebenenfalls durch Niederalkyl, z.B. Methyl oder Aethyl, Halogen, z.B. Chlor oder Brom substituiertes Phenyl, $R_5$ Wasserstoff oder Niederalkyl, z.B. Methyl oder Aethyl und Het als zweiwertiger Heteroalkylenrest mit der Gruppe C-N einen gegebenenfalls einfach ungesättigten heterocyclischen Fünfring, wie z.B. den 4-Imidazolin-, 4-Oxazolin-, 4-Thiazolin-, 4-(1,3,4)-Thiadiazolin-, 4-(1,3,4)-Oxadiazolin- oder auch Isoxazolidinring bedeutet, und Ph einen gegebenenfalls durch Niederalkyl, wie z.B. Methyl oder Aethyl, Halogen, wie z.B. Chlor oder Brom oder Niederalkoxy, wie z.B. Methoxy oder Aethoxy, substituierten Phenylrest bedeutet, als auch ihre tautomeren Verbindungen und Salze.

Hervorzuheben sind auch Verbindungen der Formel Ib

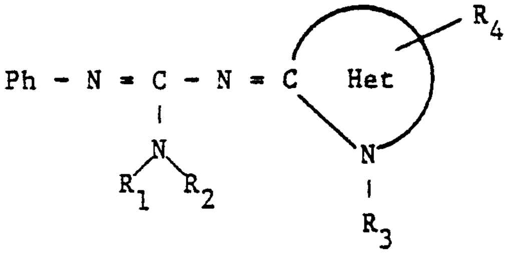

(Ib),

worin $R_1$ und $R_2$ einen gegebenenfalls substituierten aliphatischen oder cycloaliphatischen Kohlenwasserstoffrest bedeuten oder $R_1$ und $R_2$ zusammengenommen einen gegebenenfalls substituierten bivalenten Kohlenwasserstoffrest aliphatischen Charakters, in welchem die Kohlenstoffatome der Kette durch ein Heteroatom unterbrochen sein können, $R_3$ Wasserstoff, Niederalkyl, $R_4$ Wasserstoff, Niederalkyl, Niederalkoxy, Niederalkylamino, Diniederalkylamino, Halogen, Niederalkoxycarbonyl oder gegebenenfalls substituiertes Phenyl, oder an einem beidseitig einfach gebundenen Kohlenstoffatom auch Oxo, Het einen Heteroalkylenrest mit 4-6 Kettengliedern, der die Gruppe C-N zu einem heterocyclischen Sechs- bis Achtring, der neben dem Stickstoffatom noch ein Heteroatom aus der Gruppe Sauerstoff, Schwefel oder Stickstoff im Ring enthält, ergänzt, und Ph einen gegebenenfalls substituierten Phenylrest bedeuten, ihre tautomeren Verbindungen und Salze.

Die Erfindung betrifft in erster Linie Verbindungen der Formel Ib, in der $R_1$ und $R_2$ Niederalkyl oder Cycloalkyl, oder beide zusammengenommen eine gerade, verzweigte oder substituierte Niederalkylenkette, die gegebenenfalls durch ein Sauerstoff-, Schwefel- oder gegebenenfalls durch z.B. Niederalkyl, Phenyl oder Niederalkoxycarbonyl substituiertes Stickstoffatom unterbrochen sein kann, $R_3$ Wasserstoff oder Niederalkyl, $R_4$ Wasserstoff, Niederalkyl, Niederalkoxy, Niederalkylamino, Diniederalkylamino, Halogen, Niederalkoxycarbonyl, oder an einem beidseitig einfach gebundenen Kohlenstoffatom auch Oxo, oder gegebenenfalls durch Niederalkyl, Niederalkoxy oder Halogen substituiertes Phenyl, und Het einen Heteroalkylenrest mit 4-6 Kettengliedern, der die Gruppe C-N zu einem heterocyclischen Sechs- bis Achtring, der neben dem Stickstoffatom noch ein Heteroatom aus der Gruppe Sauerstoff, Schwefel oder Stickstoff im Ring enthält, ergänzt, und Ph einen gegebenenfalls substituierten Phenylrest bedeuten, als auch ihre tautomeren Verbindungen und Salze.

Insbesondere betrifft die Erfindung diejenigen Verbindungen der Formel Ib, in der $R_1$ und $R_2$ Niederalkyl oder Cycloalkyl, oder beide zusammengenommen eine gerade, verzweigte oder substituierte Niederalkylenkette, die gegebenenfalls durch ein Sauerstoff- oder

Schwefelatom oder gegebenenfalls durch z.B. Niederalkyl, Phenyl oder Niederalkoxycarbonyl substituiertes Stickstoffatom unterbrochen sein kann, als Niederalkylenamino beispielsweise Pyrrolidino, 2,5-Dimethylpyrrolidino, Piperidino, 2-Methylpiperidino, 4-Phenylpiperidino, Hexahydroazepino oder Octahydroazocino, als Oxaniederalkylenamino beispielsweise Morpholino, 2,6-Dimethylmorpholino, als Thianiederalkylenamino beispielsweise Thiomorpholino oder als Azaniederalkylenamino beispielsweise Piperazino, N-Niederalkoxycarbonyl, N-Methyl- oder N-Phenylpiperazino bedeuten, $R_3$ Wasserstoff oder Niederalkyl, $R_4$ Wasserstoff, Niederalkyl, Niederalkoxy, Niederalkylamino, Diniederalkylamino, Halogen, Niederalkoxycarbonyl, oder gegebenenfalls durch Niederalkyl, Niederalkoxy oder Halogen substituiertes Phenyl, und Het einen zweiwertigen Heteroalkylenrest mit 4-5 Kettengliedern, der die Gruppe C-N zu einem heterocyclischen Sechs- bis Siebenring, der neben dem Stickstoffatom noch ein Heteroatom aus der Gruppe Sauerstoff, Schwefel oder Stickstoff, im Ring enthält, ergänzt, und Ph einen gegebenenfalls durch Niederalkyl, Niederalkoxy oder Halogen substituierten Phenylrest bedeuten, als auch ihre tautomeren Verbindungen und Salze.

Von besonderem Interesse sind Verbindungen der Formel Ib, in der $R_1$ und $R_2$ Niederalkyl oder die Gruppe $-NR_1R_2$ gemeinsam ein Niederalkylenamino, in der die gerade, verzweigte oder durch Phenyl substituierte Niederalkylenkette gegebenenfalls durch ein Sauerstoff-, Schwefel- oder gegebenenfalls durch z.B. Niederalkyl, Phenyl oder Niederalkoxycarbonyl substituiertes Stickstoffatom unterbrochen sein kann, bedeutet und beispielsweise als Niederalkylenamino Pyrrolidino, 2,5-Dimethylpyrrolidino, Piperidino, 2-Methylpiperidino, 4-Phenylpiperidino, Hexahydroazepino oder Octahydroazocino, als Oxaniederalkylenamino beispielsweise Morpholino, 2,6-Dimethylmorpholino, als Thianiederalkylenamino beispielsweise Thiomorpholino oder als Azaniederalkylenamino beispielsweise Piperazino, N-Aethoxycarbonyl-, N-Methyl-, N-Phenylpiperazino sein kann, $R_3$ Wasserstoff oder Nieder-

alkyl, $R_4$ Wasserstoff, Niederalkyl, Niederalkoxy, Niederalkylamino, Halogen, Niederalkoxycarbonyl, oder gegebenenfalls durch Niederalkyl, Niederalkoxy oder Halogen substituiertes Phenyl und Het einen zweiwertigen Heteroalkylenrest mit 4-5 Kettengliedern, der die Gruppe C-N zu einem heterocyclischen Sechs- bis Siebenring, der neben dem Stickstoffatom noch ein Heteroatom aus der Gruppe Sauerstoff, Schwefel oder Stickstoff im Ring enthält, ergänzt, und beispielsweise ein Morpholin-, Thiomorpholin-, 2H-3,4,5,6-Tetrahydro-1,3-thiazin-, 2H-3,4,5,6-Tetrahydropyrimidin-, Piperazin- oder auch ein Hexahydro-1,4-thiazepinring sein kann, und Ph einen gegebenenfalls durch Niederalkyl, Niederalkoxy, oder Halogen substituierten Phenylrest bedeutet, als auch ihre tautomeren Verbindungen und Salze.

Von ganz besonderem Interesse sind Verbindungen der Formel Ib, in der $R_1$ und $R_2$ Niederalkyl, z.B. Methyl oder Aethyl, oder die Gruppe $-NR_1R_2$ gemeinsam ein Niederalkylenamino, in der die gerade, verzweigte oder durch Phenyl substituierte Niederalkylenkette gegebenenfalls durch ein Sauerstoff-, Schwefel- oder gegebenenfalls durch Niederalkyl, wie z.B. Methyl oder Aethyl oder Niederalkoxycarbonyl, wie z.B. Methoxy- oder Aethoxycarbonyl substituiertes Stickstoffatom unterbrochen sein kann, bedeutet und beispielsweise Pyrrolidino, Piperidino, 4-Phenylpiperidino, Morpholino, 2,6-Dimethylmorpholino, Thiomorpholino, Piperazino, N-Aethoxycarbonylpiperazino sein kann, $R_3$ Wasserstoff oder Niederalkyl, z.B. Methyl oder Aethyl, $R_4$ Wasserstoff, Niederalkyl, z.B. Methyl oder Aethyl, oder Niederalkoxycarbonyl, z.B. Methoxy- oder Aethoxycarbonyl und Het einen zweiwertigen Heteroalkylenrest, der mit der Gruppe C-N diese zu einem heterocyclischen Sechsring, der neben dem Stickstoffatom noch ein Heteroatom aus der Gruppe Sauerstoff, Schwefel oder Stickstoff im Ring enthält, ergänzt, und beispielsweise ein Morpholin-, Thiomorpholin-, oder auch Piperazinring sein kann, und Ph einen gegebenenfalls durch Niederalkyl, wie z.B. Methyl oder Aethyl, Halogen, wie z.B. Fluor, Chlor oder Brom oder Niederalkoxy, wie z.B. Methoxy oder Aethoxy, substituierten Phenylrest bedeutet, als auch ihre tautomeren Verbindungen und Salze.

Die neuen Guanidine der Formel I werden nach an sich bekannten Methoden erhalten.

So kann man z.B. die neuen Verbindungen der Formel I erhalten, indem man eine Verbindung der Formel II

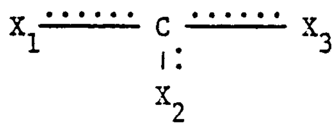

(II)

worin $X_1$ die Gruppe Ph-N= in der Ph einen gegebenenfalls substituierten Phenylrest bedeutet, oder eine abspaltbare Gruppe, $X_2$ die Gruppe

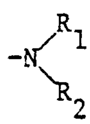

, worin $R_1$ und $R_2$ die unter der Formel I angegebene Bedeutung haben, oder eine abspaltbare Gruppe und $X_3$ die Gruppe

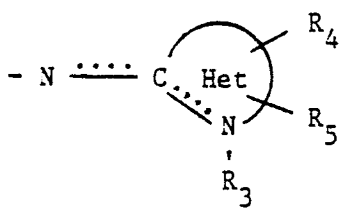

, worin $R_3$ , $R_4$, $R_5$ und Het als zweiwertiger Heteroalkylenrest mit der Gruppe C-N die unter der Formel I angegebenen Bedeutungen haben, oder eine abspaltbare Gruppe bedeutet, mit der Massgabe, dass nur einer der Substituenten $X_1$, $X_2$ oder $X_3$ eine abspaltbare Gruppe sein kann, und worin eine der Gruppen $X_1$, $X_2$ und $X_3$ durch eine Doppelbindung mit dem Kohlenstoffatom verbunden ist, mit einem Amin oder Imin umsetzt, welches mit der fehlenden Amino- oder Iminogruppe identisch ist, die unter $X_1$, $X_2$ oder $X_3$ definiert werden, um die abspaltbare Gruppe zu ersetzen, und, wenn erwünscht, zusätzliche Verfahrensschritte durchführt, und/oder, wenn erwünscht, erhaltene Verbindungen in ein Salz überführt, und/oder, wenn erwünscht, erhaltene Salze der Verbindungen der Formel I in die freien Basen umwandelt.

Als abspaltbare Gruppen $X_1$, $X_2$ und $X_3$ kommen beispielsweise Niederalkylthiogruppen, wie z.B. Methylthio oder Aethylthio, Niederalkoxy, wie z.B. Methoxy oder Aethoxy, oder auch Halogen, wie z.B. Chlor oder Brom in Frage.

Verbindungen der allgemeinen Formel II sind, je nachdem die abspaltbare Gruppe $X_1$, $X_2$ oder $X_3$ bedeutet, entweder Verbindungen der Formel IIa

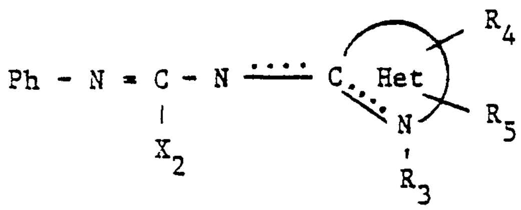

(IIa)

worin $X_2$ eine abspaltbare Gruppe, Ph, $R_3$, $R_4$, $R_5$, Het und C-N die oben angegebenen Bedeutungen haben,
Verbindungen der allgemeinen Formel IIb,

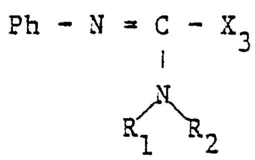

(IIb)

worin $X_3$ eine abspaltbare Gruppe bedeutet, oder Verbindungen der Formel IIc

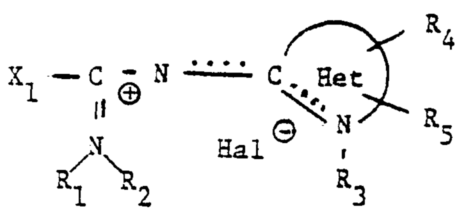

(IIc)

worin $X_1$ eine abspaltbare Gruppe bedeutet, ihre tautomeren Formen, oder ihre Säureadditionssalze.

Je nachdem als abspaltbare Gruppe $X_1$, $X_2$ oder $X_3$ in einer Verbindung der Formel II vorhanden ist, setzt man eine Verbindung der Formel IIa mit einem Amin der Formel $HNR_1R_2$, eine Verbindung der Formel IIb mit einer Iminoverbindung der Formel III

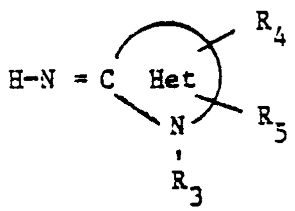 (III) oder eine Verbindung der Formel IIc mit

einem gegebenenfalls substituierten Anilin der Formel $Ph-NH_2$ um. Verbindungen der Formeln IIa, IIb und IIc könnnen auch als Säureadditionssalze, vorzugsweise als Hydrohalogenide verwendet werden. In analoger Weise können auch die verwendeten Amine, Iminoverbindungen, oder auch Aniline als Säureadditionssalze, vorzugsweise Hydrohalogenide umgesetzt werden.

Die Umsetzung einer Verbindung der Formel II, d.h. insbesondere einerVerbindung der Formel IIa, IIb oder IIc beispielsweise mit einem vorher genannten Amin oder Imin als freie Base erfolgt unter Verwendung eines stöchiometrischen Ueberschusses des Amins oder Imins, beispielsweise in einem Molverhältnis 1:1 bos 1:2,0. Bei Verwendung eines nur geringen Ueberschusses des Amins oder Imins als freie Base oder bei Verwendung des Amins oder Imins als Säureadditionssalze ist es zweckmässig, zusätzlich eine stöchiometrisch äquivalente Menge eines tertiären Alkylamins, wie z.B. Triäthylamin oder N-Aethyldiisopropylamin hinzuzusetzen.

Setzt man beispielsweise eine Iminoverbindung der Formel III als freie Base

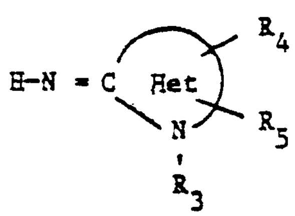 (III)

mit einer Verbindung der Formel IIb, in der $X_3$ ein Halogen bedeutet, um, so verwendet man vorzugsweise 2 Moläquivalente oder mehr der freien Base der oben erwähnten Iminoverbindung. Gemäss folgendem Reaktionsschema

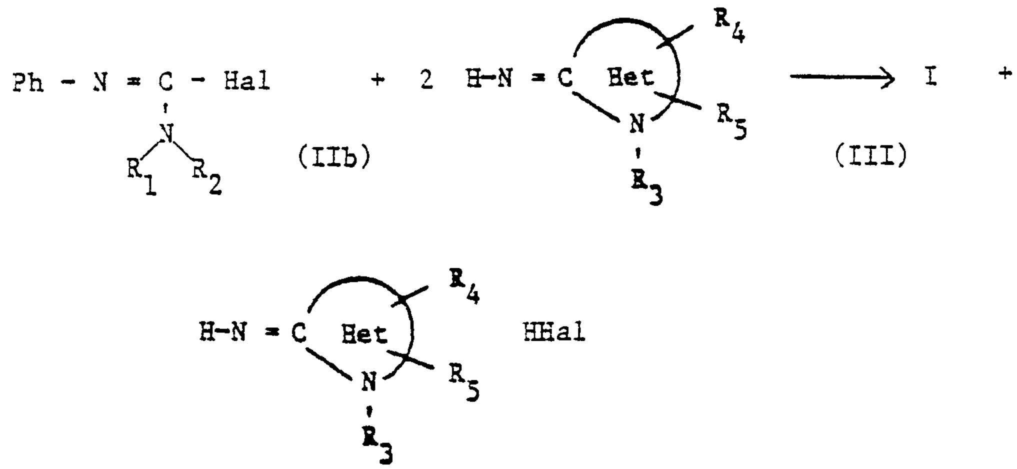

(IIb) (III)

entsteht ein Aequivalent der Iminoverbindung als Säureadditionssalz. Aus diesem Grunde wird die Umsetzung vorzugsweise in einem aprotischen Lösungsmittel durchgeführt, in dem die erhaltene Verbindung der Formel I löslich, wogegen das Additionssalz der Halogenwasserstoffsäure gemäss obigen Schema als unlösliche Verbindung ausfällt. Auf diese Weise lassen sich die beiden erhaltenen Reaktionsprodukte leicht durch einfache Filtration voneinander trennen. Das erhaltene Säureadditionssalz der Iminoverbindung wird durch basische Hydrolyse, beispielsweise durch Zusatz von Alkali- oder Erdalkalihydroxyd oder -carbonat in die freie Base übergeführt und kann hiermit als Ausgangsprodukt zur Wiederverwendung zurückgewonnen werden. Vorzugsweise setzt man jedoch Verbindungen der Formel IIb und III als Säureadditionssalze, beispielsweise als Hydrohalogenide, wie oben angegeben in Gegenwart eines zusätzlichen tertiären Alkylamins, wie z.B. Tritäthylamin oder N-Aethyldiisopropylamin um.

Die beschriebenen Umsetzungen von Verbindungen der Formel IIb mit einer Iminoverbindung der Formel III erfolgen, wie bereits erwähnt, vorzugsweise in aprotischen Lösungsmitteln. Beispiele für bevorzugt verwendbare Lösungsmittel sind Aether, wie z.B. Diäthyläther und Tetrahydrofuran, niedere aliphatische Ketone und Ester, wie z.B. Aceton, Methyläthylketon und Aethylacetat, aromatische Kohlenwasserstoffe, wie z.B. Benzol, Toluol oder Xylol, sowie Acetonitril. Besonders bevorzugt jedoch wird die Umsetzung in Diäthyläther oder Acetonitril ausgeführt. Die Umsetzungen

können bei einer Temperatur zwischen 0-150°C, vorzugsweise jedoch zwischen Raumtemperatur und Rückflusstemperatur des Reaktionsgemisches ausgeführt werden.

Verwendet man als Ausgangsverbindung der Formel II jedoch zum Beispiel eine Verbindung der Formel IIa,

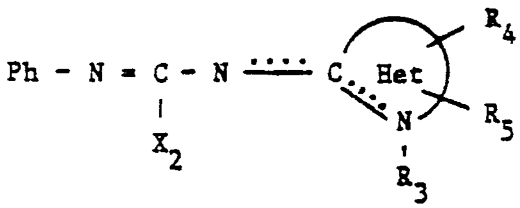

(IIa)

so hat $X_2$ als abspaltbare Gruppe vorzugsweise die Bedeutung einer Niederalkoxy- oder Niederalkylthiogruppe. Ausgangsverbindungen der Formel IIa werden in Form ihrer Salze, beispielsweise in Form ihrer Säureadditionssalze mit einer Halogenwasserstoffsäure mit einem Amin der Formel $HNR_1R_2$ als freie Base oder als Säureadditionssalz umgesetzt, in der $R_1$ und $R_2$ die vorstehend angegebene Bedeutung haben.

Die Umsetzungen werden z.B. in einem Alkohol als Lösungsmittel, vorzugsweise in einem niederen Alkanol, wie z.B. Aethanol, Isopropanol oder tert.-Butanol, besonders bevorzugt jedoch in einem Aether, wie z.B. Diäthyläther oder Tetrahydrofuran oder in Acetonitril bei Temperaturen von Raumtemperatur bis vorzugsweise zur Rückflusstemperatur des Reaktionsgemisches durchgeführt. Die Reaktionen können jedoch in einem geschlossenen Reaktionsgefäss unter Druck, wie z.B. in einem Bombenrohr oder in einem Autoklaven, bei höheren Temperaturen ausgeführt werden. Die Guanidinderivate der allgemeinen Formel I werden in Form ihrer Salze erhalten, die beispielsweise durch alkalische Hydrolyse in die entsprechenden freien Basen überführt werden können. Bei der Umsetzung der Verbindungen der allgemeinen Formel IIa mit dem Amin der allgemeinen Formel $HNR_1R_2$ wird das Amin vorzugsweise in stöchiometrischem Ueberschuss verwendet, beispielsweise in einem Molverhältnis 1:1 bis 1:2,0 und mehr. Bei Verwendung nur eines geringen Ueberschusses des Amins oder eines Säureadditionssalzes kann es zweckmässig sein, zusätzlich eine stöchiometrisch äquivalente Menge eines tertiären Alkylamind, wie z.B. Triäthylamin oder N-Aethyldiisopropylamin , zuzusetzen, um die Reaktionsgeschwindigkeit zu erhöhen.

Die Umsetzungen von Verbindungen der Formel IIc mit einer abspaltbaren Gruppe $X_1$, die neben der Bedeutung eines Halogenatoms, vorzugsweise Niederalkoxy oder Niederalkylthio bedeutet, oder ihrer tautomeren Form mit einem gegebenenfalls substituierten Anilin als freie Base erfolgen in gleicher Weise wie bei der Umsetzung einer Verbindung der Formel IIa mit einem Amin der Formel $HNR_1R_2$ beschrieben. Die Umsetzungen werden vorteilhafterweise auch in einem stöchiometrischen Ueberschuss der gegebenenfalls substituierten Aniline ausgeführt. Bei Verwendung nur eines geringen Ueberschusses des Anilins oder eines Säureadditionssalzes davon kann es zweckmässig sein, eine stöchiometrisch äquivalente Menge eines bereits oben definierten tertiären Trialkylamins zuzusetzen. Die Umsetzungen werden in analogen Lösungsmitteln, wie vorher bei der Umsetzung der Verbindung IIa mit III beschrieben, ausgeführt.

Verbindungen der allgemeinen Formel I können auch hergestellt werden, indem man eine Guanidinverbindung der allgemeinen Formel IV

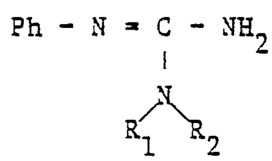

(IV)

worin Ph, $R_1$ und $R_2$ die unter der Formel I angegebenen Bedeutungen haben, mit einer Verbindung der allgemeinen Formel V

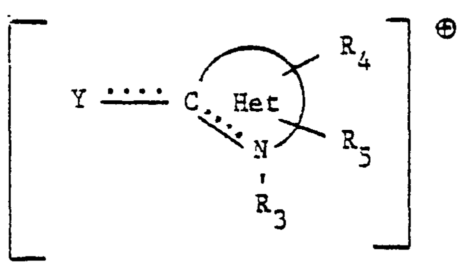

$Z^{\ominus}$ (V)

worin Y Niederalkoxy, wie z.B. Methoxy oder Aethoxy, Niederalkylthio, wie z.B. Methylthio oder Aethylthio, Halogen, wie z.B. Chlor oder Brom, oder Y zwei Niederalkoxygruppen, di am gleichen C-Atom sitzen, und Z ein Tetrafluoroborat-, ein Fluorsulfonat-, ein Niederalkylsulfat-, wie z.B. Methylsulfat- oder Niederalkansulfonat-, wie z.B. Methansulfonatanion oder ein

Halogenid, wie z.B. Chlorid oder Bromid, bedeutet, wobei, wenn Y die Bedeutung von zwei Niederalkoxygruppen am gleichen C-Atom hat, Z als Anion entfällt, oder, wenn $R_3$ Wasserstoff bedeutet, die tautomere Form als freie Base vorliegt, umsetzt, und, wenn erwünscht, zusätzliche Verfahrensschritte durchführt und, wenn erwünscht, erhaltene Verbindungen der Formel I in ein Salz überführt, und/oder, wenn erwünscht, erhaltene Salze der Verbindungen der Formel I in die freien Verbindungen überführt.

Die Verbindungen der allgemeinen Formel I werden zweckmässig in der Weise hergestellt, dass man ein Lactamsalz der vorstehend angegebenen Formel V mit einem Guanidinderivat der oben definierten Formel IV in stöchiometrischen Mengen zur Umsetzung bringt. Die Umsetzungen werden vorzugsweise in einem wasserfreien organischen Lösungsmittel durchgeführt. Organische Lösungsmittel sind z.B. niedere Alkanole, wie z.B. Methanol, Aethanol, Isopropanol, tert.-Butanol, Aether, wie z.B. Diäthyläther, Tetrahydrofuran oder Dioxan, niedere halogenierte Kohlenwasserstoffe, wie z.B. Chloroform, Methylenchlorid oder 1,2-Dichloräthan, und aromatische Kohlenwasserstoffe, wie z.B. Benzol, Toluol oder Xylol. Im allgemeinen wird die Reaktion bei Temperaturen, die zwischen -20°C und +50°C liegen, vorzugsweise jedoch zwischen 0°C und Raumtemperatur durchgeführt. Das in Salzform erhaltene Reaktionsprodukt der allgemeinen Formel I wird durch basische Hydrolyse, beispielsweise durch Zusatz von einem Alkali- oder Erdalkalihydroxyd oder -carbonat in die freie Base übergeführt.

Die verfahrensgemäss eingesetzten Lactamfluoroborate oder -fluorsulfonate der allgemeinen Formel V, in der $Z^{\ominus}$ beispielsweise die Tetrafluorborat-Gruppe der Formel $BF_4^{\ominus}$ oder Fluorsulfonatgruppe der Formel $OSO_2F^{\ominus}$ darstellt, können nach üblichen Verfahren hergestellt werden, indem man ein Lactam der Formel Va

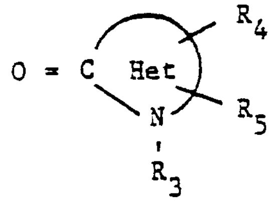

(Va)

mit dem entsprechenden Trialkyloxoniumfluoroborat oder einem Fluorsulfonsäureniederalkylester zu dem entsprechenden Lactamsalz der allgemeinen Formel V umsetzt.

Die Umsetzung wird beispielsweise bei Temperaturen zwischen -20°C und +50°C, vorzugsweise bei Temperaturen zwischen 0°C und +25°C in einem inerten Gas, wie z.B. Stickstoff oder Argon, und in Gegenwart eines inerten, wasserfreien organischen Lösungsmittels, beispielsweise in einem niederen Halogenkohlenwasserstoff, wie z.B. Chloroform, 1,2-Dichloräthan oder vorzugsweise Methylenchlorid durchgeführt. Beispiele für andere verwendbare organische Lösungsmittel sind Aether, wie z.B. Diäthyläther, Dioxan, Tetrahydrofuran oder 1,2-Dimethoxyäthan, sowie aromatische Kohlenwasserstoffe, wie z.B. Benzol, Toluol oder Xylol.

Die unter die allgemeine Formel V fallenden 2-Niederalkylthiolactimäther können durch Umsetzung des Lactams der allgemeinen Formel Va mit Phosphorpentasulfid in analoger Weise nach R.Gomper et al., Org. Syn. Coll., Vol. V, Seiten 780-785 hergestellt werden. Bei Ausführung dieser Umsetzung erhält man zunächst ein Thiolactam, welches durch Umsetzung mit einem Alkylierungsmittel den 2-Alkylthiolactimäther in Form der entsprechenden Salze liefert. Als Alkylierungsmittel kann ein Alkylhalogenid, beispielsweise Methyljodid, ein Fluorsulfonsäurealkylester, wie z.B. Fluorsulfonsäuremethylester, ein Niederalkansulfonsäurealkylester, z.B. Methansulfonsäurealkylester, wie z.B. Methansulfonsäuremethylester, ein Toluolsulfonsäurealkylester, wie z.B. Toluolsulfonsäuremethylester, oder Dialkylsulfat, wie z.B. Dimethylsulfat verwendet werden. Die Umsetzung der Lactimäthersalze mit dem Guanidinderivat der allgemeinen Formel IV liefert die entsprechenden Salze der allgemeinen Formel I.

Bei der Umsetzung der vorher beschriebenen Lactamfluorsulfonate der allgemeinen Formel V mit den Guanidinen der allgemeinen Formel IV können als Nebenreaktion auch quaternäre Ammoniumsalze der Verbindungen der allgemeinen Formel I entstehen.

 

Die auch unter die allgemeine Formel V fallenden Methylsulfatsalze werden in analoger Weise wie von H. Bredereck et al., Chem. Ber., Bd. 96, (1963), S. 1350 für Pyrrolidone beschrieben, aus Lactamen der allgemeinen Formel Va durch Umsetzung mit Dimethylsulfat erhalten. Die Umsetzung wird vorzugsweise in einem wasserfreien, inerten organischen Lösungsmittel, beispielsweise einem aromatischen Kohlenwasserstoff, wie z.B. Benzol, Toluol oder Xylol, einem Aether, wie z.B. Diäthyläther, Dioxan oder Tetrahydrofuran, oder einem halogenierten aliphatischen Kohlenwasserstoff, wie z.B. 1,2-Dichloräthan oder Chloroform durchgeführt. Das erhaltene Methylsulfat der allgemeinen Formel V wird dann mit dem entsprechenden Guanidinderivat der allgemeinen Formel IV auf vorstehend beschriebene Weise zum entsprechenden Niederalkylsulfatsalz, wie z.B. Methylsulfatsalz der Verbindung der allgemeinen Formel I überführt. Die erhaltenen Salze lassen sich durch Behandlung mit einem Alkali- oder Erdalkalihydroxyd oder -carbonat in entsprechende freie Basen der allgemeinen Formel I umwandeln.

Aus dem Niederalkylsulfat-, wie z.B. Methylsulfatsalz der allgemeinen Formel V lässt sich beispielsweise durch Umsetzung mit einem Metallalkoxid, vorzugsweise einem Alkalimetallalkoxid, wie z.B. Natriummethoxid oder -äthoxid, im entsprechenden wasserfreien niederen Alkanol das entsprechende Lactamacetal der Formel Vb

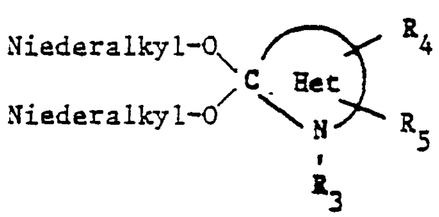

(Vb)

herstellen.

Aus den Lactamacetalen lassen sich, wie vorstehend beschrieben, mit den Guanidinderivaten der allgemeinen Formel IV die freien Basen der allgemeinen Formel I herstellen.

Die verfahrensgemäss verwendeten Halogenid-, insbesondere Chloridsalze der Lactame der allgemeinen Formel V lassen sich in analoger Weise wie von W. Jentsch und M. Seefelder, Chem. Ber., Bd. 98 (1965), S. 274 wie für Pyrrolidone beschrieben, durch Umsetzung eines Lactams der allgemeinen Formel Va mit Phosgen oder Thionylchlorid herstellen.

Wie bereits vorstehend erwähnt, können für die Herstellung der Verbindungen der allgemeinen Formel I, in der $R_3$ ein Wasserstoffatom bedeutet, auch die freien Basen der allgemeinen Formel V verwendet werden. Die Umsetzung der Salze der allgemeinen Formel V mit einer Base, wie z.B. einem Alkali- oder Erdalkalihydroxyd- oder -carbonat, vorzugsweise in einem halogenierten aliphatischen Kohlenwasserstoff als Lösungsmittel, wie z.B. Methylenchlorid oder Chloroform, liefert die freien Basen der allgemeinen Formel Vc

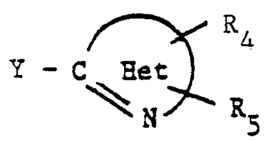

(Vc) .

Verbindungen der allgemeinen Formel I können auch nach einem weiteren Verfahren hergestellt werden, indem man Guanidinderivate der allgemeinen Formel VI

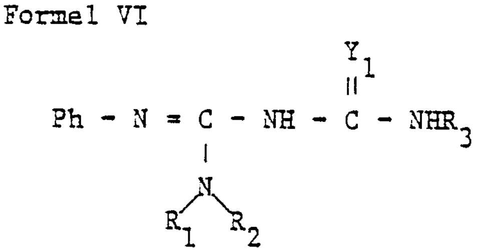

(VI)

oder eine tautomere Form davon, worin Ph, $R_1$, $R_2$ und $R_3$ die vorstehend genannten Bedeutungen haben und $Y_1$ die Bedeutung einer Oxo-, Thioxo- oder NH-Gruppe hat, mit einer Verbindung der allgemeinen Formel VII

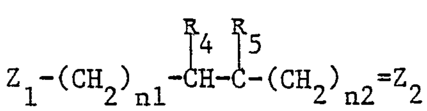

(VII)

umsetzt, worin $n_1$ oder $n_2$ 0, 1, 2 oder 3 bedeuten, mit der Massgabe, dass $n_1$ und $n_2$ zusammen nicht mehr als 3 bedeuten, $Z_1$ ein Halogenatom, $Z_2$ eine Oxogruppe oder zusammen die Gruppe bestehend aus Wasserstoff und Halogen, wobei das Wasserstoffatom Teil einer Methylengruppe sein kann,

oder $Z_1$ und $Z_2$ zusammengenommen über eine Iminogruppe den bivalenten Alkylenrest $-\overset{R_4}{\overset{|}{C}H}-\overset{R_5}{\overset{|}{C}H}-$ zu einem Aziridinderivat ergänzen, wobei $R_4$ und $R_5$ die Bedeutungen von Wasserstoff haben, und, wenn erwünscht, zusätzliche Verfahrenschritte durchführt, und/oder, wenn erwünscht, erhaltene Verbindungen in ein Salz überführt, und/oder, wenn erwünscht, erhaltene Salze der Verbindungen der Formel I in die freien Basen umwandelt.

Beispielsweise wird eine Verbindung der Formel VI, in der $Y_1$ eine Oxo- oder Thioxogruppe bedeutet, mit einem Halogenacetaldehyd der Formel VII, in der $Z_1$ ein Halogen, $Z_2$ eine Oxogruppe $n_1$ und $n_2$ 0 und $R_5$ Wasserstoff bedeutet, vorzugsweise in einem Lösungsmittel mit oder ohne Zusatz eines säurebindenden Mittels umgesetzt.

Als Lösungsmittel können beispielsweise niedere Alkanole, wie z.B. Methanol, Aethanol, Isopropanol, Butanol; Ketone, wie z.B. Aceton, Butanon, Methylisopropylketon; Aether, wie z.B. 1,2-Dimethoxy-äthan, Diisopropyläther, Tetrahydrofuran oder Dioxan; Carbonsäurederivate, wie z.B. Acetonitril, Essigsäureäthylester, Dimethylformamid; Aromaten, wie z.B. Benzol, Toluol oder Xylol, Aliphaten oder Cycloaliphaten, wie z.B. Benzine und Ligroine mit Siedebereichen zwischen 60°C bis 180°C, Cyclohexan; halogenierte aliphatische Kohlenwasserstoffe, wie z.B. Methylenchlorid, Chloroform, Tetrachlormethan oder 1,2-Dichloräthan verwendet werden.

Als säurebindende Mittel können beispielsweise verwendet werden: anorganische Basen, wie z.B. Natriumhydrogencarbonat, Natriumcarbonat, Kaliumcarbonat, Trinatriumphosphat, Natriumhydroxid, Kaliumhydroxid oder organische Basen, wie z.B. Triäthylamin oder Benzyl-dimethylamin.

Zur Umsetzung von Verbindungen der Formel VI mit Halogenacetaldehyden oder Halogenacetaldehyd-abspaltenden Verbindungen, wie z.B. entsprechenden Acetalen, werden vorzugsweise äquimolare oder annähernd äquimolare Mengen der beiden

Komponenten verwendet. Insbesondere kann es zweckmässig sein, einen geringen Ueberschuss (1 - 15 Mol-%) an Halogenacetaldehyd oder Halogenacetaldehyd-abspaltender Verbindung einzusetzen. Hierzu wird die Verbindung der Formel VI in einem Lösungsmittel gelöst oder suspendiert und der Halogenacetaldehyd oder die Halogenacetaldehyd-abspaltende Verbindung langsam zugegeben. Das säurebindende Mittel kann dabei ebenfalls mit vorgelegt oder auch erst nachträglich zugegeben werden. Die Umsetzung wird bei 0°C bis zum Siedepunkt des verwendeten Lösungsmittels, beispielsweise bis zu einer Temperatur von 150°C durchgeführt; der bevorzugte Temperaturbereich liegt bei 20°C bis 100°C.

In analoger Weise kann auch eine Verbindung der Formel VI, in der Y eine Thioxogruppe bedeutet, mit einer Dihalogenäthanverbindung der Formel VII, in der $Z_1$ ein Halogenatom und $Z_2$ ein Halogenatom und Wasserstoffatom bedeuten, zu den gleichen Verbindungen der Formel I umgesetzt werden. In analoger Weise wie oben beschrieben, werden die Umsetzungen in einem organischen Lösungsmittel oder im Ueberschuss der verwendeten Dihalogenäthanverbindung der Formel VII ausgeführt. Vorzugsweise erfolgt die Umsetzung in einem niederen Alkanol, wie z.B. Methanol, Aethanol, Isopropanol oder Butanol.

Die unter die Formel VII fallenden Aziridine, in denen $R_4$ und $R_5$ Wasserstoff bedeuten, lassen sich mit einem Thioharnstoffderivat der Formel VI in wässerig-saurer Lösung oder vorzugsweise in einem nichtpolaren Lösungsmittel, wie z.B. in einem der oben angegebenen Ketone bei einer Temperatur zwischen 0°C bis 100°C, vorzugsweise jedoch zwischen 0°C und 30°C umsetzen. Verwendet man als Lösungsmittel jedoch eine wässerig-saure Lösung, so muss das Reaktionsgemisch nach erfolgter Umsetzung alkalisch gestellt werden, um die erhaltene Verbindung der Formel I als freie Base zu erhalten.

Die erfindungsgemässen Verbindungen der Formel I, worin Het einen Heteroalkylenrest mit 4 - 6 Kettengliedern, der die Gruppe C-N zu einem heterocyclischen 6 - 8 Ring ergänzt, können hergestellt werden, in dem man Verbindungen der Formel IV

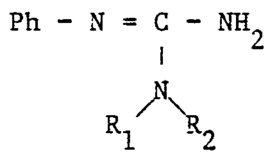

(IV) ,

worin $R_1$, $R_2$ und Ph die oben angegebenen Bedeutungen haben, mit einem Halogenalkylenisothiocyanat der Formel

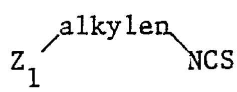

(VIII) ,

worin $Z_1$ ein Halogenatom bedeutet, und der bivalente Rest "alkylen" eine Alkylenkette mit 3-5 Kohlenstoffatomen bedeutet, wobei ein Wasserstoff in der Alkylenkette durch den Substituenten $R_4'$, der die oben angegebene Bedeutung für $R_4$ mit Ausnahme von Wasserstoff hat, ersetzt sein kann, umsetzt, und, wenn erwünscht, zusätzliche Verfahrensschritte durchführt, und/oder, wenn erwünscht, erhaltene Verbindungen in ein Salz überführt, und/oder, wenn erwünscht, erhaltene Salze der Verbindungen der Formel I in die freien Basen umwandelt.

Die Umsetzung wird vorzugsweise in einem wasserfreien, inerten organischen Lösungsmittel, beispielsweise einem aromatischen Kohlenwasserstoff, wie z.B. Benzol, Toluol oder Xylol, einem Aether, wie z.B. Diäthyläther, Dioxan oder Tetrahydrofuran, durchgeführt. Die Umsetzungen werden bei einer Temperatur von 0°C bis zum Siedepunkt des verwendeten Lösungsmittels, beispielsweise bis zu einer Temperatur von 120°C durchgeführt; der bevorzugte Temperaturbereich liegt bei 20°C bis 100°C.

Die erfindungsgemässen Verbindungen der Formel I, in der $R_1$ die oben definierten Bedeutung hat und $R_2$ und/oder $R_3$ Wasserstof bedeuten, können nach einem weiteren Verfahren durch Umsetzung mit einem reaktionsfähigen Ester eines aliphatischen oder cycloaliphatischen Alkohols in Verbindungen der Formel I umgewandelt werden, in denen $R_2$ und/oder $R_3$ im Rahmen der vorstehenden Definition für $R_2$ und $R_3$ verschieden von Wasserstoff sind, und, wenn erwünscht, zusätzliche Verfahrensschritte durchgeführt, und/oder, wenn erwünscht, erhaltene Verbindungen der Formel I in ein Salz übergeführt, und/oder, wenn erwünscht, erhaltene Salze der Verbindungen der Formel I in die freien Basen umgewandelt werden.

Als reaktionsfähigen Ester, als sogenanntes Alkylierungsmittel kann man z.B. ein Alkyl- oder Cycloalkylhalogenid wie z.B. Methyl- oder Cyclohexyliodid, einen entsprechenden Fluorsulfonsäurealkylester, wie z.B. Fluorsulfonsäuremethylester, einen entsprechenden Niederalkansulfonsäureester, wie z.B. Methansulfonsäuremethylester, einen entsprechenden Toluolsulfonsäurealkylester, wie z.B. Toluolsulfonsäuremethylester, oder ein Dialkylsulfat, wie z.B. Dimethyl- oder Diäthylsulfat, verwenden.

Die erfindungsgemässen Verbindungen der Formel I, in denen $R_2$ und/oder $R_3$ Wasserstoff bedeuten, können anch einem weiteren Verfahren hergestellt werden, indem man in Verbindungen der allgemienen Formel IX

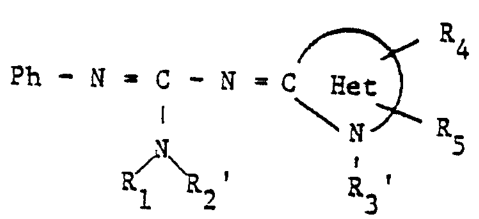

(IX)

worin $R_1$, $R_4$, $R_5$, Ph und Het die unter der Formel I angegebene Bedeutung haben, und der eine von den Substituenten $R_2'$ und $R_3'$ die Bedeutung von $R_2$ oder $R_3$ hat, und der andere eine Aminoschutzgruppe bedeutet, oder beide $R_2'$ und $R_3'$ eine Aminoschutzgruppe bedeuten, diese abspaltet und gewünschtenfalls zusätzliche Verfahrensschritte durchführt, und/oder, wenn erwünscht, erhaltene Verbindungen der Formel I in ein Salz überführt, und/oder, wenn erwünscht, erhaltene Salze der Verbindungen der Formel I in die freien Basen umwandelt.

Eine Aminoschutzgruppe $R_2'$ bzw. $R_3'$ ist in erster Linie eine Acylgruppe, wie Acyl einer aliphatischen, aromatischen oder araliphatischen Carbonsäure, insbesondere Niederalkanoyl, z.B. Acetyl oder Propionyl, oder Aroyl, z.B. Benzoyl, oder Acyl der Ameisensäure oder eines Kohlensäurehalbderivates, z.B. -esters, wie Formyl, Niederalkoxycarbonyl, z.B. Aethoxycarbonyl oder tert.-Butyloxycarbonyl, oder Arylniederalkoxycarbonyl, z.B. Benzyloxycarbonyl.

Die Abspaltung eines als Aminoschutzgruppe $R_2'$ und/oder $R_3'$ verwendeten Acylrestes erfolgt in an sich bekannter Weise, z.B. durch Solvolyse, in erster Linie mittels Alkoholyse, ferner mittels Hydrolyse. Die alkoholytische Abspaltung eines Acylrestes $R_2'$ und/oder $R_3'$ kann z.B. in Gegenwart eines stark basischen Mittels, bei erhöhter Temperatur, z.B. bei etwa 50°C bis etwa 120°C, erfolgen. Dabei verwendet man insbesondere einen Niederalkanol, z.B. n-Butanol oder Aethanol, und als starke Base ein Alkalimetall-, z.B. Natrium- oder Kalium-niederalkanolat, z.B. -n-butylat oder -äthylat, oder ein Alkalimetall-hydroxid, z.B. Natrium- oder Kaliumhydroxid.

Aminoschutzgruppen $R_2'$ und $R_3'$ , beispielsweise Niederalkoxycarbonylgruppen, wie tert.-Butyloxycarbonyl, lassen sich besonders schonend acidolytisch, z.B. durch Behandeln mit Trifluoressigsäure, abspalten.

Eine weitere, besonders schonend abspaltbare Aminoschutzgruppe ist eine Aethoxycarbonylgruppe, die in β-Stellung eine mit drei Kohlenwasserstoffresten substituierte Silylgruppe, wie Triphenylsilyl-, Dimethyl-butyl-silyl- oder vor allem Trimethylsilylgruppe, trägt. Eine solche β-(Trimethylsilyl)-äthoxycarbonylgruppe bildet mit der zu schützenden Aminogruppe eine entsprechende β-Trimethylsilyl-äthoxycarbonylaminogruppe, welche sich unter milden Bedingungen durch Einwirkung von Fluoridionen abspalten lässt. Als Fluoridionen-abgebende Reagentien kommen beispielsweise Fluoride quaternärer organischer Basen, wie Tetraäthylammoniumfluorid in Frage.

Es ist dabei zu beachten, dass als Aminoschutzgruppe $R_2'$ und/oder $R_3'$ nur solche in Frage kommen, die selektiv unter Erhaltung der Struktur der Verbindungen der allgemeinen Formel I abspaltbar sind.

Die Ausgangsstoffe sind bekannt, oder falls sie neu sind, lassen sie sich nach an sich bekannten Methoden herstellen. Wo es sich als zweckdienlich erweist, sind die verwendeten Ausgangsprodukte im Anschluss an das beschriebene Verfahren bereits beschrieben worden.

Verbindungen der allgemeinen Formel IIa, in der $X_2$ eine Niederalkylthiogruppe bedeutet, lassen sich beispielsweise aus den entsprechenden Thioharnstoffen der allgemeinen Formel X

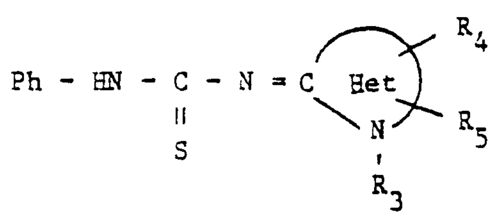

(X)

herstellen, indem man diese mit einem vorstehend aufgezählten Alkylierungsmittel der Formel $R_xR_y$, in der $R_x$ eine Niederalkyl-, beispielsweise Methyl- oder Aethylgruppe und $Z_y$ beispielsweise eine p-Toluolsulfonat-, Methansulfonat-, Fluorsulfonat- oder eine Niederalkylsulfat-, wie z.B. Methylsulfatgruppe, vorzugsweise Halogen, wie z.B. Chlor oder Brom, bedeutet, umsetzt.

Die Umsetzung wird in einem bereits vorstehend definierten organischen Lösungsmittel ausgeführt. Vorzugsweise verwendet man als Lösungsmittel einen Aether, wie z.B. Diäthyläther, Tetrahydrofuran oder Dioxan, ein Keton, wie z.B. Aceton oder 2-Butanon, einen halogenierten aliphatischen Kohlenwasserstoff, wie z.B. Chloroform oder Methylenchlorid oder einen niederen Alkanol, wie z.B. Methanol oder Aethanol. Besonders geeignet ist ein Alkylhalogenid in Methanol bzw. Aethanol. Im allgemeinen wird das Alkylierungsmittel in mindestens äquimolarer Menge verwendet. Die Alkylierung kann gegebenenfalls bei Raumtemperatur oder bei höheren Temperaturen und nötigenfalls in einem geschlossenen Reaktionsgefäss durchgeführt werden.

Verbindungen der allgemeinen Formel IX wiederum lassen sich aus den bereits erwähnten und bekannten Iminoverbindungen der Formel III

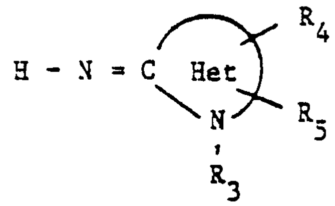

(III)

durch Umsetzung mit einem gegebenenfalls substituierten Phenylisothiocyanat der Formel Ph - NCS in einem bereits vorstehend definierten inerten organischen Lösungsmittel, vorzugsweise in Benzol, Methylenchlorid oder Chloroform, bei Temperaturen von 0°C bis Raumtemperatur während 2-24 Stunden in etwa äquimolaren Mengen herstellen.

Verbindungen der Formel IIb, in der $X_3$ als abspaltbare Gruppe Halogen, vorzugsweise Chlor bedeutet, werden nach der von E. Kühle, Angew. Chem., Intern. Ed., Bd. 8 (1969), S. 24-26 beschriebenen Methode erhalten, indem man ein Isocyaniddihalogenid der Formel XI

Ph - N = C - Cl<br>
|<br>
Cl (XI)

mit einem Amin der Formel $HNR_1R_2$, in Gegenwart eines Trialkylamins, wie z.B. Triäthylamin, in einem inerten aprotischen, wasserfreien Lösungsmittel umsetzt. Verbindungen der Formel X können auch als Immoniumchloride vorliegen. Als Lösungsmittel verwendet man z.B. einen Aether, beispielsweise Diäthyläther, Dioxan oder Tetrahydrofuran, einen halogenierten aliphatischen Kohlenwasserstoff, wie z.B. Chloroform oder Methylenchlorid, oder einen aromatischen Kohlenwasserstoff, wie z.B. Benzol, Toluol oder Xylol. Verbindungen der allgemeinen Formel X sind bekannt und lassen sich in analoger Weise, wie in der Angew. Chem., Intern. Ed. Bd. 6 (1967), S. 649 beschrieben, herstellen.

Verbindungen der allgemeinen Formel IIb, in der die abspaltbare Gruppe $X_3$ beispielsweise Halogen bedeutet, lassen sich leicht auf bekannte Weise in Verbindungen der Formel IIb umwandeln, in der $X_3$ eine Niederalkoxygruppe bedeutet.

Ausgangsverbindungen der allgemeinen Formel IIc, in der als abspaltbare Gruppe $X_1$ ein Halogen, vorzugsweise Chlor, bedeutet, lassen sich durch Umsetzung eines Immoniumchlorids der Formel

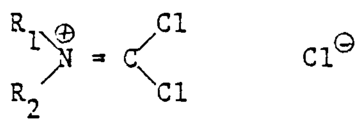

mit einer Iminoverbindung der Formel III

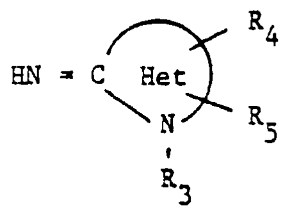

(III)

nach der von R.G. Glushkow, Khim.-Farmatsevt. Zh. 12, No. 6, 59-64/1978 beschriebenen Methode herstellen.

Die Umsetzung erfolgt in analoger Weise wie oben bei der Umsetzung einer Verbindung der Formel X beschrieben.

Ausgangsverbindungen der allgemeinen Formel VI, in der $Y_1$ beispielsweise eine Oxo- oder Thioxogruppe bedeutet und $R_3$ verschieden von Wasserstoff ist, lassen sich durch Umsetzung einer Guanidinverbindung der Formel IV

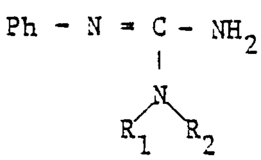

(IV)

mit einem Niederalkylisothiocyanat oder -isocyanat herstellen. Die Umsetzung erfolgt in einem organischen Lösungsmittel, vorzugsweise in einem Aether, wie z.B. Diäthyläther, Diisopropyläther, Tetrahydrofuran oder Dioxan, einem Carbonsäurederivat, wie z.B. Acetonitril, bei einer Temperatur, die zwischen 0°C und dem Siedepunkt des Lösungsmittels, vorzugsweise bei 20°C bis 100°C liegt.

Verbindungen der allgemeinen Formel VI, in der beispielsweise $Y_1$ eine Thioxogruppe bedeutet, und $R_3$ Wasserstoff ist, lassen sich nach dem von H. Hartmann et al., J. Prakt. Chem. 315 (1973), S. 144, beschriebenen Verfahren herstellen. Guanidinverbindungen der allgemeinen Formel IV werden mit einem Niederalkoxycarbonylisothiocyanat zu einer Verbindung der Formel XII

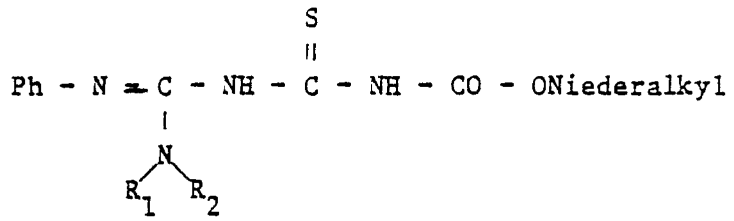

(XII)

umgesetzt, die durch Hydrolyse mit einer Mineralsäure, vorzugsweise Salzsäure zu einer Verbindung der Formel VI umgewandelt wird.

Verbindungen der allgemeinen Formel IX lassen sich nach einem für die Herstellung von Verbindungen der allgemeinen Formel I vorstehend beschriebenen ersten drei Verfahren herstellen, wobei jedoch in den verwendeten Ausgangsprodukten $R_2$ und/oder $R_3$ einen Acylrest bedeuten. Diese als Aminoschutzgruppen verwendeten Acylreste sind wie oben definiert.

Die beschriebenen Verfahren können in gewohnter Weise bei Raumtemperatur, unter Kühlen oder Erwärmen, bei Normaldruck oder erhöhtem Druck, und, wenn notwendig, in Gegenwart oder Abwesenheit eines Verdünnungsmittels, Katalysators oder Kondensationsmittels ausgeführt werden. Wenn notwendig, können die Umsetzungen auch in der Atmosphäre eines inerten Gases, wie z.B. Stickstoff, erfolgen.

In erhaltenen Verbindungen kann man im Rahmen der Definition der Endprodukte Substituenten einführen abwandeln oder abspalten.

Die neuen Ausgangsstoffe und Verfahren zu ihrer Herstellung sind ebenfalls Gegenstand der vorliegenden Erfindung.

Je nach den Verfahrensbedingungen und Ausgangsstoffen erhält man die Endstoffe in freier Form oder in der ebenfalls in der Erfindung inbegriffenen Form ihrer Salze, insbesondere Säureadditionssalze. Die Säureadditionssalze der neuen Verbindungen können in an sich bekannter Weise in die freie Verbindung übergeführt werden, z.B. mit basischen Mitteln, wie Alkalien oder Ionenaustauschern. Andererseits können die erhaltenen freien Basen mit organischen oder anorganischen Säuren Salze bilden. Zur Herstellung von Säureadditionssalzen werden insbesondere solche Säuren verwendet, die zur Bildung von therapeutisch verwendbaren Salzen geeignet sind. Als solche Säuren seien beispielsweise genannt: Halogenwasserstoffsäuren, Schwefelsäuren, Phosphorsäuren, Salpetersäure, Perchlorsäure, aliphatische, alicyclische, aromatische oder heterocyclische Carbon- oder Sulfonsäuren, wie Ameisen-, Essig-, Propion-, Bernstein-, Glykol-, Milch-, Aepfel-, Wein-, Zitronen-, Ascorbin-, Malein-, Hydroxymalein- oder Brenztraubensäure; Phenylessig-, Benzoe-, p-Aminobenzoe-, Anthranil-, p-Hydroxybenzoe-, Salicyl- oder p-Aminosalicylsäure, Embonsäure, Methansulfon-, Aethansulfon-, Hydroxyäthansulfon-, Aethylensulfonsäure; Halogenbenzolsulfon-, Toluolsulfon-, Naphthalinsulfonsäure oder Sulfanilsäure; Methionin, Trypthophan, Lysin oder Arginin.

Diese oder andere Salze der neuen Verbindungen, wie z.B. die Pikrate, können auch zur Reinigung der erhaltenen freien Basen dienen, indem man die freien Basen in Salze überführt, diese abtrennt und aus den Salzen wiederum die Basen freimacht. Infolge der engen Beziehungen zwischen den neuen Verbindungen in freier Form und in Form ihrer Salze sind im Vorausgegangenen und nachfolgend unter den freien Verbindungen sinn- und zweckmässig, gegebenenfalls auch die entsprechenden Salze zu verstehen.

Die Erfindung betrifft auch diejenigen Ausführungsformen eines Verfahrens, bei denen man ein Verfahren auf irgendeiner Stufe abbricht oder bei denen man von einer auf irgendeiner Stufe als Zwischenprodukt erhältlichen Verbindung ausgeht und die fehlenden Schritte durchführt, oder einen Ausgangsstoff unter den Reaktionsbedingungen bildet oder gegebenenfalls in Form eines Salzes verwendet. Die Erfindung beinhaltet auch daraus resultierende neue Zwischenprodukte.

Von der Erfindung ebenfalls umfasst sind therapeutische Stoffzusammenstellungen, die aus einem hypoglykämisch wirksamen Anteil der Verbindungen der allgemeinen Formel I oder einem Säureadditionssalz und einem pharmakologisch annehmbaren festen Trägerstoff oder flüssigen Verdünnungsmitteln bestehen.

Die erfindungsgemässen pharmazeutischen Präparate enthalten mindestens eine Verbindung der allgemeinen Formel I oder ein Salz davon als Wirkstoff zusammen mit einem üblichen pharmazeutischen Trägerstoff. Die Art der Trägerstoffe richtet sich weitgehend nach dem Anwendungsgebiet. Die erfindungsgemässen pharmazeutischen Stoffzusammensetzungen, die als Wirkstoffe Verbindungen der Formel I enthalten, können oral, parenteral oder rektal verabreicht werden.

Zur oralen Behandlung von Hyperglykämie kommen insbesondere feste Doseneinheitsformen, wie Tabletten, Dragées und Kapseln, in Frage, die vorzugsweise zwischen 10 und 90% eines Wirkstoffes der allgemeinen Formel I oder eines Salzes enthalten, um die Verabreichung von täglichen Dosen zwischen 1,5 bis 100 mg/kg an Warmblütern zu ermöglichen. Zur Herstellung von Tabletten und Dragéekernen kombiniert man die Verbindungen der allgemeinen Formel I mit festen, pulverförmigen Trägerstoffen, wie Lactose, Saccharose, Sorbit, Maisstärke, Kartoffelstärke oder Amylopektin, Cellulosederivaten oder Gelatine, vorzugsweise unter Zusatz von Gleitmitteln, wie Magnesium- oder Calciumstearat, oder Polyäthylenglykolen von geeignetem Molekulargewicht. Dragéekerne überzieht man anschliessend beispielsweise mit konzentrierten Zuckerlösungen, welche z.B. noch arabischen Gummi, Talk und/oder Titandioxid enthalten können, oder mit einem in leicht-flüchtigen organischen Lösungsmitteln oder Lösungsmittelgemischen gelösten Lack. Diesen Ueberzügen können Farbstoffe zugefügt werden, z.B. zur Kennzeichnung verschiedener Wirkstoffdosen. Weiche Gelatinekapseln und andere geschlossene Kapseln bestehen beispielsweise aus einem Gemisch von Gelatine und Glycerin und können

z.B. Mischungen einer Verbindung der Formel I mit Polyäthylenglykol enthalten. Steckkapseln enthalten z.B. Granulate eines Wirkstoffes mit festen, pulverförmigen Trägerstoffen, wie z.B. Lactose, Saccharose, Sorbit, Mannit; Stärken, wie Kartoffelstärke, Maisstärke oder Amylopektin, Cellulosederivate und Gelatine sowie Magnesiumstearat oder Stearinsäure.

Als Doseneinheitsformen für die rektale Anwendung kommen z.B. Suppositorien in Betracht, welche aus einer Kombination eines Wirkstoffes mit einer Suppositorien-Grundmasse auf der Basis von natürlichen oder synthetischen Triglyceriden (z.B. Kakaobutter), Polyäthylenglykolen oder geeigneten höheren Fettalkoholen bestehen, und Gelatine-Rektalkapseln, welche eine Kombination des Wirkstoffes mit Polyäthylenglykolen enthalten.

Ampullenlösungen zur parenteralen, insbesondere intramuskulären oder intravenösen Verabreichung enthalten eine Verbindung der Formel I oder ein Salz davon in einer Konzentration von vorzugsweise 0,5 bis 5% als wässrige, mit Hilfe von üblichen Lösungsvermittlern und/oder Emulgiermitteln, sowie gegebenenfalls von Stabilisierungsmitteln bereitete Dispersion, oder vorzugsweise eine wässrige Lösung eines pharmazeutisch annehmbaren, wasserlöslichen Säureadditionssalzes einer Verbindung der allgemeinen Formel I.

Für Flüssigkeiten zur oralen Einnahme, wie Sirups und Elixiere, wird die Konzentration des Wirkstoffes derart gewählt, dass eine Einzeldosis leicht abgemessen werden kann, z.B. als Inhalt eines Teelöffels oder eines Messlöffels von z.B. 5 ml, oder auch als Mehrfaches dieser Volumina.

Die nachfolgenden Beispiele a) bis e) sollen die Herstellung einiger typischer Applikationsformen erläutern, jedoch keineswegs die einzigen Ausführungsformen von solchen darstellen.

a) 250,0 g Wirkstoff werden mit 550,0 g Lactose und 292,0 g Kartoffelstärke vermischt, die Mischung mit einer alkoholischen Lösung von 8 g Gelatine befeuchtet und durch ein Sieb granuliert. Nach dem Trocknen mischt man 60,0 g Talk, 10,0 g Magnesiumstearat und 20,0 g kolloidales Siliciumdioxid zu und presst die Mischung zu 10'000 Tabletten von je 125 mg Gewicht und 25 mg Wirkstoffgehalt, die gewünschtenfalls mit Teilkerben zur feineren Anpassung der Dosierung versehen sein können.

b) Aus 100,0 g Wirkstoff, 379,0 g Lactose und der alkoholischen Lösung von 6,0 g Gelatine stellt man ein Granulat her, das man nach dem Trocknen mit 10,0 g kolloidalem Siliciumdioxid, 40,0 g Talk, 60,0 g Kartoffelstärke und 5,0 g Magnesiumstearat mischt und zu 10'000 Dragéekernen presst. Diese werden anschliessend mit einem konzentrierten Sirup aus 533,5 g krist. Saccharose, 20,0 g Schellack, 75,0 g arabischem Gummi, 250,0 g Talk, 20,0 g kolloidalem Siliciumdioxid und 1,5 g Farbstoff überzogen und getrocknet. Die erhaltenen Dragées wiegen je 150 mg und enthalten je 10 mg Wirkstoff.

c) 25,0 g Wirkstoff und 1975 g fein geriebene Suppositoriengrundmasse (z.B. Kakaobutter) werden gründlich gemischt und dann geschmolzen. Aus der durch Rühren homogen gehaltenen Schmelze werden 1000 Suppositorien von 2,0 g gegossen. Sie enthalten je 25 mg Wirkstoff.

d) Zur Bereitung eines Sirups mit 0,25% Wirkstoffgehalt löst man in 3 Litern dest. Wasser 1,5 Liter Glycerin, 42 g p-Hydroxybenzoesäuremethylester, 18 g p-Hydroxybenzoesäure-n-propylester und unter leichtem Erwärmen 25,0 g Wirkstoff, fügt 4 Liter 70%-ige Sorbitlösung, 1000 g krist. Saccharose, 350 g Glucose und einen Aromastoff, z.B. 250 g "Orange Peel Soluble Fluid" von Eli Lilly and Co., Indianapolis, oder je 5 g natürliches Zitronenaroma und 5 g "Halb und Halb"-Essenz, beide von der Firma Haarmann und Reimer, Holzminden, Deutschland, zu, filtriert die erhaltene Lösung und ergänzt das Filtrat mit dest. Wasser auf 10 Liter.

e) Zur Bereitung einer Tropflösung mit 1,5% Wirkstoffgehalt löst man 150,0 g Wirkstoff und 30 g Natriumcyclamat in einem Gemisch von 4 Liter Aethanol (96%) und 1 Liter Propylenglykol. Andererseits mischt man 3,5 Liter 70%-ige Sorbitlösung mit 1 Liter Wasser und fügt die Mischung zur obigen Wirkstofflösung. Hierauf wird ein Aromastoff, z.B. 5 g Hustenbonbon-Aroma oder 30 g Grapefruit-Essenz, beide von der Firma Haarmann und Reimer, Holzminden, Deutschland, zugegeben, das Ganze gut gemischt, filtriert und mit dest. Wasser auf 10 Liter ergänzt.

Die nachfolgenden Beispiele erläutern die Herstellung der neuen Verbindungen der allgemeinen Formel I, sollen jedoch den Umfang der Erfindung in keiner Weise beschränken. Die Temperaturen sind in Celsiusgraden angegeben.

Beispiel 1: Zu einer auf 5° gekühlten Lösung von 24,2 g (0,1 Mol) 2,5-Dimethyl-3-isoxazolidinimin-hydrojodid [vgl. Bull. Soc. Chim. Fr. 1974, 1651-1655] in 120 ml Acetonitril tropft man unter Rühren 21,25 g (0,21 Mol) Triäthylamin. Unter weiterem Kühlen gibt man portionenweise 22,47 g (0,1 Mol) N-Phenyl-4-morpholincarboximidoylchlorid [vgl. Chem. Ber. 105, 1532-1539 (1972)] zu, wobei die Reaktionstemperatur bei 5-10° gehalten wird. Nach beendeter Zugabe rührt man noch 1/2 Stunde bei 10° und eine Stunde bei Raumtemperatur. Das Reaktionsgemisch wird filtriert, der Filterrückstand mit wenig Acetonitril nachgewaschen und das Filtrat am Vakuum eingedampft. Man versetzt den Rückstand mit eiskalter 2-n. Natronlauge und extrahiert mehrmals mit Chloroform. Die vereinigten mit Wasser neutral gewaschenen Chloroformphasen werden nach Trocknen über Natriumsulfat eingedampft, wobei man rohes N-(2,5-Dimethyl-3-isoxazolidinyliden)-N'-phenyl-4-morpholincarboximidamid erhält. Das durch Umsetzen mit Fumarsäure hergestellte Fumarat schmilzt nach Umkristallisation aus Aethanol/Aethylacetat bei 192-193°.

Beispiel 2: Analog Beispiel 1 erhält man, ausgehend von 12,1 g (0,05 Mol) 2,5-Dimethyl-3-isoxazolidinimin-hydrojodid, 11,13 g (0,05 Mol) N-Phenyl-1-piperidin-carboximidoylchlorid [vgl. Organometal. Chem. Synt. 1, 23-30 (1970-71)], 10,63 g (0,105 Mol) Triäthylamin und 60 ml Acetonitril rohes N-(2,5-Dimethyl-3-isoxazolidinyliden)-N'-phenyl-1-piperidincarboximidamid. Das daraus mit Fumarsäure hergestellte Fumarat schmilzt bei 171,5-173° nach Umkristallisation aus Isopropanol/Aethylacetat.

Beispiel 3: Analog Beispiel 1 erhält man, ausgehend von 11,4 g (0,05 Mol) 2-Methyl-3-isoxazolidinimin-hydrojodid [vgl. Bull. Soc. Chim. Fr. 1974, 1651-1655], 11,23 g (0,05 Mol) N-Phenyl-4-morpholin-carboximidoylchlorid, 10,63 g (0,105 Mol) Triäthylamin und 60 ml Acetonitril rohes N-(2-Methyl-3-isoxazolidinyliden)-N'-phenyl-4-morpholincarboximidamid [Smp. 80-88°, aus Petroläther]. Das daraus mit Fumarsäure hergestellte Fumarat schmilzt bei 167-168° nach Umkristallisation aus Aethanol/Aether.

Beispiel 4: Analog Beispiel 1 erhält man, ausgehend von 12,8 g (0,05 Mol) 2,5,5-Trimethyl-3-isoxazolidinimin-hydrojodid (vgl. Bull. Soc. Chim. Fr. 1974, 1651-1655), 11,23 g (0,05 Mol) N-Phenyl-4-morpholin-carboximidoylchlorid, 10,63 g (0,105 Mol) Triäthylamin und 60 ml Acetonitril rohes N-(2,5,5-Trimethyl-3-isoxazolidinyliden)-N'-phenyl-4-morpholincarboximidamid. Das daraus mit Fumarsäure hergestellte Fumarat schmilzt bei 203-204° nach Umkristallisation aus Aethanol/Aether.

Beispiel 5: Zu einer Lösung von 12,26 g (0,05 Mol) N,N-Tetramethylen-N'-phenyl-chlorformamidiniumchlorid (bzw. Phenylimino-chlorkohlensäure-pyrrolidinylamid-hydrochlorid) [vgl. Chem. Ber. 97, 1232-1245 (1964)] und 12,1 g (0,05 Mol) 2,5-Dimethyl-3-isoxazolidinimin-hydrojodid in 50 ml Acetonitril tropft man unter Rühren und Kühlen 15,18 g (0,15 Mol) Triäthylamin, wobei die Reaktionstemperatur bei 5-10° gehalten wird.

Das Reaktionsgemisch wird noch 15 Stunden bei Raumtemperatur gerührt und anschliessend filtriert. Man dampft das Filtrat im Vakuum ein und filtriert den Rückstand mit Chloroform über Kieselgel der Korngrösse 0,063 - 0,200 mm. Die das gewünschte Produkt enthaltenden Fraktionen werden vereinigt und mit eiskalter Natronlauge und Wasser gewaschen. Man trocknet die Chloroformphase über Natriumsulfat und dampft am Vakuum ein, wobei man rohes N-(2,5-Dimethyl-3-isoxazolidinyliden)-N'-phenyl-1-pyrrolidincarboximidamid erhält. Das daraus mit Maleinsäure hergestellte Maleat schmilzt bei 114-116° nach Umkristallisation aus Aethylacetat/Isopropanol.

Beispiel 6:

a) Man versetzt unter Rühren 200 ml Isopropanol mit 19,5 g (0,05 Mol) N-(1,3-Dimethyl-2-imidazolidinyliden)-N'-phenyl-carbamimido-thiomethylester-hydrojodid und 10,66 g (0,15 Mol) Pyrrolidin. Das Reaktionsgemisch wird 36 Stunden unter Rückfluss gekocht und anschliessend eingedampft. Den Rückstand verteilt man zwischen Chloroform und 1-n. Natronlauge. Die mit Wasser gewaschene und über Natriumsulfat getrocknete organische Phase wird eingedampft und der Rückstand mit einem Gemisch Chloroform/Methanol/konz. Ammoniak = 40:10:1 über Kieselgel der Korngrösse 0,063 - 0,200 mm filtriert. Die das gewünschte Produkt enthaltenden Fraktionen dampft man im Vakuum ein. Das so erhaltene rohe N-(1,3-Dimethyl-2-imidazolidinyliden)-N'-phenyl-1-pyrrolidincarboximidamid wird mit Maleinsäure in das Maleat übergeführt, welches bei 126-127° schmilzt nach Umkristallisation aus Aethylacetat/Isopropanol.

Die Ausgangsverbindung, N-(1,3-Dimethyl-2-imidazolidinyliden)-N'-phenylcarbamimido-thiomethylester-hydrojodid, wird wie folgt hergestellt:

b) Zu einer Lösung von 44,1 g (0,5 Mol) N,N'-Dimethyläthylendiamin in 300 ml Toluol tropft man unter Rühren während 1 Stunde eine Lösung von 52,9 g Bromcyan in 300 ml Toluol. Das Reaktionsgemisch wird noch zwei Stunden bei 80° gerührt. Man lässt auf Raumtemperatur abkühlen, filtriert und wäscht den Rückstand mit Aether. Das nach Trocknen am Vakuum erhaltene rohe 1,3-Dimethyl-2-imino-imidazolidin-hydrobromid schmilzt bei 153-160°.

c) Eine Suspension von 58,2 g (0,3 Mol) 1,3-Dimethyl-2-imino-imidazolidin-hydrobromid in 470 ml Chloroform wird unter Rühren bei 5° mit 30,4 g (0,3 Mol) Triäthylamin versetzt. Man tropft dann eine Lösung von 40,6 g (0,3 Mol) Phenylisothiocyanat in 235 ml Chloroform zu und kocht das Reaktionsgemisch während 15 Stunden unter Rückfluss. Nach dem Abkühlen wird es mit Wasser gewaschen, über Natriumsulfat getrocknet und am Vakuum eingedampft. Man erhält rohen N-(1,3-Dimethyl-2-imidazolidinyliden)-N'-phenyl-thioharnstoff, der nach Umkristallisation aus Aethanol bei 195-196° schmilzt.

d) Zu einer Suspension von 24,8 g (0,1 Mol) N-(1,3-Dimethyl-2-imidazolidinyliden)-N'-phenyl-thioharnstoff in 165 ml Tetrahydrofuran wird unter Rühren bei Raumtemperatur eine Lösung von 21,3 g (0,15 Mol) Methyljodid in 35 ml Tetrahydrofuran zugetropft. Man rührt 15 Stunden bei Raumtemperatur und versetzt dann das Reaktionsgemisch mit 160 ml Aether. Der Niederschlag wird abfiltriert, mit Aether gewaschen und am Vakuum getrocknet. Das erhaltene N-(1,3-Dimethyl-2-imidazolidinyliden)-N'-phenylcarbamimido-thiomethylester-hydrojodid schmilzt bei 157-158°.

<u>Beispiel 7:</u>

a) Eine Lösung von 19,6 g N-[3-Methyl-4-(1,3,4)-thiadiazolin-2-yliden]-N'-phenylcarbamimido-thiomethylester-hydrojodid und 10,7 g Pyrrolidin in 150 ml Isopropanol wird 15 Stunden unter Rückfluss erhitzt. Anschliessend wird das Reaktionsgemisch zur Trockne eingedampft und der Rückstand über eine kurze, mit Kieselgel gefüllte Säule chromatographiert. Man eluiert zuerst mit Chloroform und dann mit einem Chloroform/Aceton-Gemisch (95:5). Die letzten Fraktionen werden vereinigt, mit Aether verrührt und das so erhaltene Hydrojodid mit Hilfe eines Ionenaustauschers in das Hydrochlorid übergeführt. Das erhaltene N-[3-Methyl-4-(1,3,4)-thiadiazolin-2-yliden]-N'-phenyl-1-pyrrolidin-carboximidamid-hydrochlorid schmilzt bei 217-218° (Zersetzung).

Das als Ausgangsverbindung verwendete N-[3-Methyl-4-(1,3,4)-thiadiazolin-2-yliden]-N'-phenylcarbamimido-thiomethylester-hydrojodid wird wie folgt erhalten:

b) Eine Suspension von 52,7 g 2-Imino-3-methyl-4-(1,3,4)-thiadiazolin-hydrojodid und 24,3 g Kalium-tert.-butylat in 500 ml Tetrahydrofuran wird 1 Stunde bei Raumtemperatur gerührt. Anschliessend wird durch ein Bett von Diatomenerde filtriert. Zum Filtrat tropft man innert 5 Minuten 29,3 g Phenylisothiocyanat und rührt 16 Stunden bei Raumtemperatur. Dann wird das Reaktionsgemisch zur Trockne eingedampft, der Rückstand mit Aether verrührt, abgenutscht und nochmals mit heissem Isopropanol verrührt. Man erhält so den N-[3-Methyl-4-(1,3,4)-thiadiazolin-2-yliden]-N'-phenylthioharnstoff, Smp. 174-175°.

c) Eine Lösung von 25,5 g der in b) erhaltenen Verbindung in 300 ml Acetonitril wird mit 43,4 g Methyljodid versetzt. Die Lösung wird 30 Minuten unter Rückfluss erhitzt. Die beim Abkühlen ausgeschiedenen Kristalle werden abgenutscht, zuerst mit Acetonitril, dann mit Aether gewaschen und getrocknet. Es wird so das N-[3-Methyl-4-(1,3,4)-thiadiazolin-2-yliden]-N'-phenylcarbamimido-thiomethylester-hydrojodid erhalten, Smp. 190°(Zersetzung).

Beispiel 8:

a) Eine Lösung von 17,6 g 2-Amino-5-methyl-1,3,4-thiadiazol in 350 ml Isopropanol wird mit 65,1 g Methyljodid versetzt. Die Lösung wird 15 Stunden unter Rückfluss erhitzt. Anschliessend wird das Reaktionsgemisch zur Trockne eingedampft, der kristalline Eindampfrückstand mit Essigester angerührt und das Lösungsmittel abgenutscht. Der kristalline Rückstand wird zuerst mit Essigester, dann mit Aether gewaschen und anschliessend getrocknet. Man erhält so 2-Imino-3,5-dimethyl-4-(1,3,4)-thiadiazolin-hydrojodid vom Smp. 208° (Zers.).

Analog können folgende Salze der 2-Iminoverbindungen hergestellt werden:

b) 2-Imino-3-methyl-5-äthyl-4-(1,3,4)-thiadiazolin-hydrojodid, Smp. 137-138° (Zers.)

c) 2-Imino-3-methyl-5-trifluormethyl-4-(1,3,4)-thiadiazolin-hydrojodid, Smp. 187-188° (Zers.)

d) 2-Imino-3-methyl-5-methylmercapto-4-(1,3,4)-thiadiazolin-hydrojodid, Smp. 205-206°

e) 2-Imino-3-methyl-4-phenyl-4-thiazolin-hydrojodid, Smp. 235° (Zers.)

f) 2-Imino-3,5-dimethyl-4-thiazolin-hydrojodid, Smp. 187-188°

g) 2-Imino-1,3-dimethyl-4-imidazolin-hydrojodid, Smp. 126-127°

h) 2-Imino-3-methyl-4-oxazolin-hydrojodid, Smp. 191-192° (Zers.)

i) 2-Imino-3,4,5-trimethyl-4-oxazolin-hydrojodid, Smp. 169-171°

j) 2-Imino-3,5-dimethyl-4-(1,3,4)-oxadiazolin-hydrojodid, Smp. 169° (Zers.).

Beispiel 9: Gemäss Beispiel 7 b), jedoch unter Verwendung einer äquivalenten Menge der in Beispiel 8 erhaltenen 2-Iminoverbindung, werden bei der Umsetzung mit einer äquivalenten Menge Phenylisothiocyanat folgende Thioharnstoffe erhalten:

a) N-[3,5-Dimethyl-4-(1,3,4)-thiadiazolin-2-yliden]-N'-phenylthioharnstoff, Smp. 175-176°

b) N-[3-Methyl-5-äthyl-4-(1,3,4)-thiadiazolin-2-yliden]-N'-phenylthioharnstoff, Smp. 135-136°

c) N-[3-Methyl-5-methylmercapto-4-(1,3,4)-thiadiazolin-2-yliden]-N'-phenylthioharnstoff, Smp. 160-161°

d) N-(3-Methyl-4-thiazolin-2-yliden)-N'-phenylthioharnstoff, Smp. 174-175°

e) N-(3-Methyl-4-phenyl-4-thiazolin-2-yliden)-N'-phenylthioharnstoff, Smp. 183-184°

f) N-(3,5-Dimethyl-4-thiazolin-2-yliden)-N'-phenylthioharnstoff, Smp. 185-186°

g) N-(1,3-Dimethyl-4-imidazolin-2-yliden)-N'-phenylthioharnstoff, Smp. 221-222°

h) N-(3-Methyl-4-oxazolin-2-yliden)-N'-phenylthioharnstoff, Smp. 169-170°

i) N-(3,4,5-Trimethyl-4-oxazolin-2-yliden)-N'-phenylthioharnstoff, Smp. 192-192,5°

In analoger Weise werden hergestellt aus:

j) 2-Imino-3-allyl-4-thiazolin-hydrojodid und Phenylisocyanat der N-(3-Allyl-4-thiazolin-2-yliden)-N'-phenylthioharnstoff, Smp. 129,5-130,5° und

k) 2-Imino-3-methyl-thiazolidin-hydrojodid und Phenylisocyanat der N-(3-Methyl-thiazolidin-2-yliden)-N'-phenylthioharnstoff, Smp. 170-172°.

<u>Beispiel 10</u>: Gemäss Beispiel 7 c), jedoch unter Verwendung der vorstehend in Beispiel 9 aufgeführten Thioharnstoffe in jeweils spezifischen Lösungsmitteln, erhält man die Methylester-hydrojodide:

a) in Isopropanol das N-[3,5-Dimethyl-4-(1,3,4)-thiadiazolin-2-yliden]-N'-phenylcarbamimido-thiomethylester-hydrojodid, Smp. 134-135,5°;

b) in Tetrahydrofuran das N-[3-Methyl-5-äthyl-4-(1,3,4)-thiadiazolin-2-yliden]-N'-phenylcarbamimido-thiomethylester-hydrojodid, rotes Oel;

c) in Tetrahydrofuran das N-(3-Methyl-5-methylmercapto-4-(1,3,4)-thiadiazolin-2-yliden]-N'-phenylcarbamimido-thiomethylester-hydrojodid, Smp. 160-161°;

d) in Tetrahydrofuran das N-(3-Methyl-4-thiazolin-2-yliden)-N'-phenylcarbamimido-thiomethylester-hydrojodid, Smp. 174-175°;

e) in Tetrahydrofuran das N-(3-Methyl-4-phenyl-4-thiazolin-2-yliden)-N'-phenylcarbamimido-thiomethylester-hydrojodid, Smp. 183-184°;

f) in Tetrahydrofuran das N-(3,5-Dimethyl-4-thiazolin-2-yliden)-N'-phenylcarbamimido-thiomethylester-hydrojodid, Smp. 185-186°;

g) in Acetonitril das N-(1,3-Dimethyl-4-imidazolin-2-yliden)-N'-phenylcarbamimido-thiomethylester-hydrojodid, Smp. 221-222°;

h) in Isopropanol das N-(3-Methyl-4-oxazolin-2-yliden)-N'-phenylcarbamimido-thiomethylester-hydrojodid, Smp. 140-141°;

i) in Isopropanol das N-(3,4,5-Trimethyl-4-oxazolin-2-yliden)-N'-phenylcarbamimido-thiomethylester-hydrojodid, Smp. 192-192,5°;

j) in Isopropanol das N-(3-Allyl-4-thiazolin-2-yliden)-N'-phenylcarbamimido-thiomethylester-hydrojodid, Smp. 138-139°;

k) in Tetrahydrofuran das N-(3,4,5-Trimethyl-4-oxazolin-2-yliden)-N'-phenylcarbamimido-thiomethylester-hydrojodid, Smp. 139-141°.

Beispiel 11: Gemäss Beispiel 7 a) werden die entsprechenden Methylthio-hydrojodide von Beispiel 10 sowie die entsprechenden Amine der allgemeinen Formel $HNR_1R_2$ in einem Molverhältnis von 1:3 in unter Rückfluss kochendem Isopropanol, tert.-Butanol oder auch Aethanol zu den nun folgenden Verbindungen a - k in Form der Hydrojodide umgesetzt. Die Hydrojodide werden als solche isoliert, mit Hilfe eines Anionenaustauscherharzes in die Hydrochloride übergeführt oder gegebenenfalls mit wässrigem Alkali in die freien Basen übergeführt. Durch Umsetzung der freien Basen mit einer geeigneten Säure können auch andere Salze hergestellt werden.

a) das N-[3,5-Dimethyl-4-(1,3,4)-thiadiazolin-2-yliden]-N'-phenyl-1-pyrrolidin-carboximidamid als freie Base, Smp. 98-99°;

b) das N-(3-Methyl-5-äthyl-4-(1,3,4)-thiadiazolin-2-yliden]-N'-phenyl-1-pyrrolidin-carboximidamid als freie Base, Smp. 85-86°;

c) das N-(3-Methyl-5-methylmercapto-4-(1,3,4)-thiadiazolin-2-yliden]-N'-phenyl-1-pyrrolidin-carboximidamid als freie Base, Smp. 98-99°;

d) das N-(3-Methyl-4-thiazolin-2-yliden)-N'-phenyl-1-pyrrolidin-carboximidamid als freie Base, Smp. 129-130°;

e) das N-(3-Methyl-4-phenyl-4-thiazolin-2-yliden)-N'-phenyl-1-pyrrolidin-carboximidamid als Hydrochlorid, Smp. 196-197°;

f) als N-(3,5-Dimethyl-4-thiazolin-2-yliden)-N'-phenyl-1-pyrrolidin-carboximidamid als Hydrochlorid, Smp. 220,5-221,5°;

g) als N-(1,3-Dimethyl-4-imidazolin-2-yliden)-N'-phenyl-1-pyrrolidin-carboximidamid · 2HCl, Smp. 170° (Zersetzung);

h) das N-(3-Methyl-4-oxazolin-2-yliden)-N'-phenyl-1-pyrrolidin-carboximidamid als freie Base, Smp. 94,5-95,5°;

i) das N-(3,4,5-Trimethyl-4-oxazolin-2-yliden)-N'-phenyl-1-pyrrolidin-carboximidamid als Hydrochlorid, Smp. 161-163°;

j) das N-(3-Allyl-4-thiazolin-2-yliden)-N'-phenyl-1-pyrrolidin-carboximidamid als freie Base, Smp. 91-92°;

k) das N-(3,4,5-Trimethyl-4-oxazolin-2-yliden)-N'-phenyl-1-pyrrolidin-carboximidamid als Hydrochlorid, Smp. 225-226°;

l) das N-[3,5-Dimethyl-4-(1,3,5)-thiadiazolin-2-yliden]-N'-phenyl-dimethylamino-carboximidamid als Hydrochlorid, Smp. 238-239,5° (Zersetzung);

m) das N-(3,4,5-Trimethyl-4-oxazolin-2-yliden)-N'-phenyl-dimethylamino-carboximidamid als freie Base, Smp. 84-86°.

Beispiel 12: Eine Lösung von 9,3 g 2-Imino-3-methyl-thiazolidin- und 10,3 g N-Aethyldiisopropylamin in 50 ml Acetonitril wird innerhalb 15 Minuten bei Raumtemperatur zu einer Lösung von 14,8 g N-Phenyl-4-morpholin-carboximidoylchlorid in 50 ml Acetonitril getropft. Anschliessend wird 2 Stunden bei Raumtemperatur und dann noch 3 Stunden unter Rückfluss gerührt. Das Reaktionsgemisch wird zur Trockne eingedampft, der kristalline Rückstand mit Isopropanol angerührt, das Lösungsmittel abgenutscht und das erhaltene Festprodukt dann während 30 Minuten mit 50 ml Wasser verrührt, abgenutscht, zuerst mit Wasser, dann mit Isopropanol gewaschen und getrocknet. Das so erhaltene N-(3-Methyl-thiazolidin-2-yliden)-N'-phenyl-4-morpholin-carboximidamid schmilzt bei 144,5-145,5°.

Beispiel 13: Aus 18,5 g 2-Imino-3-methyl-5-trifluormethyl-4-(1,3,4)-thiadiazolin-hydrojodid wird mit 10%iger Natronlauge die Base freigesetzt, in Methylenchlorid aufgenommen, mit wasserfreiem Magnesiumsulfat getrocknet und eingedampft. Die so erhaltene Base und 7,6 g N-Aethyldiisopropylamin werden in 50 ml Acetonitril gelöst und innerhalb von 15 Minuten zu einer Lösung von 11,9 g N-Phenyl-4-morpholin-carboximidoylchlorid in 50 ml Acetonitril getropft. Anschliessend wird 4 Stunden bei Raumtemperatur gerührt, das Reaktionsgemisch dann zur Trockne eingedampft, der Rückstand mit Aether verrührt, abgenutscht, der Aetherauszug eingedampft und der Eindampfrückstand über eine kurze, mit Kieselgel gefüllte Säule chromatographiert. Man eluiert zuerst mit Methylenchlorid, dann mit einem Methylenchlorid-Aceton-Gemisch 97:3. Die mittleren Fraktionen werden vereinigt, mit wenig Hexan verrührt, abgenutscht, mit Hexan gewaschen und getrocknet. Das so erhaltene N-[3-Methyl-5-trifluormethyl-4-(1,3,4)-thiadiazolin-2-yliden]-N'-phenyl-4-morpholin-carboximidamid schmilzt bei 110,5-112°.

Beispiel 14: Aus 14,6 g 2-Imino-3-methyl-4-(1,3,4)-thiadiazolin-hydrojodid wird mit 30%iger Natronlauge die Base freigesetzt, in Methylenchlorid aufgenommen, mit wasserfreiem Magnesiumsulfat getrocknet und eingedampft. Die so erhaltene Base und 7,1 g N-Aethyldiisopropylamin werden in 50 ml Acetonitril gelöst und innerhalb 15 Minuten zu einer Lösung von 11,2 g N-Phenyl-4-morpholin-carboximidoylchlorid in 50 ml Acetonitril getropft. Anschliessend wird 1 Stunde bei Raumtemperatur und dann noch 3 Stunden unter Rückfluss gerührt. Das Reaktionsgemisch wird zur Trockne eingedampft und der Eindampfrückstand über eine mit Kieselgel gefüllte Säule chromatographiert. Man eluiert mit Chloroform. Die so erhaltene Base wird mit alkoholischer Salzsäure in das Hydrochlorid übergeführt. Nach Umkristallisation aus Acetonitril-Essigester erhält man reines N-[3-Methyl-4-(1,3,4)-thiadiazolin-2-yliden]-N'-phenyl-4-morpholin-carboximidamid-hydrochlorid vom Smp. 201,5-202,5°.

Beispiel 15: Nach dem im Beispiel 14 beschriebenen Verfahren erhält man aus 8,6 g 2-Imino-3-methyl-4-(1,3,4)-thiadiazolin und 16,7 g N-Phenyl-1-piperidin-carboximidoylchlorid das N-[3-Methyl-4-(1,3,4)-thiadiazolin-2-yliden]-N'-phenyl-1-piperidin-carboximidamid, Smp. 94,5-95,5°.

Beispiel 16: Nach dem im Beispiel 14 beschriebenen Verfahren erhält man aus 6,3 g 2-Imino-3-methyl-4-thiazolin und 11,2 g N-Phenyl-4-morpholin-carboximidoylchlorid das N-(3-Methyl-4-thiazolin-2-yliden)-N'-phenyl-4-morpholin-carboximidamid-hydrochlorid, Smp. 209-210°.

Beispiel 17: 12,0 g 2-Imino-3,5-dimethyl-4-(1,3,4)-oxadiazolin-hydrojodid werden mit 11,2 g N-Phenyl-4-morpholin-carboximidoylchlorid, wie in Beispiel 8 beschrieben, umgesetzt. Das Reaktionsgemisch wird jedoch nur bei Raumtemperatur, und zwar während 16 Stunden gerührt. Das Reaktionsgemisch wird eingedampft und über eine mit Kieselgel gefüllte Säule chromatographiert. Man eluiert zuerst mit Chloroform, dann mit einem Chloroform-Aceton-Gemisch 95:5 und zum Schluss mit Chloroform-Methanol 95:5. Aus den letzten Fraktionen gewinnt man ein Hydrochlorid, welches mit 30%iger Natronlauge verrührt wird. Die zuerst ölige Base kristallisiert bald. Das so erhaltene N-[3,5-Dimethyl-4-(1,3,4)-oxadiazolin-2-yliden]-N'-phenyl-4-morpholin-carboximidamid schmilzt bei 117-118°.

Beispiel 18: Zu 200 ml Tetrahydrofuran und 111 ml einer 20%-igen Lösung von Phosgen in Toluol (0,21 Mol) gibt man unter Rühren 43,3 g (0,21 Mol) N-Phenyl-N',N'-tetramethylenthioharnstoff [ vergl. J. Org. Chem. 23, 1760-1764 (1958)], wobei darauf geachtet wird, dass die Temperatur nicht über 35° steigt. Man rührt noch 15 Minuten bei 20°, kühlt dann auf 10° ab und versetzt das Reaktionsgemisch (Suspension von N,N-Tetramethylen-N'-phenyl-chlorformamidinium-chlorid) mit 100 ml Acetonitril und 50,8 g (0,21 Mol) 2,5-Dimethyl-3-isoxazolidinimin-hydroiodid. Nach Zutropfen von 64,8 g (0,64 Mol) Triäthylamin bei 10-15° wird die Suspension noch 12 Stunden bei 20° gerührt. Man filtriert, dampft das Filtrat am Vakuum ein und löst der Rückstand in eiskaltes 2-n. Salzsäure. Das rohe N-(2,5-Dimethyl-3-isoxazolidinyliden)-N'-phenyl-1-pyrrolidincarboximidamid-hydrochlorid wird mit Chloroform extrahiert und der Chloroformextrakt mit 2-n. Natronlauge und Wasser

gewaschen, über Natriumsulfat getrocknet und eingedampft. Man erhält rohes N-(2,5-Dimethyl-3-isoxazolidinyliden)-N'-phenyl-1-pyrrolidincarboximidamid.
Das daraus mit Maleinsäure hergestellte Maleat schmilzt bei 114-116° (aus Aethylacetat).

Variante:
Eine weitere Vereinfachung des Verfahrens besteht darin, dass man zu einer Lösung von Pyrrolidin in Aether oder Tetrahydrofuran Phenylisothiocyanat zutropft, in die entstandene Suspension von N-Phenyl-N',N'-tetramethylenthioharnstoff eine äquivalente Menge Phosgen einleitet und das Reaktionsgemisch wie oben beschrieben, weiterbehandelt.

Beispiel 19: Zu einer Lösung von 8,7 g (0,05 Mol) Phenylisocyaniddichlorid in 120 ml Acetonitril wird unter Rühren bei -10° bis 0° ein Gemisch von 3,6 g (0,05 Mol) Pyrrolidin und 15,2 g (0,45 Mol) Triäthylamin zugetropft. Man rührt das Reaktionsgemisch noch 15 Minuten bei 0° und gibt dann 12,1 g (0,05 Mol) 2,5-Dimethyl-3-isoxazolidiniminhydroiodid zu, wobei darauf geachtet wird, dass die Temperatur nicht über 10° steigt. Die Suspension wird 1 Stunde bei 20° gerührt. Man filtriert, dampft das Filtrat ein und verteilt den Rückstand zwischen Chloroform und 2-n. Natronlauge. Die mit Wasser neutral gewaschene und über Natriumsulfat getrocknete Chloroformphase wird eingedampft, wobei man rohes N-(2,5-Dimethyl-3-isoxazolidinyliden)-N'-phenyl-1-pyrrolidincarboximidamid erhält. Das daraus mit Maleinsäure hergestellte rohe Maleat reinigt man durch Filtration über Kieselgel der Korngrösse 0,063-0,2 mm mit einem Lösungsmittelgemisch Chloroform/Methanol = 95:5. Das reine N-(2,5-Dimethyl-3-isoxazolidinyliden)-N'-phenyl-1-pyrrolidincarboximidamidmaleat schmilzt nach Umkristallisation aus Aethylacetat bei 112-114°.

Beispiel 20: Eine Suspension von 6,6 g N-Phenyl-4-morpholincarboximidamid-hydrojodid in 40 ml Acetonitril wird mit 3,5 g 3-Aethoxy-5,6-dihydro-2H-1,4-thiazin gemischt. Das Gemisch wird auf dem Wasserbad 12 Stunden unter kräftigem Rühren erhitzt. Das Acetonitril wird unter vermindertem Druck abgedampft und der Rückstand aus einem Gemisch von Aceton-Essigsäureäthylester umkristallisiert. Man erhält das N-(Thiomorpholin-3-yliden)-N'-phenyl-4-morpholincarboximidamid-hydrojodid der Formel

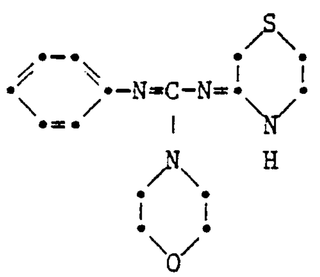

• HJ ,

welches bei 220° schmilzt.

Die entsprechende freie Base wird wie folgt hergestellt: Eine Suspension von 4,3 g des obigen Hydrojodids in 50 ml Methylenchlorid wird mit 10 ml 10%iger wässriger Natriumhydroxydlösung versetzt. Die organische Phase wird abgetrennt,und nach Eindampfen unter vermindertem Druck erhält man die freie Base, welche nach Umkristallisation aus einem Gemisch von Essigsäureäthylester-Hexan bei 80-81° schmilzt.

Die folgenden Salze werden wie beschrieben hergestellt:

p-Toluolsulfonat:

Eine Lösung von 3 g der freien Base in 30 ml Methylenchlorid-Aceton wird mit 1,8 g p-Toluolsulfonsäure unter Rühren versetzt. Das Produkt scheidet sich in Form von farblosen Kristallen aus, welche aus einem Gemisch von Isopropanol-Essigsäureäthylester umkristallisiert werden. Man erhält das N-(Thiomorpholin-3-yliden)-N'-phenyl-4-morpholincarboximidamid-p-toluolsulfonat, welches bei 172° schmilzt.

Hydrochlorid:

Eine Lösung von 5 g der freien Base in 40 ml Isopropanol wird mit Chlorwasserstoffsäure in Isopropanol angesäuert. Das Lösungsmittel wird unter vermindertem Druck abgedampft und der Rückstand aus einem Gemisch von Isopropanol-Aceton umkristallisiert. Man erhält das N-(Thiomorpholin-3-yliden)-N'-phenyl-4-morpholincarboximidamid-hydrochlorid, welches bei 228° schmilzt.

Sulfat:

Eine Lösung von 3,1 g der freien Base in 30 ml Methylenchlorid-Aceton wird unter Rühren mit 2 g Schwefelsäure in Methylenchlorid versetzt. Das Produkt scheidet sich in Form von weissen Kristallen aus, welche aus einem Gemisch von Isopropanol-Essigsäureäthylester umkristallisiert werden. Man erhält das N-(Thiomorpholin-3-yliden)-N'-phenyl-4-morpholincarboximidamid-sulfat, welches bei 220° schmilzt.

Tartrat:

Eine Lösung von 3,2 g der freien Base in 40 ml Aceton wird mit 2,1 g vorher gereinigter und getrockneter d-Weinsäure in Aceton versetzt. Das ausgeschiedene Produkt wird mehrmals mit Diäthyläther und Aceton gewaschen. Der Rückstand wird aus Aceton umkristallisiert. Man erhält das N-(Thiomorpholin-3-yliden)-N'-phenyl-4-morpholincarboximidamid-tartrat, welches bei 102° schmilzt.

Methansulfonat:

Eine Lösung von 3,2 g der freien Base in 30-40 ml Methylenchlorid-Aceton wird mit 1,2 g Methansulfonsäure in Methylenchlorid versetzt. Das Produkt scheidet sich in Form von weissen Kristallen aus, welche aus einem Gemisch von Aethanol-Aceton umkristallisiert werden. Man erhält das N-(Thiomorpholin-3-yliden)-N'-phenyl-4-morpholincarboximidamid-methansulfonat, welches bei 182° schmilzt.

Beispiel 21: Eine Suspension von 8,1 g N,4-Diphenyl-1-piperidincarboximidamid-hydrojodid in 35 ml Acetonitril wird mit 4 g 3-Aethoxy-5,6-dihydro-2H-1,4-thiazin versetzt. Das Gemisch wird 14 Stunden auf dem Wasserbad erhitzt und stark gerührt. Das Acetonitril wird unter vermindertem Druck abgedampft und der Rückstand unter Triturieren mit Essigsäureäthylester kristallisiert. Das Produkt wird aus Aceton-Essigsäureäthylester umkristallisiert. Man erhält das N-(Thiomorpholin-3-yliden)-N',4-diphenyl-1-piperidincarboximidamid-hydrojodid, welches bei 210° schmilzt.

Der Ausgangsstoff für die obige Synthese wird wie folgt hergestellt:

Eine Suspension von 29 g N-Phenyl-S-methyl-isothioharnstoff-hydrojodid in 100 ml Acetonitril wird mit 20 g 4-Phenyl-piperidin versetzt. Das Gemisch wird 15 Stunden unter Rückfluss gekocht und dann das Acetonitril unter vermindertem Druck abgedampft. Der Rückstand wird aus einem Gemisch von Methylenchlorid-Aceton umkristallisiert. Man erhält das N,4-Diphenyl-1-piperidincarboximidamid-hydrojodid, welches bei 151° schmilzt.

Beispiel 22: Eine Suspension von 6,5 g N-Phenyl-4-morpholincarboximidamid-hydrojodid in 35 ml Acetonitril wird mit 4 g 3-Aethoxy-5,6-dihydro-2H-1,4-oxazin unter Rühren versetzt. Das Gemisch wird auf dem Wasserbad unter kräftigem Rühren 12 Stunden erhitzt. Das Acetonitril wird unter vermindertem Druck abgedampft und der Rückstand aus einem Gemisch von Essigsäureäthylester-Aceton umkristallisiert. Man erhält das N-(Morpholin-3-yliden)-N'-phenyl-4-morpholincarboximidamid-hydrojodid, welches bei 228° schmilzt.

Beispiel 23: Eine Suspension von 9,8 g N-Phenyl-4-piperidincarboximidamid-hydrojodid in 40 ml Acetonitril wird unter Rühren mit 5,5 g 3-Aethoxy-5,6-dihydro-2H-1,4-oxazin versetzt. Das Gemisch wird unter heftigem Rühren 12 Stunden auf dem Wasserbad erhitzt. Das Acetonitril wird unter vermindertem Druck abgedampft und der Rückstand aus einem

Gemisch von Isopropanol-Essigsäureäthylester umkristallisiert. Man erhält das N-(Morpholin-3-yliden)-N'-phenyl-4-piperidincarboximidamid-hydrojodid, welches bei 202° schmilzt.

Beispiel 24: Eine Suspension von 7,2 g N-(4-Methoxy-phenyl)-4-morpholincarboximidamid-hydrojodid in 35 ml Acetonitril wird mit 4 g 3-Aethoxy-5,6-dihydro-2H-1,4-thiazin unter Rühren versetzt. Das Gemisch wird 12 Stunden auf dem Wasserbad unter kräftigem Rühren erhitzt. Das Acetonitril wird unter vermindertem Druck abgedampft und der Rückstand aus einem Gemisch von Isopropanol-Essigsäureäthylester umkristallisiert. Man erhält das N-(Thiomorpholin-3-yliden)-N'-(4-methoxy-phenyl)-4-morpholincarboximidamid-hydrojodid, welches bei 172° schmilzt.

Beispiel 25: Eine Suspension von 3,6 g N-Phenyl-4-(2,6-dimethyl-morpholin)-carboximidamid-hydrojodid in 15 ml Acetonitril wird unter Rühren mit 1,8 g 3-Aethoxy-5,6-dihydro-2H-1,4-oxazin versetzt. Das Gemisch wird unter Rühren 16 Stunden unter Rückfluss gekocht. Das Acetonitril wird unter vermindertem Druck abgedampft und der Rückstand aus Isopropanol-Essigsäureäthylester umkristallisiert. Man erhält das N-(Morpholin-3-yliden)-N'-phenyl-2,6-dimethyl-4-morpholincarboximid-amid-hydrojodid, welches bei 230° schmilzt.

Beispiel 26: Eine Suspension von 3,5 g N-Phenyl-4-(2,6-dimethyl-morpholin)-carboximidamid-hydrojodid in 15 ml Acetonitril wird unter Rühren mit 2 g 3-Aethoxy-5,6-dihydro-2H-1,4-thiazin versetzt. Das Gemisch wird unter Rückfluss 16 Stunden unter Rückfluss gekocht. Das Acetonitril wird unter vermindertem Druck abgedampft und der Rückstand aus einem Gemisch von Aceton-Essigsäureäthylester umkristallisiert. Man erhält das N-(Thiomorpholin-3-yliden)-N'-phenyl-2,6-dimethyl-4-morpholincarboximidamid-hydrojodid, welches bei 246° schmilzt.

Beispiel 27: Eine Suspension von 6,2 g N-Phenyl-1-pyrrolidincarboximidamid-hydrojodid in 30 ml Acetonitril wird mit 3,8 g 3-Aethoxy-5,6-dihydro-2H-1,4-oxazin unter Rühren versetzt. Das Gemisch wird auf dem Wasserbad 12 Stunden stark gerührt. Das Acetonitril wird unter vermindertem Druck eingedampft und der Rückstand aus einem Gemisch von Methylenchlorid-Essigsäureäthylester umkristallisiert. Man erhält das N-(Morpholin-3-yliden)-N'-phenyl-1-pyrrolidincarboximidamid-hydrojodid, welches bei 226° schmilzt.

Beispiel 28: Eine Suspension von 3,5 g N-(4-Fluorphenyl)-4-morpholincarboximidamid-hydrojodid in 15 ml Acetonitril wird unter Rühren mit 2 g 3-Aethoxy-5,6-dihydro-2H-1,4-thiazin versetzt. Das Gemisch wird auf dem Wasserbad 12 Stunden unter starkem Rühren erhitzt. Das Acetonitril wird unter vermindertem Druck abgedampft und der Rückstand aus einem Gemisch von Aceton-Essigsäureäthylester umkristallisiert. Man erhält das N-(Thiomorpholin-3-yliden)-N'-(4-fluorphenyl)-4-morpholincarboximidamid-hydrojodid, welches bei 212° schmilzt.

Beispiel 29: Eine Suspension von 3,6 g N-(4-Fluorphenyl)-4-morpholincarboximidamid-hydrojodid in 15 ml Acetonitril wird unter Rühren mit 2 g 3-Aethoxy-5,6-dihydro-2H-1,4-oxazin unter Rühren versetzt. Das Gemisch wird auf dem Wasserbad 12 Stunden unter kräftigem Rühren erhitzt. Das Acetonitril wird unter vermindertem Druck abgedampft und der Rückstand aus einem Gemisch von Isopropanol-Essigsäureäthylester umkristallisiert. Man erhält das N-(Morpholin-3-yliden)-N'-(4-fluorphenyl)-4-morpholincarboximidamid-hydrojodid, welches bei 215° schmilzt.

Beispiel 30: Eine Suspension von 8 g N'-(4-Methyl-morpholin-3-yliden)-N-phenyl-S-methyl-isothioharnstoff-hydrojodid in 25 ml Acetonitril wird unter Rühren mit 2,5 g Morpholin versetzt. Das Gemisch wird in einem Oelbad 36 Stunden unter Rückfluss gekocht. Das Acetonitril wird unter vermindertem Druck abgedampft und der Rückstand aus einem Gemisch von Isopropanol-Essigsäureäthylester umkristallisiert. Man erhält das N'-(4-Methyl-morpholin-3-yliden)-N-phenyl-4-morpholincarboximidamid-hydrojodid, welches nach Umkristallisation aus einem

Gemisch von Aceton-Essigsäureäthylester bei 165° schmilzt.

Der Ausgangsstoff für die obige Synthese wird wie folgt hergestellt:

Eine Lösung von 23 g 4-Methyl-morpholin-3-on in 100 ml wasserfreiem Methylenchlorid wird unter Kühlen und Rühren mit 80 g Triäthyloxonium-fluoroborat in 100 ml Methylenchlorid versetzt. Das Reaktionsgemisch wird in einer Stickstoffatmosphäre 18 Stunden gerührt. Dann leitet man 3 Stunden Ammoniak in das Gemisch ein. Das Reaktionsgemisch wird 30 Stunden bei Zimmertemperatur stehengelassen und das Lösungsmittel unter vermindertem Druck auf einem Wasserbad von 40° abgedampft. Das rohe 4-Methyl-3-imino-morpholin wird in 100 ml Acetonitril gelöst und unter Rühren bei Zimmertemperatur mit 24 g Phenylisothiocyanat in 60 ml Acetonitril versetzt. Das Gemisch wird 14 Stunden gerührt und konzentriert, wobei sich ein kristallines Produkt ausscheidet. Dieses wird aus einem Gemisch von Isopropanol-Essigsäureäthylester umkristallisiert. Man erhält den N'-(4-Methyl-morpholin-3-yliden)-N-phenyl-thioharnstoff, welcher bei 121-122° schmilzt.

Eine Lösung von 15 g N'-(4-Methyl-morpholin-3-yliden)-N-phenyl-thioharnstoff in 50 ml Dioxan wird unter Rühren tropfenweise mit 10 g Methyljodid in 30 ml Dioxan versetzt. Das Gemisch wird auf dem Wasserbad 3 Stunden erhitzt. Das Dioxan wird unter vermindertem Druck zurückgewonnen und der Rückstand aus einem Gemisch von Aceton-Essigsäureäthylester umkristallisiert. Man erhält das N'-(4-Methyl-morpholin-3-yliden)-N-phenyl-S-methyl-isothioharnstoff-hydrojodid, welches bei 155° schmilzt.

Beispiel 31: Eine Suspension von 3,8 g N-(p-Tolyl)-4-morpholincarboximidamid-hydrojodid in 15 ml Acetonitril wird unter Rühren mit 1,8 g 3-Aethoxy-5,6-dihydro-2H-1,4-oxazin versetzt. Das Gemisch wird auf dem Wasserbad 12 Stunden stark gerührt. Das Acetonitril wird unter vermindertem Druck eingedampft und der Rückstand aus einem Gemisch von Essigsäureäthylester-Aceton umkristallisiert. Man erhält das N-(Morpholin-3-yliden)-N'-(p-tolyl)-4-morpholincarboximidamid-hydrojodid, welches bei 192° schmilzt.

Beispiel 32: Eine Suspension von 4 g N-(p-Tolyl)-4-morpholincarboximidamid-hydrojodid in 20 ml Acetonitril wird mit 2 g 3-Aethoxy-5,6-dihydro-2H-1,4-thiazin unter Rühren versetzt. Das Gemisch wird auf dem Wasserbad 12 Stunden stark gerührt. Das Acetonitril wird unter vermindertem Druck abgedampft und der Rückstand aus einem Gemisch von Isopropanol-Essigsäureäthylester umkristallisiert. Man erhält das N-(Thiomorpholin-3-yliden)-N'-(p-tolyl)-4-morpholincarboximidamid-hydrojodid, welches bei 225° schmilzt.

Beispiel 33: Eine Suspension von 3,1 g N-Phenyl-1-pyrrolidincarboxmidamid-hydrojodid in 7 ml Acetonitril wird mit 5 g 4-Carbäthoxy-2-äthoxy-3,4,5,6-tetrahydropyrazin versetzt. Das Gemisch wird 12 Stunden unter Rückfluss gekocht, dann mit Essigsäureäthylester verdünnt und der erhaltene Niederschlag abfiltriert. Dieser wird mit Kaliumcarbonatlösung behandelt, mit Wasser gewaschen und getrocknet. Durch Behandlung des erhaltenen Produktes mit Chlorwasserstoffsäure in Isopropanol erhält man das N-(4-Carbäthoxy-piperazin-2-yliden)-N'-phenyl-1-pyrrolidincarboximidamid-hydrochlorid, welches nach Umkristallisation aus Acetonitril-Essigsäureäthylester bei 246-247° schmilzt.

Der Ausgangsstoff wird wie folgt hergestellt:
Eine gekühlte Lösung von 43 g 4-Carbäthoxy-2-piperazinon in 400 ml Methylenchlorid wird mit 120 g Triäthyloxonium-fluoroborat in 150 ml Methylenchlorid versetzt. Das Reaktionsgemisch wird in einer Stickstoffatmosphäre 16 Stunden gerührt und dann mit wässriger gesättigter Kaliumcarbonatlösung schwach basisch (pH 7,5) gemacht. Die organische Schicht wird abgetrennt und über wasserfreiem Kaliumcarbonat getrocknet. Das Lösungsmittel wird abgedampft und der Rückstand bei 115°/0,1 mmHg destilliert. Man erhält das 4-Carbäthoxy-2-äthoxy-3,4,5,6-tetrahydropyrazin.

Beispiel 34: Ein Gemisch von 2 g N-Phenyl-1-(4-carbäthoxy-piperazin)-carboximidamid-hydrojodid in Acetonitril und 2,5 g 3-Methoxy-5,6-dihydro-2H-1,4-thiazin wird eine Stunden auf 70° erhitzt. Das Reaktionsgemisch wird bei Zimmertemperatur 7 Tage stehen gelassen und dann mit Diäthyläther verdünnt. Der erhaltene Niederschlag wird aus einem Gemisch von Isopropanol-Essigsäureäthylester umkristallisiert. Man erhält das N-(Thiomorpholin-3-yliden)-N'-phenyl-4-carbäthoxy-1-piperazin-carboximidamid-hydrojodid, welches bei 206° schmilzt.

Der Ausgangsstoff wird wie folgt hergestellt:
Eine Suspension von 8,7 g N-Phenyl-5-methyl-isothioharnstoff-hydrojodid in 30 ml Isopropanol wird mit 9,6 g 1-Carbäthoxy-piperazin versetzt. Das Gemisch wird unter Rühren 18 Stunden unter Rückfluss gekocht und dann unter vermindertem Druck eingedampft. Man erhält das N-Phenyl-4-carbäthoxy-1-piperazincarboximidamid-hydrojodid, welches nach Umkristallisation aus Isopropanol-Essigsäureäthylester bei 218-219° schmilzt.

Beispiel 35: Ein Gemisch von 4 g N-(p-Tolyl)-4-carbäthoxy-1-piperazincarboximidamid-hydrojodid in Acetonitril und 8 g 3-Aethoxy-5,6-dihydro-2H-1,4-thiazin wird eine Stunde auf 80° erhitzt. Die erhaltene Lösung wird 15 Tage bei Zimmertemperatur stehen gelassen. Das dabei erhaltene feste Material wird abfiltriert und mit Essigsäureäthylester gewaschen. Man erhält das N-(Thiomorpholin-3-yliden)-N'-(p-tolyl)-4-carbäthoxy-1-piperazincarboximidamid-hydrojodid, welches nach Umkristallisation aus Acetonitril-Aceton bei 228-229° schmilzt.

Der Ausgangsstoff wird wie folgt hergestellt:
Ein Gemisch von 17,8 g N-(p-Tolyl)-S-methyl-isothioharnstoff-hydrojodid und 18,9 g 1-Carbäthoxy-piperazin in 50 ml Isopropanol wird unter Rühren 12 Stunden unter Rückfluss gekocht. Das Lösungsmittel wird dann unter vermindertem Druck eingedampft und der Rückstand mit Essigsäureäthylester trituriert. Man erhält das N-(p-Tolyl)-4-carbäthoxy-1-piperazincarboximidamid-hydrojodid, welches nach Umkristallisation aus Isopropanol-Essigsäureäthylester bei 201-202° schmilzt.

Beispiel 36: Ein Gemisch von 2,2 g N-(p-Methoxyphenyl)-4-carbäthoxy-1-piperazincarboximidamid-hydrojodid und 1,5 g 3-Aethoxy-5,6-dihydro-2H-1,4-thiazin in 5 ml Acetonitril wird 10 Stunden unter Rückfluss gekocht. Die klare Lösung wird abgekühlt und mit Essigsäureäthylester verdünnt. Man erhält einen Niederschlag, der abfiltriert und aus Acetonitril umkristallisiert wird. Man erhält das N-(Thiomorpholin-3-yliden)-N'-(p-methoxyphenyl)-4-carbäthoxy-1-piperazincarboximidamid-hydrojodid, welches bei 203-204° schmilzt.

Der Ausgangsstoff wird wie folgt hergestellt:
Ein Gemisch von 10 g p-Anisidin-hydrochlorid und 12,25 g Kaliumthiocyanat wird in 100 ml Aethanol 16 Stunden erhitzt. Das Reaktionsgemisch wird abgekühlt und filtriert. Der Rückstand wird mit Wasser gewaschen und aus Methanol umkristallisiert. Man erhält ein festes Material, welches bei 226° schmilzt und aus p-Methoxy-phenylthioharnstoff besteht. Man erhitzt 5,3 g der letztgenannten Verbindung mit 10 ml Methyljodid in 20 ml Aceton 8 Stunden auf 50°. Das Reaktionsgemisch wird abgekühlt, der erhaltene Niederschlag abfiltriert und mit Aceton gewaschen. Man erhält das S-Methyl-p-methoxyphenyl-isothioharnstoff-hydrojodid, welches bei 165-166° schmilzt.

Ein Gemisch von 6,4 g S-Methyl-N-p-methoxyphenyl-isothioharnstoff-hydrojodid und 6,2 g 1-Carbäthoxy-piperazin in 50 ml Acetonitril wird 10 Stunden unter Rückfluss gekocht. Das Lösungsmittel wird unter vermindertem Druck eingedampft und der erhaltene Rückstand mit Essigsäureäthylester trituriert. Man erhält das N-(p-Methoxyphenyl)-4-carbäthoxy-1-piperazincarboximidamid-hydrojodid, welches nach Umkristallisation aus Acetonitril bei 156-157° schmilzt.

Beispiel 37: Eine Suspension von 7,4 g N-(p-Chlorphenyl)-4-morpholincarboximidamid-hydrojodid in 50 ml Acetonitril wird mit 4 g 3-Aethoxy-5,6-dihydro-2H-1,4-thiazin versetzt. Das Gemisch wird unter Rühren 14 Stunden unter Rückfluss gekocht. Das Acetonitril wird unter vermindertem Druck abgedampft und der Rückstand aus einem Gemisch von Isopropanol-Essigsäureäthylester umkristallisiert. Man erhält das

N-(Thiomorpholin-3-yliden)-N'-(p-chlorphenyl)-4-morpholincarboximidamid-hydrojodid.

Der Ausgangsstoff wird wie folgt hergestellt:
Eine Suspension von 32 g N-(p-Chlorphenyl)-S-methyl-isothioharnstoff-hydrojodid in 100 ml Acetonitril wird mit 12 g Morpholin versetzt. Das Gemisch wird unter Rühren 15 Stunden unter Rückfluss gekocht. Das Acetonitril wird unter vermindertem Druck abgedampft und der Rückstand aus einem Gemisch von Methylenchlorid-Essigsäureäthylester umkristallisiert. Man erhält das N-(p-Chlorphenyl)-4-morpholincarboximidamid-hydrojodid, welches bei 185-187° schmilzt.

Beispiel 38: Eine Suspension von 7 g N-(p-Chlorphenyl)-1-pyrrolidin-carboximidamid-hydrojodid in 40 ml Acetonitril wird unter Rühren mit 4 g 3-Aethoxy-5,6-dihydro-2H-1,4-thiazin versetzt. Das Gemisch wird auf einem Wasserbad 12 Stunden stark gerührt. Das Acetonitril wird unter vermindertem Druck abgedampft und der Rückstand aus einem Gemisch von Methylenchlorid-Essigsäureäthylester umkristallisiert. Man erhält das N-(Thiomorpholin-3-yliden)-N'-(p-chlorphenyl)-1-pyrrolidincarboximidamid-hydrojodid, welches bei 198° schmilzt.

Der Ausgangsstoff wird wie folgt hergestellt:
Eine Suspension von 16 g N-(4-Chlorphenyl)-S-methyl-isothioharnstoff-hydrojodid in 50 ml Acetonitril wird mit 5 g Pyrrolidin versetzt. Das Gemisch wird unter Rühren 15 Stunden unter Rückfluss gekocht. Das Acetonitril wird unter vermindertem Druck abgedampft und der Rückstand aus einem Gemisch von Essigsäureäthylester-Isopropanol umkristallisiert. Man erhält das N-(4-Chlorphenyl)-1-pyrrolidincarboximidamid-hydrojodid, welches bei 195-196° schmilzt.

Beispiel 39: Ein Gemisch von 1,9 g N-Phenyl-1-pyrrolidincarboximidamid und 1,4 g 3-Chlorpropyl-isothiocyanat in 25 ml wasserfreiem Dioxan wird 3 Stunden auf 80° erhitzt. Das Lösungsmittel wird unter vermindertem Druck abgedampft, der Rückstand mit Diäthyläther gewaschen und aus Aceton-Methanol umkristallisiert. Man erhält das

N-(Thiomorpholin-3-yliden)-N'-phenyl-1-pyrrolidincarboximidamid-hydrochlorid, welches bei 260-262° schmilzt.

Beispiel 40: Ein Gemisch von 5,5 g N-Phenyl-4-morpholincarboximidamid-hydrojodid und 3,8 g 5-Aethoxy-2,3,6,7-tetrahydro-1H-1,4-thiazepin in 20 ml Acetonitril wird unter Rühren 6 Stunden auf 80-90° erhitzt. Die Lösung wird unter vermindertem Druck konzentriert, das erhaltene feste Material abfiltriert und aus Acetonitril umkristallisiert. Man erhält das N-(Hexahydro-5H-1,4-thiazepin-5-yliden)-N'-phenyl-4-morpholincarboximidamid-hydrojodid, welches unter Zersetzung bei 225° schmilzt.

Beispiel 41: Ein Gemisch von 3,6 g N'-Phenyl-1-(4-morpholinyl)-S-methyl-isothioharnstoff-hydrojodid und 2 g 2-Amino-2-thiazolin in 30 ml Acetonitril wird 8 Stunden unter Rückfluss gekocht. Das Lösungsmittel wird unter vermindertem Druck abgedampft, der Rückstand mit Alkali basisch gemacht und mit Methylenchlorid extrahiert. Die rohe Base wird auf Silicagel in Essigsäureäthylester chromatographiert und die Säule mit Essigsäureäthylester, der steigende Mengen Methanol enthält, eluiert. Die späten Fraktionen ergeben eine ölige Base, die mit Chlorwasserstoff in Isopropanol behandelt wird. Man erhält das 1-(4-Morpholinyl)-N'-phenyl-N-(2-thiazolinyl)-formamidin-hydrochlorid, welches nach Umkristallisation aus einem Gemisch von Methanol und Diäthyläther bei 225 - 227° schmilzt.

Patentansprüche (für alle benannten Länder ausser Oesterreich)

1. Neue Guanidinderivate der Formel I

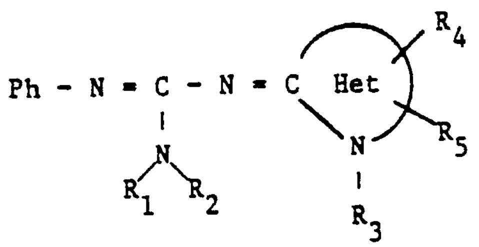

(I)

worin $R_1$ ein gegebenenfalls substituierter aliphatischer oder cycloaliphatischer Kohlenwasserstoffrest, $R_2$ Wasserstoff, ein gegebenenfalls substituierter aliphatischer oder cycloaliphatischer Kohlenwasserstoffrest ist oder $R_1$ und $R_2$ zusammengenommen einen gegebenenfalls substituierten bivalenten Kohlenwasserstoffrest aliphatischen Charakters, in welchem die Kohlenstoffatome der Kette durch ein Heteroatom unterbrochen sein können, $R_3$ Wasserstoff, Niederalkyl, $R_4$ Wasserstoff, Niederalkyl, Niederalkoxy, Niederalkylthio, Niederalkylamino, Diniederalkylamino, Halogen, Trifluormethyl oder gegebenenfalls substituiertes Phenyl, oder an einem beidseitig einfach gebundenen Kohlenstoffatom auch Oxo, $R_5$ Wasserstoff oder Niederalkyl, Het einen Heteroalkylenrest mit 3 Kettengliedern, der die Gruppe C-N zu einem gegebenenfalls ungesättigten heterocyclischen Fünfring mit 2 bis 3 Heteroatomen aus der Gruppe Sauerstoff, Schwefel oder Stickstoff im Ring ergänzt, bzw. Het einen Heteroalkylenrest mit 4-6 Kettengliedern, der die Gruppe C-N zu einem heterocyclischen Sechs- bis Achtring, der neben dem Stickstoffatom noch ein Heteroatom aus der Gruppe Sauerstoff, Schwefel oder Stickstoff im Ring enthält, ergänzt, und Ph einen gegebenenfalls substituierten Phenylrest bedeuten, ihre tautomeren Verbindungen und Salze.

2. Verbindungen der Formel I

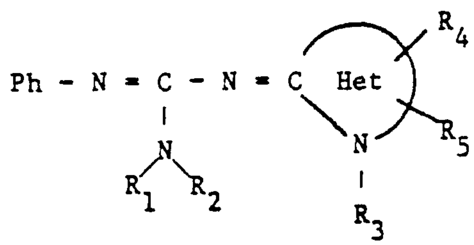

(I)

worin $R_1$ ein gegebenenfalls substituierter aliphatischer oder cycloaliphatischer Kohlenwasserstoffrest, $R_2$ Wasserstoff oder ein gegebenenfalls substituierter aliphatischer Kohlenwasserstoffrest ist, oder $R_1$ und $R_2$ zusammengenommen einen gegebenenfalls substituierten bivalenten Kohlenwasserstoffrest aliphatischen Charakters, in welchem die Kohlenstoffatome der Kette durch ein Heteroatom unterbrochen sein können, $R_3$ Wasserstoff, Niederalkyl, $R_4$ Wasserstoff, Niederalkyl, Niederalkoxy, Niederalkylthio, Niederalkylamino, Diniederalkylamino, Halogen, Trifluormethyl oder gegebenenfalls substituiertes Phenyl, oder an einem beidseitig einfach gebundenen Kohlenstoffatom auch Oxo, $R_5$ Wasserstoff oder Niederalkyl, Het einen Heteroalkylenrest mit 3 Kettengliedern, der die Gruppe C-N zu einem gegebenenfalls ungesättigten heterocyclischen Fünfring mit 2 bis 3 Heteroatomen aus der Gruppe Sauerstoff, Schwefel oder Stickstoff im Ring ergänzt, und Ph einen gegebenenfalls substituierten Phenylrest bedeuten, ihre tautomeren Verbindungen und Salze.

3. Verbindungen der Formel I, in der $R_1$ Niederalkyl oder Cycloalkyl, $R_2$ Wasserstoff oder Niederalkyl oder beide zusammengenommen eine Niederalkylenkette, die gegebenenfalls durch ein Sauerstoff-, Schwefel- oder gegebenenfalls durch z.B. Niederalkyl oder Phenylsubstituiertes Stickstoffatom unterbrochen sein kann, $R_3$ Wasserstoff oder Niederalkyl, $R_4$ Wasserstoff, Niederalkyl, Niederalkoxy, Niederalkylthio, Niederalkylamino, Dinieralkylamino, Halogen, Trifluormethyl, oder an einem beidseitig einfach gebundenen Kohlenstoffatom auch Oxo, oder gegebenenfalls durch Niederalkyl, Niederalkoxy oder Halogen substituiertes Phenyl, $R_5$ Wasserstoff oder Niederalkyl, und Het einen Heteroalkylenrest mit 3 Kettengliedern, der die Gruppe C-N zu einem gesättigten oder einfach gesättigten heterocyclischen Fünfring mit

2-3 Heteroatomen aus der Gruppe Sauerstoff, Schwefel oder Stickstoff im Ring ergänzt, und Ph einen gegebenenfalls substituierten Phenylrest bedeuten, als auch ihre tautomeren Verbindungen und Salze.

4. Verbindungen der Formel I, in der $R_1$ Niederalkyl oder Cycloalkyl, $R_2$ Wasserstoff oder Niederalkyl oder beide zusammengenommen eine Niederalkylenkette, die gegebenenfalls durch ein Sauerstoff- oder Schwefelatom oder gegebenenfalls durch z.B. Niederalkyl oder Phenyl substituiertes Stickstoffatom unterbrochen sein kann, z.B. Niederalkylenamino,z.B. Pyrrolidino, 2,5-Dimethylpyrrolidino, Piperidino, 2-Methylpiperidino, Hexahydroazepino oder Octahydroazocino, Oxaniederalkylenamino, z.B. Morpholino, Thianiederalkylenamino, z.B. Thiomorpholino oder Azaniederalkylenamino, z.B. Piperazino, N-Methyl- oder N-Phenylpiperazino bedeuten, $R_3$ Wasserstoff oder Niederalkyl, $R_4$ Wasserstoff, Niederalkyl, Niederalkoxy, Niederalkylthio, Niederalkylamino, Diniederalkylamino, Halogen, Trifluormethyl, oder an einem beidseitig einfach gebundenen Kohlenstoffatom auch Oxo, oder gegebenenfalls durch Niederalkyl, Niederalkoxy oder Halogen substituiertes Phenyl, $R_5$ Wasserstoff oder Niederalkyl, und Het einen zweiwertigen Heteroalkylenrest mit 3 Kettengliedern, der die Gruppe C-N zu einem gesättigten oder einfach ungesättigten heterocyclischen Fünfring mit 2-3 Heteroatomen im Ring ergänzt und beispielsweise ein Imidazolin-, Imidazolidin-, Oxazolin-, Oxazolidin-, Thiazolin-, Thiazolidin-, Isoxazolin-, Isoxazolidin-, Isothiazolin-, Isothiazolidin-, Oxadiazolin-, Oxadiazolidin-, Thiadiazolin-, Thiadiazolidin-, Pyrazolin-, Pyrazolidin- oder auch Triazolinring sein kann, und Ph einen gegebenenfalls durch Niederalkyl, Niederalkoxy oder Halogen substituierten Phenylrest bedeuten, als auch ihre tautomeren Verbindungen und Salze.

5. Verbindungen der Formel I, in der $R_1$ Niederalkyl oder Cycloalkyl, $R_2$ Wasserstoff oder Niederalkyl oder die Gruppe $-NR_1R_2$ beispielsweise Niederalkylenamino, in der die Niederalkylenkette gegebenenfalls durch

ein Sauerstoff- oder Schwefelatom unterbrochen sein kann, bedeutet und beispielsweise Pyrrolidino, 2,5-Dimethylpyrrolidino, Piperidino, 2-Methylpiperidino, Morpholino oder Thiomorpholino sein kann, $R_3$ Wasserstoff oder Niederalkyl, $R_4$ Wasserstoff, Niederalkyl, Niederalkoxy, Niederalkylthio, Halogen, Trifluormethyl oder an einem beidseitig einfach gebundenen Kohlenstoffatom auch Oxo, oder gegebenenfalls durch Niederalkyl, Niederalkoxy oder Halogen substituiertes Phenyl, $R_5$ Wasserstoff oder Niederalkyl, und Het als zweiwertiger Heteroalkylenrest mit der Gruppe C-N zusammen einen gegebenenfalls einfach ungesättigten heterocyclischen Fünfring, wie z.B. Imidazolin-, Imidazolidin-, Oxazolin-, Oxazolidin-, Thiazolin-, Thiazolidin-, Oxadiazolin-, Oxadiazolidin-, Triazolin-, Thiadiazolin- oder Thiadiazolidinring bedeutet, und Ph einen gegebenenfalls durch Niederalkyl, Niederalkoxy oder Halogen substituierten Phenylrest bedeutet, als auch ihre tautomeren Verbindungen und Salze.

6. Verbindungen der Formel I, in der $R_1$ Niederalkyl, z.B. Methyl oder Aethyl, $R_2$ Wasserstoff oder Niederalkyl, z.B. Methyl oder Aethyl bedeutet oder die Gruppe $-NR_1R_2$ als gegebenenfalls durch ein Sauerstoff- oder Schwefelatom unterbrochenes Niederalkylenamino, beispielsweise als Pyrrolidino, Piperidino, Morpholini oder Thiomorpholino vorliegt, $R_3$ Wasserstoff oder Niederalkyl, z.B. Methyl oder Aethyl, $R_4$ Wasserstoff, Niederalkyl, z.B. Methyl oder Aethyl, Niederalkoxy, z.B. Methoxy oder Aethoxy, Niederalkylthio, z.B. Methylthio oder Aethylthio, Halogen, z.B. Chlor oder Brom, Trifluormethyl oder gegebenenfalls durch Niederalkyl, z.B. Methyl oder Aethyl, Halogen, z.B. Chlor oder Brom substituiertes Phenyl, $R_5$ Wasserstoff oder Niederalkyl, z.B. Methyl oder Aethyl und Het als zweiwertiger Heteroalkylenrest mit der Gruppe C-N einen gegebenenfalls einfach ungesättigten heterocyclischen Fünfring, wie z.B. den 4-Imidazolin-, 4-Oxazolin-, 4-Thiazolin-, 4-(1,3,4)-Thiadiazolin-, 4-(1,3,4)-Oxadiazolin-, oder auch Isoxazolidinring bedeutet, und Ph einen gegebenenfalls durch Niederalkyl, wie z.B. Methyl oder Aethyl, Halogen, wie z.B. Chlor oder Brom oder Niederalkoxy, wie z.B. Methoxy oder Aethoxy, substituierten Phenylrest bedeutet, als auch ihre tautomeren Verbindungen und Salze.

7. N-(2,5-Dimethyl-3-isoxazolidinyliden)-N'-phenyl-4-morpholin-carboximidamid als auch ihre tautomeren Verbindungen oder Salze davon.

8. N-(2,5-Dimethyl-3-isoxazolidinyliden)-N'-phenyl-1-piperidin-carboximidamid als auch ihre tautomeren Verbindungen oder Salze davon.

9. N-[3-Methyl-4-(1,3,4)-thiadiazolin-2-yliden]-N'-phenyl-4-morpholin-carboximidamid als auch ihre tautomeren Verbindungen oder Salze davon.

10. N-[3-Methyl-4-(1,3,4)-thiadiazolin-2-yliden]-N'-phenyl-1-piperidin-carboximidamid als auch ihre tautomeren Verbindungen oder Salze davon.

11. Hypoglykämisch wirksame Verbindungen der Formel I und ihre tautomeren Verbindungen gemäss Anspruch 1 und Salze von solchen Verbindungen.

12. Hypoglykämisch wirksame Verbindungen der Formel I und ihre tautomeren Verbindungen gemäss Anspruch 2 und Salze von solchen Verbindungen.

13. Verbindungen der Formel Ib

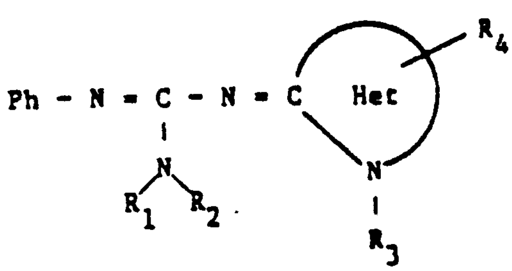

(Ib),

worin $R_1$ und $R_2$ einen gegebenenfalls substituierten aliphatischen oder cycloaliphatischen Kohlenwasserstoffrest bedeuten oder $R_1$ und $R_2$ zusammengenommen einen gegebenenfalls substituierten bivalenten

Kohlenwasserstoffrest aliphatischen Charakters, in welchem die Kohlenstoffatome der Kette durch ein Heteroatom unterbrochen sein können, $R_3$ Wasserstoff, Niederalkyl, $R_4$ Wasserstoff, Niederalkyl, Niederalkoxy, Niederalkylamino, Diniederalkylamino, Halogen, Niederalkoxycarbonyl oder gegebenenfalls substituiertes Phenyl, oder an einem beidseitig einfach gebundenen Kohlenstoffatom auch Oxo, Het einen Heteroalkylenrest mit 4 bis 6 Kettengliedern, der die Gruppe C-N zu einem heterocyclischen Sechs- bis Achtring, der neben dem Stickstoffatom noch ein Heteroatom aus der Gruppe Sauerstoff, Schwefel oder Stickstoff im Ring enthält, ergänzt, und Ph einen gegebenenfalls substituierten Phenylrest bedeuten, ihre tautomeren Verbindungen und Salze.

14. Verbindungen der Formel Ib, in der $R_1$ und $R_2$ Niederalkyl oder Cycloalkyl, oder beide zusammengenommen eine gerade, verzweigte oder substituierte Niederalkylenkette, die gegebenenfalls durch ein Sauerstoff-, Schwefel- oder gegebenenfalls durch z.B. Niederalkyl, Phenyl oder Niederalkoxycarbonyl substituiertes Stickstoffatom unterbrochen sein kann, $R_3$ Wasserstoff oder Niederalkyl, $R_4$ Wasserstoff, Niederalkyl, Niederalkoxy, Niederalkylamino, Diniederalkylamino, Halogen, Niederalkoxycarbonyl, oder an einem beidseitig einfach gebundenen Kohlenstoffatom auch Oxo, oder gegebenenfalls durch Niederalkyl, Niederalkoxy oder Halogen substituiertes Phenyl, und Het einen Heteroalkylenrest mit 4 bis 6 Kettengliedern, der die Gruppe C-N zu einem heterocyclischen Sechs- bis Achtring, der neben dem Stickstoffatom noch ein Heteroatom aus der Gruppe Sauerstoff, Schwefel oder Stickstoff im Ring enthält, ergänzt, und Ph einen gegebenenfalls substituierten Phenylrest bedeuten, als auch ihre tautomeren Verbindungen und Salze.

15. Verbindungen der Formel Ib, in der $R_1$ und $R_2$ Niederalkyl oder Cycloalkyl, oder beide zusammengenommen eine gerade, verzweigte oder substituierte Niederalkylenkette, die gegebenenfalls durch ein Sauerstoff- oder Schwefelatom oder gegebenenfalls durch z.B. Niederalkyl, Phenyl

oder Niederalkoxycarbonyl substituiertes Stickstoffatom unterbrochen sein kann, als Niederalkylenamino beispielsweise Pyrrolidino, 2,5-Dimethylpyrrolidino, Piperidino, 2-Methylpiperidino, 4-Phenylpiperidino, Hexahydroazepino oder Octahydroazocino, als Oxaniederalkylenamino beispielsweise Morpholino, 2,6-Dimethylmorpholino, als Thianiederalkylenamino beispielsweise Thiomorpholino oder als Azaniederalkylenamino, beispielsweise Piperazino, N-Niederalkoxycarbonyl, N-Methyl- oder N-Phenylpiperazino bedeuten, $R_3$ Wasserstoff oder Niederalkyl, $R_4$ Wasserstoff, Niederalkyl, Niederalkoxy, Niederalkylamino, Diniederalkylamino, Halogen, Niederalkoxycarbonyl, oder gegebenenfalls durch Niederalkyl, Niederalkoxy oder Halogen substituiertes Phenyl, und Het einen zweiwertigen Heteroalkylenrest mit 4 bis 5 Kettengliedern, der die Gruppe C-N zu einem heterocyclischen Sechs- bis Siebenring, der neben dem Stickstoffatom noch ein Heteroatom aus der Gruppe Sauerstoff, Schwefel oder Stickstoff, im Ring enthält, ergänzt, und Ph einen gegebenenfalls durch Niederalkyl, Niederalkoxy oder Halogen substituierten Phenylrest bedeuten als auch ihre tautomeren Verbindungen und Salze.

16. Verbindungen der Formel Ib,in der $R_1$ und $R_2$ Niederalkyl oder die Gruppe $-NR_1R_2$ gemeinsam ein Niederalkylamino, in der die gerade, verzweigte oder durch Phenyl substituierte Niederalkylenkette gegebenenfalls durch ein Sauerstoff-, Schwefel- oder gegebenenfalls

durch ein Sauerstoff-, Schwefel- oder gegebenenfalls durch z.B. Niederalkyl, Phenyl oder Niederalkoxycarbonyl substituiertes Stickstoffatom unterbrochen sein kann, bedeutet und beispielsweise als Niederalkylenamino Pyrrolidino, 2,5-Dimethylpyrrolidino, Piperidino, 2-Methylpiperidino, 4-Phenylpiperidino, Hexahydroazepino oder Octahydroazocino, als Oxaniederalkylenamino beispielsweise Morpholino, 2,6-Dimethylmorpholino, als Thianiederalkylenamino beispielsweise Thiomorpholino oder als Azaniederalkylenamino beispielsweise Piperazino, N-Aethoxycarbonyl-, N-Methyl-, N-Phenylpiperazino sein kann, $R_3$ Wasserstoff oder Niederalkyl, $R_4$ Wasserstoff, Niederalkyl, Niederalkoxy, Niederalkylamino, Halogen, Niederalkoxycarbonyl, oder gegebenenfalls durch Niederalkyl, Niederalkoxy oder Halogen substituiertes Phenyl und Het einen zweiwertigen Heteroalkylenrest mit 4 bis 5 Kettengliedern, der die Gruppe

C-N zu einem heterocyclischen Sechs- bis Siebenring, der neben dem Stickstoffatom noch ein Heteroatom aus der Gruppe Sauerstoff, Schwefel oder Stickstoff im Ring enthält, ergänzt, und beispielsweise ein Morpholin-, Thiomorpholin-, 2H-3,4,5,6-Tetrahydro-1,3-thiazin-, 2H-3,4,5,6-Tetrahydropyrimidin-, Piperazin- oder auch Hexahydro-1,4-thiazepinring sein kann, und Ph einen gegebenenfalls durch Niederalkyl, Niederalkoxy, oder Halogen substituierten Phenylrest bedeutet, als auch ihre tautomeren Verbindungen und Salze.

17. Verbindungen der Formel Ib, in der $R_1$ und $R_2$ Niederalkyl, z.B. Methyl oder Aethyl, oder die Gruppe $-NR_1R_2$ gemeinsam ein Niederalkylenamino, in der die gerade, verzweigte oder durch Phenyl substituierte Niederalkylenkette gegebenenfalls durch ein Sauerstoff-, Schwefel- oder gegebenenfalls durch Niederalkyl, wie z.B. Methyl oder Aethyl oder Niederalkoxycarbonyl, wie z.B. Methoxy- oder Aethoxycarbonyl substituiertes Stickstoffatom unterbrochen sein kann, bedeutet und beispielsweise Pyrrolidino, Piperidino, 4-Phenylpiperidino, Morpholino, 2,6-Dimethylmorpholino, Thiomorpholino, Piperazino, N-Aethoxycarbonylpiperazino sein kann, $R_3$ Wasserstoff oder Niederalkyl, z.B. Methyl oder Aethyl, $R_4$ Wasserstoff, Niederalkyl, z.B. Methyl oder Aethyl, oder Niederalkoxycarbonyl, z.B. Methoxy- oder Aethoxycarbonyl und Het einen zweiwertigen Heteroalkylenrest, der mit der Gruppe C-N diese zu einem heterocyclischen Sechsring, der neben dem Stickstoffatom noch ein Heteroatom aus der Gruppe Sauerstoff, Schwefel oder Stickstoff im Ring enthält, ergänzt, und beispielsweise ein Morpholin-, Thiomorpholin-, oder auch Piperazinring sein kann, und Ph einen gegebenenfalls durch Niederalkyl, wie z.B. Methyl oder Aethyl, Halogen, wie z.B. Fluor, Chlor oder Brom oder Niederalkoxy, wie z.B. Methoxy oder Aethoxy, substituierten Phenylrest bedeutet, als auch ihre tautomeren Verbindungen und Salze.

18. N-(Thiomorpholin-3-yliden)-N'-phenyl-4-morpholincarboximid-amid als auch ihre tautomeren Verbindungen oder Salze davon.

19. N-(Morpholin-3-yliden)-N'-phenyl-4-morpholincarboximidamid als auch ihre tautomeren Verbindungen oder Salze davon.

20. N-(Thiomorpholin-3-yliden)-N'-(4-methoxyphenyl)-4-morpholin-carboximidamid als auch ihre tautomeren Verbindungen oder Salze davon.

21. Hypoglykämisch wirksame Verbindungen der Formel Ib und ihre tautomeren Verbindungen gemäss Anspruch 13 und Salze von solchen Verbindungen.

22. Pharmazeutisch verwendbare Präparate enthaltend Verbindungen der Formel I gemäss Anspruch I oder pharmazeutisch verwendbare Salze von solchen Verbindungen.

23. Pharmazeutisch verwendbare Präparate enthaltend Verbindungen der Formel I gemäss Anspruch 2 oder pharmazeutisch verwendbare Salze von solchen Verbindungen.

24. Pharmazeutisch verwendbare Präparate enthaltend Verbindungen der Formel Ib gemäss Anspruch 13 oder pharmazeutisch verwendbare Salze von solchen Verbindungen.

25. Die Verbindungen gemäss Anspruch 1 zur Anwendung in einem Verfahren zur therapeutischen Behandlung des menschlichen oder tierischen Körpers.

26. Die Verbindungen gemäss Ansprüchen 2 - 10 zur Anwendung in einem Verfahren zur therapeutischen Behandlung des menschlichen oder tierischen Körpers.

27. Die Verbindungen gemäss Ansprüchen 13-20 zur Anwendung in einem Verfahren zur therapeutischen Behandlung des menschlichen oder tierischen Körpers.

28. Die Verbindungen gemäss Anspruch 1 zur Behandlung von Hyperglykämie.

29. Die Verbindungen gemäss Ansprüchen 2-10 zur Behandlung von Hyperglykämie.

30. Die Verbindungen gemäss Ansprüchen 13-20 zur Behandlung von Hyperglykämie.

31. Verwendung der Verbindungen gemäss Anspruch 1 zur Herstellung von pharmazeutischen Präparaten.

32. Verwendung der Verbindungen gemäss Ansprüchen 2-10 zur Herstellung von pharmazeutischen Präparaten.

33. Verwendung der Verbindungen gemäss Ansprüchen 13-19 zur Herstellung von pharmazeutischen Präparaten.

34. Verfahren zur Herstellung von Guanidinderivaten der Formel

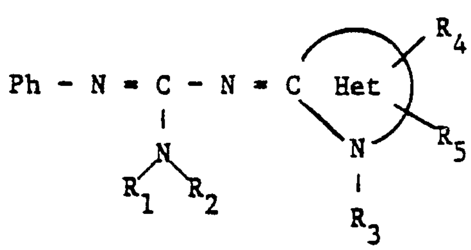

(I)

worin $R_1$ ein gegebenenfalls substituierter aliphatischer oder cycloaliphatischer Kohlenwasserstoffrest, $R_2$ Wasserstoff, ein gegebenenfalls substituierter aliphatischer oder cycloaliphatischer Kohlenwasserstoffrest ist oder $R_1$ und $R_2$ zusammengenommen einen gegebenenfalls substituierten bivalenten Kohlenwasserstoffrest aliphatischen Charakters, in welchem die Kohlenstoffatome der Kette durch ein Heteroatom unterbrochen sein können, $R_3$ Wasserstoff, Niederalkyl, $R_4$ Wasserstoff, Niederalkyl, Nideralkoxy, Niederalkylthio, Niederalkylamino, Diniederalkylamino, Halogen, Trifluormethyl oder gegebenenfalls substituiertes Phenyl, oder an einem beidseitig einfach gebundenen Kohlenstoffatom auch Oxo, $R_5$ Wasserstoff oder Niederalkyl, Het einen Heteroalkylenrest mit 3 Kettengliedern, der die Gruppe C-N zu einem gegebenenfalls ungesättigten heterocyclischen Fünfring mit 2 bis 3 Heteroatomen aus der Gruppe Sauerstoff, Schwefel oder Stickstoff im Ring ergänzt, bzw. Het einen Heteroalkylenrest mit 4 bis 6 Kettengliedern, der die Gruppe C-N zu einem heterocyclischen Sechs- bis Achtring, der neben dem Stickstoffatom noch ein Heteroatom aus der Gruppe Sauerstoff, Schwefel oder Stickstoff im Ring enthält, ergänzt, und Ph einen gegebenenfalls substituierten Phenylrest bedeuten, ihre tautomeren Verbindungen und Salze, dadurch gekennzeichnet, dass man

a) eine Verbindung der Formel

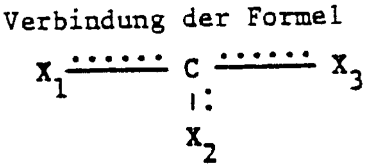

(II)

worin $X_1$ die Gruppe Ph-N=, in der Ph einen gegebenenfalls substituierten Phenylrest bedeutet, oder eine abspaltbare Gruppe, $X_2$ die Gruppe

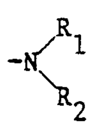 , worin $R_1$ und $R_2$ die unter der Formel I angegebene Bedeutung

haben, oder eine abspaltbare Gruppe und $X_3$ die Gruppe

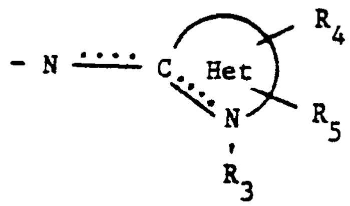

worin $R_3$, $R_4$, $R_5$ und Het als zweiwertiger Heteroalkylenrest mit der Gruppe C-N die unter der Formel I angegebenen Bedeutungen haben, oder eine abspaltbare Gruppe bedeutet, mit der Massgabe, dass nur einer der Substituenten $X_1$, $X_2$ oder $X_3$ eine abspaltbare Gruppe sein kann, und worin eine der Gruppen $X_1$, $X_2$ und $X_3$ durch eine Doppelbindung mit dem Kohlenstoffatom verbunden ist, mit einem Amin oder Imin umsetzt, welches mit der fehlenden Amino- oder Iminogruppe identisch ist, die unter $X_1$, $X_2$ oder $X_3$ definiert werden, um die abspaltbare Gruppe zu ersetzen, oder

b) eine Guanidinverbindung der allgemeinen Formel IV

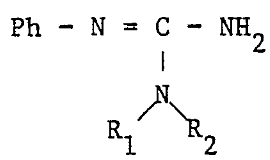

(IV)

worin Ph, $R_1$ und $R_2$ die unter der Formel I angegebenen Bedeutungen haben, mit einer Verbindung der allgemeinen Formel V

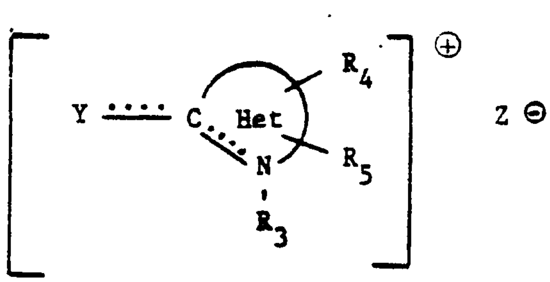

worin Y Niederalkoxy, Niederalkylthio, Halogen, oder Y zwei Niederalkoxygruppen, die am gleichen C-Atom sitzen, und Z ein Tetrafluoroborat-, ein Fluorsulfonat-, ein Niederalkylsulfat-, wie z.B. Methylsulfat- oder Niederalkansulfonat-, wie z.B. Methansulfonatanion oder ein Halogenid, wie z.B. Chlorid oder Bromid, bedeutet, wobei, wenn Y die Bedeutung von zwei Niederalkoxygruppen am gleichen C-Atom hat, Z als Anion entfällt, oder, wenn $R_3$ Wasserstoff bedeutet, die tautomere Form als freie Base vorliegt, umsetzt, oder

c) Guanidinderivate der allgemeinen Formel VI

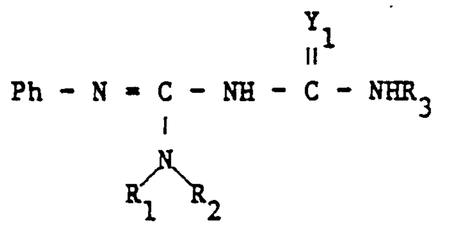 (VI)

oder eine tautomere Form davon, worin Ph, $R_1$, $R_2$ und $R_3$ die vorstehend genannten Bedeutungen haben und $Y_1$ die Bedeutung einer Oxo-, Thioxo- oder NH-Gruppe hat, mit einer Verbindung der allgemeinen Formel VII

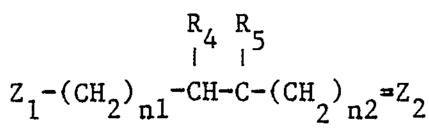 (VII) ,

worin $n_1$ oder $n_2$ 0, 1, 2 oder 3 bedeuten, mit der Massgabe, dass $n_1$ und $n_2$ zusammen nicht mehr als 3 bedeuten, $Z_1$ ein Halogenatom $Z_2$ eine Oxogruppe oder zusammen die Gruppe bestehend aus Wasserstoff und Halogen, wobei das Wasserstoffatom Teil einer Methylen gruppe sein kann, oder $Z_1$ und $Z_2$ zusammengenommen über eine Iminogruppe den bivalenten Alkylenrest 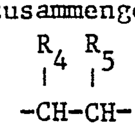 zu einem Aziridinderivat ergänzen, wobei $R_4$ und $R_5$ die Bedeutungen von Wasserstoff haben, oder

d) für die Herstellung von Verbindungen der Formel I, worin Het einen Heteroalkylenrest mit 4-6 Kettengliedern, der die Gruppe C-N zu einem heterocyclischen 6-8 Ring ergänzt, Verbindungen der Formel IV

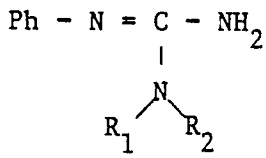

(IV) ,

worin $R_1$, $R_2$ und Ph die oben angegebenen Bedeutungen haben, mit einem Halogenalkylenisothiocyanat der Formel VIII

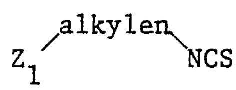

(VIII) ,

worin $Z_1$ ein Halogenatom bedeutet, und der bivalente Rest "alkylen" eine Alkylenkette mit 3 - 5 Kohlenstoffatomen bedeutet, wobei ein Wasserstoffatom in der Alkylenkette durch den Substituenten $R_4'$, der die Bedeutung für $R_4$ mit Ausnahme von Wasserstoff hat, ersetzt sein kann, umsetzt, oder

e) Verbindungen der Formel I, in der $R_1$ die oben definierte Bedeutung hat und $R_2$ und/oder $R_3$ Wasserstoff bedeuten, durch Umsetzung mit einem reaktionsfähigen Ester eines aliphatischen oder cycloaliphatischen Alkohols in Verbindungen der Formel I umwandelt, in denen $R_2$ und/oder $R_3$ im Rahmen der vorstehenden Definitionen für $R_2$ und $R_3$ verschieden von Wasserstoff sind, oder

f) in Verbindungen der allgemeinen Formel IX

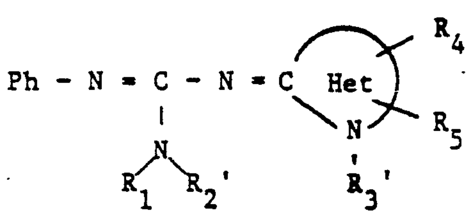

(IX) ,

worin $R_1$, $R_4$, $R_5$, Ph und Het die unter der Formel I angegebene Bedeutung haben, und der eine von den Substituenten $R_2'$ und $R_3'$ die Bedeutung von $R_2$ oder $R_3$ hat, und der andere eine Aminoschutzgruppe

bedeuten, diese abspaltet und gewünschtenfalls zusätzliche Verfahrensschritte durchführt, und/oder, wenn erwünscht, erhaltene Verbindungen der Formel I in ein Salz überführt, und/oder, wenn erwünscht, erhaltene Salze der Verbindungen der Formel I in die freien Basen umwandelt.

Patentansprüche (für Oesterreich)

1. Verfahren zur Herstellung von Guanidinderivaten der Formel

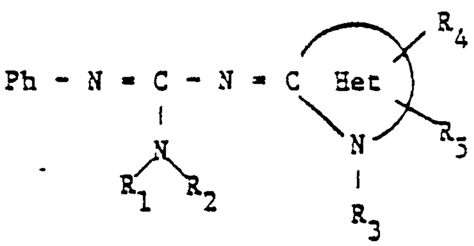

(I)

worin $R_1$ ein gegebenenfalls substituierter aliphatischer oder cycloaliphatischer Kohlenwasserstoffrest, $R_2$ Wasserstoff, ein gegebenenfalls substituierter aliphatischer oder cycloaliphatischer Kohlenwasserstoffrest ist oder $R_1$ und $R_2$ zusammengenommen einen gegebenenfalls substituierten bivalenten Kohlenwasserstoffrest aliphatischen Charakters, in welchem die Kohlenstoffatome der Katte durch ein Heteroatom unterbrochen sein können, $R_3$ Wasserstoff, Niederalkyl, $R_4$ Wasserstoff, Niederalkyl, Niederalkoxy, Niederalkylthio, Niederalkylamino, Diniederalkylamino, Halogen, Trifluormethyl oder gegebenenfalls substituiertes Phenyl, oder an einem beidseitig einfach gebundenen Kohlenstoffatom auch Oxo, $R_5$ Wasserstoff oder Niederalkyl, Het einen Heteroalkylenrest mit 3 Kettengliedern, der die Gruppe C-N zu einem gegebenenfalls ungesättigten heterocyclischen Fünfring mit 2 bis 3 Heteroatomen aus der Gruppe Sauerstoff, Schwefel oder Stickstoff im Ring ergänzt, bzw Het einen Heteroalkylenrest mit 4 bis 6 Kettengliedern, der die Gruppe C-N zu einem heterocyclischen Sechs- bis Achtring, der neben dem Stickstoffatom noch ein Heteroatom aus der Gruppe Sauerstoff, Schwefel oder Stickstoff im Ring enthält, ergänzt, und Ph einen gegebenenfalls substituierten Phenylrest bedeuten, ihre tautomeren Verbindungen und Salze, dadurch gekennzeichnet, dass man

a) eine Verbindung der Formel

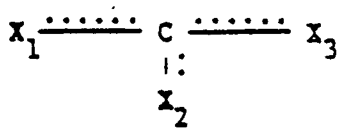

(II)

worin $X_1$ die Gruppe Ph-N=, in der Ph einen gegebenenfalls substituierten Phenylrest bedeutet, oder eine abspaltbare Gruppe, $X_2$ die Gruppe

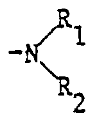 , worin $R_1$ und $R_2$ die unter der Formel I angegebene Bedeutung haben, oder eine abspaltbare Gruppe und $X_3$ die Gruppe

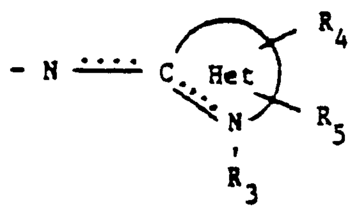

worin $R_3$, $R_4$, $R_5$ und Het als zweiwertiger Heteroalkylenrest mit der Gruppe C-N die unter der Formel I angegebenen Bedeutungen haben, oder eine abspaltbare Gruppe bedeutet, mit der Massgabe, dass nur einer der Substituenten $X_1$, $X_2$ oder $X_3$ eine abspaltbare Gruppe sein kann, und worin eine der Gruppen $X_1$, $X_2$ und $X_3$ durch eine Doppelbindung mit dem Kohlenstoffatom verbunden ist, mit einem Amin oder Imin umsetzt, welches mit der fehlenden Amino- oder Iminogruppe identisch ist, die unter $X_1$, $X_2$ oder $X_3$ definiert werden, um die abspaltbare Gruppe zu ersetzen, oder

b) eine Guanidinverbindung der allgemeinen Formel IV

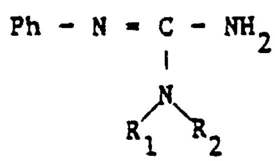

(IV)

worin Ph, $R_1$ und $R_2$ die unter der Formel I angegebenen Bedeutungen haben, mit einer Verbindung der allgemeinen Formel V

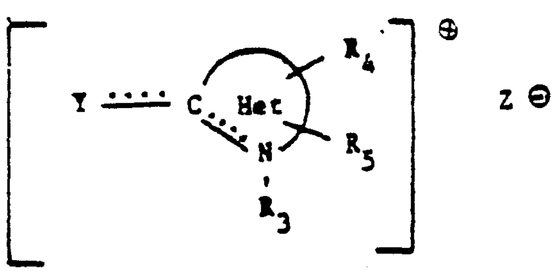

worin Y Niederalkoxy, Niederalkylthio, Halogen, oder Y zwei Niederalkoxygruppen, die am gleichen C-Atom sitzen, und Z ein Tetrafluoroborat-, ein Fluorsulfonat-, ein Niederalkylsulfat-, wie z.B. Methylsulfat- oder Niederalkansulfonat-, wie z.B. Methansulfonatanion oder ein Halogenid, wie z.B. Chlorid oder Bromid, bedeutet, wobei, wenn Y die Bedeutung von zwei Niederalkoxygruppen am gleichen C-Atom hat, Z als Anion entfällt, oder, wenn $R_3$ Wasserstoff bedeutet, die tautomere Form als freie Base vorliegt, umsetzt, oder

c) Guanidinderivate der allgemeinen Formel VI

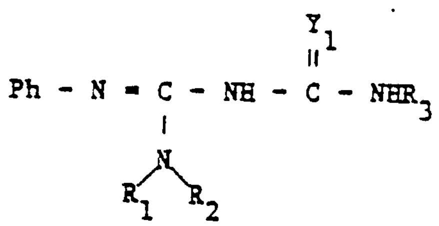

(VI)

oder eine tautomere Form davon, worin Ph, $R_1$, $R_2$ und $R_3$ die vorstehend genannten Bedeutungen haben und $Y_1$ die Bedeutung einer Oxo-, Thioxo- oder NH-Gruppe hat, mit einer Verbindung der allgemeinen Formel VII

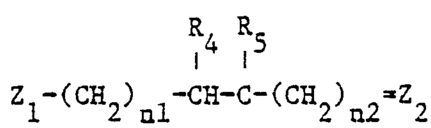

(VII)

umsetzt, worin $n_1$ oder $n_2$ 0, 1, 2 oder 3 bedeuten, mit der Massgabe, dass $n_1$ und $n_2$ zusammen nicht mehr als 3 bedeuten, $Z_1$ ein Halogenatom, $Z_2$ eine Oxogruppe oder zusammen die Gruppe bestehend aus Wasserstoff und Halogen, wobei das Wasserstoffatom Teil einer Methylengruppe sein kann, oder $Z_1$ und $Z_2$ zusammengenommen über eine Iminogruppe den bivalenten Alkylenrest $-\overset{R_4}{\overset{|}{CH}}-\overset{R_5}{\overset{|}{CH}}-$ zu einem Aziridinderivat ergänzen, wobei $R_4$ und $R_5$ die Bedeutungen von Wasserstoff haben,

d) für die Herstellung von Verbindungen der Formel I, worin Het einen Heteroalkylenrest mit 4 - 6 Kettengliedern, der die Gruppe C-N zu einem heterocyclischen 6 - 8 Ring ergänzt, Verbindungen der Formel IV

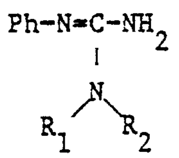

(IV) ,

worin $R_1$, $R_2$ und Ph die oben angegebenen Bedeutungen haben mit einem Halogenalkylenisothiocyanat der Formel VIII

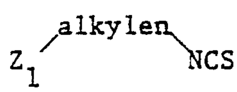

(VIII) ,

worin $Z_1$ ein Halogenatom bedeutet, und der bivalente Rest "alkylen" eine Alkylenkette mit 3 - 5 Kohlenstoffatomen bedeutet, wobei ein Wasserstoffatom in der Alkylenkette durch den Substituenten $R_4'$, der die Bedeutung für $R_4$ mit Ausnahme von Wasserstoff hat, ersetzt sein kann, umsetzt, oder

e) Verbindungen der Formel I, in der $R_1$ die oben definierte Bedeutung hat und $R_2$ und/oder $R_3$ Wasserstoff bedeuten, durch Umsetzung mit einem reaktionsfähigen Ester eines aliphatischen oder cycloaliphatischen Alkohols in Verbindungen der Formel I umwandelt, in denen $R_2$ und/oder $R_3$ im Rahmen der vorstehenden Definitionen für $R_2$ und $R_3$ verschieden von Wasserstoff sind, oder

f) in Verbindungen der allgemeinen Formel IX

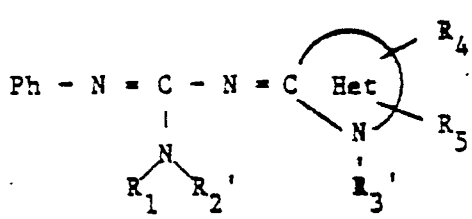

(IX) ,

worin $R_1$, $R_4$, $R_5$, Ph und Het die unter der Formel I angegebene Bedeutung haben, und der eine von den Substituenten $R_2'$ und $R_3'$ die Bedeutung von $R_2$ oder $R_3$ hat, und der andere eine Aminoschutzgruppe bedeutet, oder beide $R_2'$ und $R_3'$ eine Aminoschutzgruppe bedeuten, diese und gewünschtenfalls zusätzliche Verfahrensschritte durchführt, und/oder, wenn erwünscht, erhaltene Verbindungen der Formel I in ein Salz überführt, und/oder, wenn erwünscht, erhaltene Salze der Verbindungen der Formel I in die freien Basen umwandelt.

2. Verfahren nach Patentanspruch 1, dadurch gekennzeichnet, dass man eine Verbindung der Formel IIa

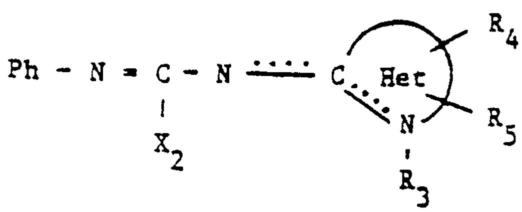

(IIa)

worin $X_2$ eine abspaltbare Gruppe bedeutet, mit einem Amin der Formel $HNR_1R_2$ umsetzt.

3. Verfahren nach Patentanspruch 1, dadurch gekennzeichnet, dass man eine Verbindung der Formel IIb

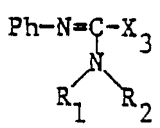

(IIb)

worin $X_3$ eine abspaltbare Gruppe bedeutet, mit einer Iminoverbindung der Formel III

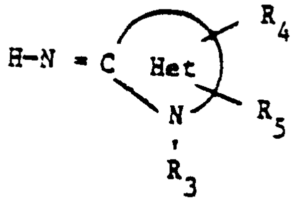

(III)

umsetzt.

4. Verfahren nach Patentanspruch 1, dadurch gekennzeichnet, dass man eine Verbindung der Formel IIc

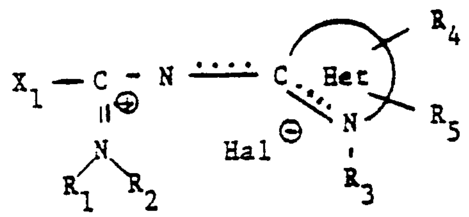

(IIc)

in der $X_1$ eine abspaltbare Gruppe bedeutet, mit einem gegebenenfalls substituierten Anilin der Formel $Ph-NH_2$ umsetzt.

5. Verfahren nach Patentansprüche 1-4, dadurch gekennzeichnet, dass ein abspaltbarer Rest in den Verbindungen der Formeln II, IIa, IIb und IIc eine Niederalkylthio-, Niederalkoxy- oder ein Halogen bedeutet.

6. Verfahren zur Herstellung neuer Guanidinderivate der Formel I

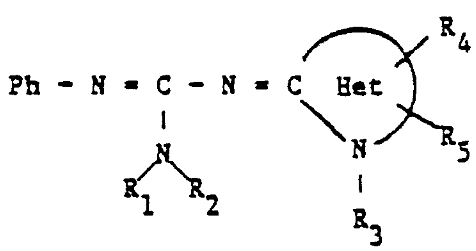

(I)

worin $R_1$ ein gegebenenfalls substituierter aliphatischer oder cycloaliphatischer Kohlenwasserstoffrest, $R_2$ Wasserstoff oder ein gegebenenfalls substituierter aliphatischer Kohlenwasserstoffrest ist oder $R_1$ und $R_2$ zusammengenommen einen gegebenenfalls substituierten bivalenten Kohlenwasserstoffrest aliphatischen Charakters, in welchem die Kohlenstoffatome der Kette durch ein Heteroatom unterbrochen sein können, $R_3$ Wasserstoff, Niederalkyl, $R_4$ Wasserstoff, Niederalkyl, Niederalkoxy,

Niederalkylthio, Niederalkylamino, Diniederalkylamino, Halogen, Trifluormethyl oder gegebenenfalls substituiertes Phenyl, oder an einem beidseitig einfach gebundenen Kohlenstoffatom auch Oxo, $R_5$ Wasserstoff oder Niederalkyl, Het einen Heteroalkylenrest mit 3 Kettengliedern, der die Gruppe C-N zu einem gegebenenfalls ungesättigten heterocyclischen Fünfring mit 2 bis 3 Heteroatomen aus der Gruppe Sauerstoff, Schwefel oder Stickstoff im Ring ergänzt, und Ph einen gegebenenfalls substituierten Phenylrest bedeuten, ihre tautomeren Verbindungen und Salze, dadurch gekennzeichnet, dass man,

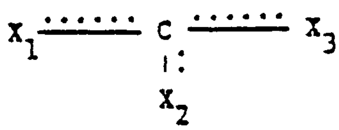 (II)

worin $X_1$ die Gruppe Ph-N=, in der Ph einen gegebenenfalls substituierten Phenylrest bedeutet, oder eine abspaltbare Gruppe, $X_2$ die Gruppe

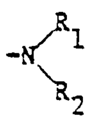 , worin $R_1$ und $R_2$ die unter der Formel I angegebene Bedeutung

haben, oder eine abspaltbare Gruppe und $X_3$ die Gruppe

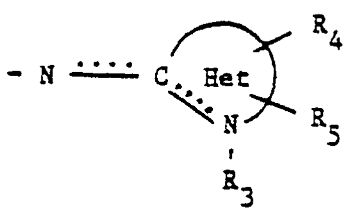 ,

worin $R_3$, $R_4$, $R_5$ und Het als zweiwertiger Heteroalkylenrest mit der Gruppe C-N die unter der Formel I angegebenen Bedeutungen haben, oder eine abspaltbare Gruppe bedeutet, mit der Massgabe, dass nur einer der Substituenten $X_1$, $X_2$ oder $X_3$ eine abspaltbare Gruppe sein kann, und worin eine der Gruppen $X_1$, $X_2$ und $X_3$ durch eine Doppelbindung mit dem Kohlenstoffatom verbunden ist, mit einem Amin oder Imin umsetzt, welches mit der fehlenden Amino- oder Iminogruppe identisch ist, die unter $X_1$, $X_2$ oder $X_3$ definiert werden, um die abspaltbare Gruppe zu ersetzen, oder

worin $R_3$, $R_4$, $R_5$ und Het als zweiwertiger Heteroalkylenrest mit der Gruppe C-N die unter der Formel I angegebenen Bedeutungen haben, oder eine abspaltbare Gruppe bedeutet, mit der Massgabe, dass nur einer der Substituenten $X_1$, $X_2$ oder $X_3$ eine abspaltbare Gruppe sein kann, mit einem Amin oder Imin umsetzt, welches mit der fehlenden Amino- oder Iminogruppe identisch ist, die unter $X_1$, $X_2$ oder $X_3$ definiert werden, um die abspaltbare Gruppe zu ersetzen, oder

b) eine Guanidinverbindung der allgemeinen Formel IV

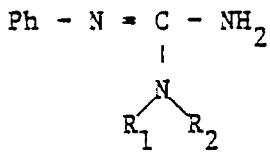

(IV)

worin Ph, $R_1$ und $R_2$ die unter der Formel I angegebenen Bedeutungen haben, mit einer Verbindung der allgemeinen Formel V

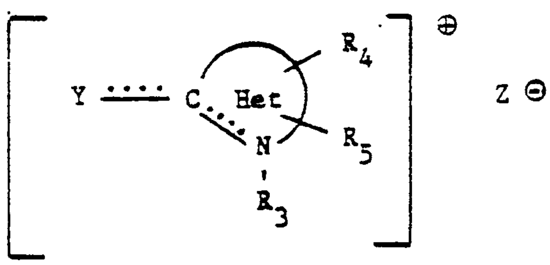

worin Y Niederalkoxy, wie z.B. Methoxy oder Aethoxy, Niederalkylthio, wie z.B. Methylthio oder Aethylthio, Halogen, wie z.B. Chlor oder Brom, oder Y zwei Niederalkoxygruppen, die am gleichen C-Atom sitzen, und Z ein Tetrafluoroborat-, ein Fluorsulfonat-, ein Niederalkylsulfat-, wie z.B. Methylsulfat- oder Alkansulfonat-, wie z.B. Methansulfonatanion oder ein Halogenid, wie z.B. Chlorid oder Bromid, bedeutet, wobei, wenn Y die Bedeutung von zwei Niederalkoxygruppen am gleichen C-Atom hat, Z als Anion entfällt, oder, wenn $R_3$ Wasserstoff bedeutet, die tautomere Form als freie Base vorliegt, umsetzt, oder

c) Guanidinderivate der allgemeinen Formel VI

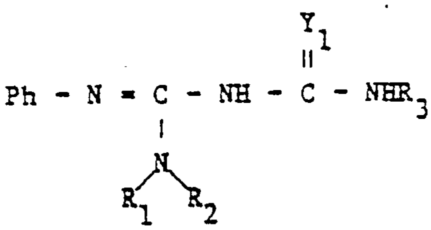

(VI)

oder eine tautomere Form davon, worin Ph, $R_1$, $R_2$ und $R_3$ die vorstehend genannten Bedeutungen haben und $Y_1$ die Bedeutung einer Oxo-, Thioxo- oder NH-Gruppe hat, mit einer Verbindung der allgemeinen Formel VII

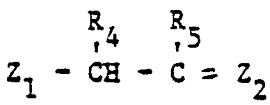

(VII)

umsetzt, worin $Z_1$ ein Halogenatom, $Z_2$ eine Oxogruppe oder zusammen die Gruppe bestehend aus Wasserstoff und Halogen, oder $Z_1$ und $Z_2$ zusammengenommen über eine Iminogruppe den bivalenten Alkylenrest

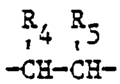

zu einem Aziridinderivat ergänzen, wobei $R_4$ und $R_5$ die Bedeutungen von Wasserstoff haben,

d) Verbindungen der Formel I, in der $R_1$ die oben definierte Bedeutung hat und $R_2$ und/oder $R_3$ Wasserstoff bedeuten, durch Umsetzung mit einem reaktionsfähigen Ester eines aliphatischen Alkohols, bespielsweise Niederalkanols in Verbindungen der Formel I umwandelt, in denen $R_2$ und/oder $R_3$ im Rahmen der vorstehenden Definitionen für $R_2$ und $R_3$ verschieden von Wasserstoff sind, oder

e) in Verbindungen der allgemeinen Formel VIII

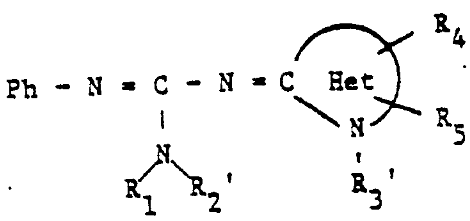

(VIII)

worin $R_1$, $R_4$, $R_5$, Ph und Het die unter der Formel I angegebene Bedeutung haben, und der eine von den Substituenten $R_2'$ und $R_3'$ die Bedeutung von $R_2$ oder $R_3$ hat, und der andere eine Aminoschutzgruppe bedeutet, oder beide $R_2'$ und $R_3'$ eine Aminoschutzgruppe bedeuten, diese abspaltet und gewünschtenfalls zusätzliche Verfahrensschritte durchführt, und/oder, wenn erwünscht, erhaltene Verbindungen der Formel I in ein Salz überführt, und/oder, wenn erwünscht, erhaltene Salze der Verbindungen der Formel I in die freien Basen umwandelt.

7. Verfahren nach Patentanspruch 6, dadurch gekennzeichnet, dass ein abspaltbarer Rest $X_1$, $X_2$ oder $X_3$ in den Verbindungen der Formel II eine Niederalkylthio-, Niederalkoxygruppe oder ein Halogen bedeutet.

8. Verfahren zur Herstellung von Guanidinderivaten der Formel

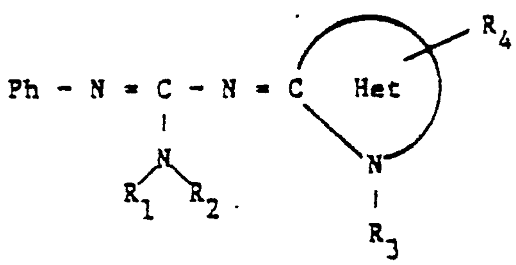

(I),

worin $R_1$ und $R_2$ einen gegebenenfalls substituierten aliphatischen oder cycloaliphatischen Kohlenwasserstoffrest bedeuten oder $R_1$ und $R_2$ zusammengenommen einen gegebenenfalls substituierten bivalenten Kohlenwasserstoffrest aliphatischen Charakters, in welchem die Kohlenstoffatome der Kette durch ein Heteroatom unterbrochen sein können, $R_3$ Wasserstoff, Niederalkyl, $R_4$ Wasserstoff, Niederalkyl, Niederalkoxy, Niederalkylamino, Diniederalkylamino, Halogen, Niederalkoxycarbonyl oder gegebenenfalls substituiertes Phenyl, oder an einem beidseitig einfach gebundenen Kohlenstoffatom auch Oxo, Het einen Heteroalkylenrest mit 4 bis 6 Kettengliedern, der die Gruppe C-N zu einem heterocyclischen Sechs- bis Achtring, der neben dem Stickstoffatom noch ein Heteroatom aus der Gruppe Sauerstoff, Schwefel oder Stickstoff im Ring enthält, ergänzt, und Ph einen gegebenenfalls substituierten Phenylrest bedeuten, ihre tautomeren Verbindungen

und Salze, dadurch gekennzeichnet, dass man

a) eine Verbindung der Formel

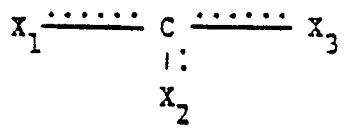

(II)

worin $X_1$ die Gruppe Ph-N=, in der Ph einen gegebenenfalls substituierten Phenylrest bedeutet, oder eine abspaltbare Gruppe, $X_2$ die Gruppe

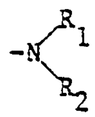

, worin $R_1$ und $R_2$ die unter der Formel I angegebene Bedeutung haben, oder eine abspaltbare Gruppe und $X_3$ die Gruppe

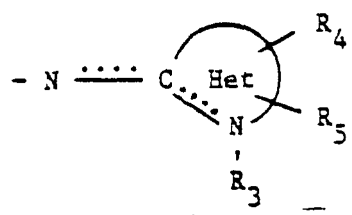

worin $R_3$, $R_4$, $R_5$ und Het als zweiwertiger Heteroalkylenrest mit der Gruppe C-N die unter der Formel I angegebenen Bedeutungen haben, oder eine abspaltbare Gruppe bedeutet, mit der Massgabe, dass nur einer der Substituenten $X_1$, $X_2$ oder $X_3$ eine abspaltbare Gruppe sein kann und worin eine der Gruppen $X_1$, $X_2$ und $X_3$ durch eine Doppelbindung mit dem Kohlenstoffatom verbunden ist, mit einem Amin oder Imin umsetzt, welches mit der fehlenden Amino- oder Iminogruppe identisch ist, die unter $X_1$, $X_2$ oder $X_3$ definiert werden, um die abspaltbare Gruppe zu ersetzen, oder

b) eine Guanidinverbindung der allgemeinen Formel IV

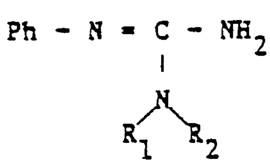

(IV)

worin Ph, $R_1$ und $R_2$ die unter der Formel I angegebenen Bedeutungen haben, mit einer Verbindung der allgemeinen Formel V

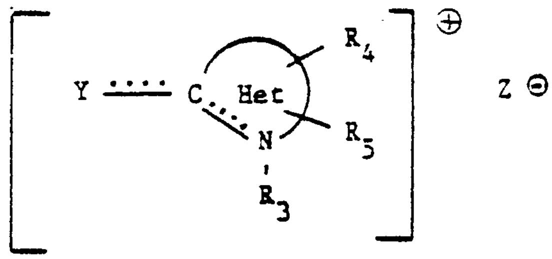

worin Y Niederalkoxy, Niederalkylthio, Halogen, oder Y zwei Niederalkoxygruppen, die am gleichen C-Atom sitzen, und Z ein Tetrafluoroborat-, ein Fluorsulfonat-, ein Niederalkylsulfat-, wie z.B. Methylsulfat- oder Niederalkansulfonat-, wie z.B. Methansulfonatanion oder ein Halogenid, wie z.B. Chlorid oder Bromid, bedeutet, wobei, wenn Y die Bedeutung von zwei Niederalkoxygruppen am gleichen C-Atom hat, Z als Anion entfällt, oder wenn $R_3$ Wasserstoff bedeutet, die tautomere Form als freie Base vorliegt, umsetzt, oder

c) Guanidinderivate der allgemeinen Formel VI

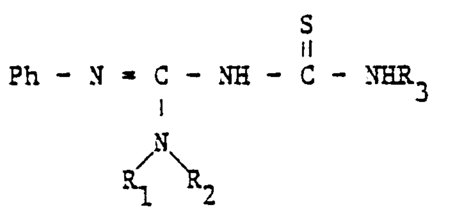

(VI)

oder eine tautomere Form davon, worin Ph, $R_1$, $R_2$ und $R_3$ die vorstehend genannten Bedeutungen haben mit einer Verbindung der allgemeinen Formel VII

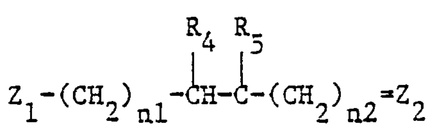

(VII)

umsetzt, worin $Z_1$ ein Halogenatom und $Z_2$ ein Halogenatom und ein Wasserstoffatom bedeuten, und $n_1$ und $n_2$ die unter der Formel VII im Anspruch 1 angegebenen Bedeutungen, $R_5$ die Bedeutung von Wasserstoff hat und $R_4$ die unter der Formel I vorher angegebene Bedeutung hat, ersetzt sein kann, oder

d) in dem man eine Verbindung der Formel IV

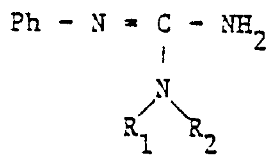

(IV)

worin $R_1$, $R_2$ und Ph die oben angegebenen Bedeutungen haben, mit einem Halogenalkylenisothiocyanat der Formel

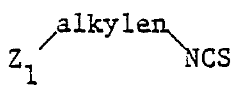

(VIII) ,

worin $Z_1$ ein Halogenatom bedeutet, und der bivalente Rest "alkylen" eine Alkylenkette mit 3-5 Kohlenstoffatomen bedeutet, wobei ein Wasserstoff in der Alkylenkette durch den Substituenten $R_4'$, der die oben angegebene Bedeutung für $R_4$ mit Ausnahme von Wasserstoff hat, ersetzt sein kann, umsetzt, oder

e) Verbindungen der Formel I, in der $R_1$ und $R_2$ die oben definierten Bedeutungen haben und $R_3$ Wasserstoff bedeuten, durch Umsetzung mit einem reaktionsfähigen Ester eines Niederalkanols in Verbindungen der Formel I umwandelt, in denen $R_3$ im Rahmen der vorstehenden Definitionen für $R_3$ verschieden von Wasserstoff ist, oder

f) in Verbindungen der allgemeinen Formel IX

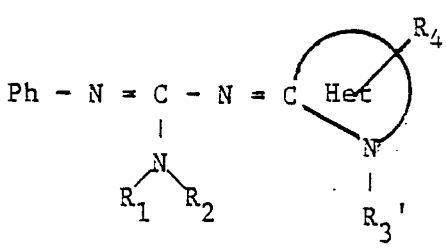

(IX)

worin $R_1$, $R_4$, Ph und Het die unter der Formel I angegebene Bedeutung haben, und der Substituent $R'_3$ eine Aminoschutzgruppe bedeutet, diese abspaltet und gewünschtenfalls zusätzliche Verfahrensschritte durchführt, und/oder, wenn erwünscht, erhaltene Verbindungen der Formel I in ein Salz überführt, und/oder, wenn erwünscht, erhaltene Salze der Verbindungen der Formel I in die freien Basen umwandelt.

9. Verfahren nach Patentanspruch 8, dadurch gekennzeichnet, dass ein abspaltbarer Rest $X_1$, $X_2$ und $X_3$ in den Verbindungen der Formeln II eine Niederalkylthio-, Niederalkoxygruppe oder ein Halogen bedeutet.

10. Verfahren nach Patentansprüchen 6 und 7, dadurch gekennzeichnet, dass man Verbindungen der Formel I

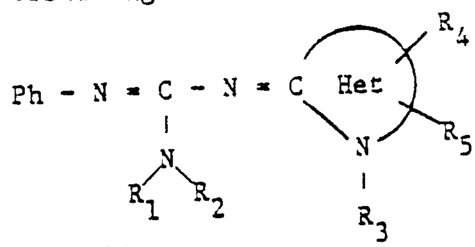

(I)

worin $R_1$ ein gegebenenfalls substituierter aliphatischer oder cycloaliphatischer Kohlenwasserstoffrest, $R_2$ Wasserstoff oder ein gegebenenfalls substituierter aliphatischer Kohlenwasserstoffrest ist, oder $R_1$ und $R_2$ zusammengenommen einen gegebenenfalls substituierten bivalenten Kohlenwasserstoffrest aliphatischen Charakters, in welchem die Kohlenstoffatome der Kette durch ein Heteroatom unterbrochen sein können, $R_3$ Wasserstoff, Niederalkyl, $R_4$ Wasserstoff, Niederalkyl, Niederalkoxy, Niederalkylthio, Niederalkylamino, Diniederalkylamino, Halogen, Trifluormethyl oder gegebenenfalls substituiertes Phenyl, oder an einem beidseitig einfach gebundenen Kohlenstoffatom auch Oxo, $R_5$ Wasserstoff oder Niederalkyl, Het einen Heteroalkylenrest mit 3 Kettengliedern, der die Gruppe C-N zu einem gegebenenfalls ungesättigten heterocyclischen Fünfring mit 2 bis 3 Heteroatomen aus der Gruppe Sauerstoff, Schwefel oder Stickstoff im Ring ergänzt, und Ph einen gegebenenfalls substituierten Phenylrest bedeuten, ihre tautomeren Verbindungen und Salze, herstellt.

11. Verfahren nach Patentansprüchen 6 und 7, dadurch gekennzeichnet, dass man Verbindungen der Formel I, in der $R_1$ Niederalkyl oder Cycloalkyl, $R_2$ Wasserstoff oder Niederalkyl oder beide zusammengenommen eine Niederalkylenkette, die gegebenenfalls durch ein Sauerstoff-, Schwefel- oder gegebenenfalls durch z.B. Niederalkyl substituiertes Stickstoffatom unterbrochen sein kann, $R_3$ Wasserstoff oder Niederalkyl, $R_4$ Wasserstoff, Niederalkyl, Niederalkoxy, Niederalkylthio, Niederalkylamino, Diniederalkylamino, Halogen, Trifluormethyl, oder an einem beidseitig einfach gebundenen Kohlenstoffatom auch Oxo, oder gegebenenfalls durch Niederalkyl, Niederalkoxy oder Halogen substituiertes Phenyl, $R_5$ Wasserstoff oder Niederalkyl, und Het einen Heteroalkylenrest mit 3 Kettengliedern, der die Gruppe C-N zu einem gesättigten oder einfach gesättigten heterocyclischen Fünfring mit 2-3 Heteroatomen aus der Gruppe Sauerstoff, Schwefel oder Stickstoff im Ring ergänzt, und Ph einen gegebenenfalls substituierten Phenylrest bedeuten, als auch ihre tautomeren Verbindungen und Salze, herstellt.

12. Verfahren nach Patentansprüchen 6 und 7, dadurch gekennzeichnet, dass man Verbindungen der Formel I, in der $R_1$ Niederalkyl oder Cycloalkyl, $R_2$ Wasserstoff oder Niederalkyl oder beide zusammengenommen eine Niederalkylenkette, die gegebenenfalls durch ein Sauerstoff- oder Schwefelatom oder gegebenenfalls durch z.B. Niederalkyl substituiertes Stickstoffatom unterbrochen sein kann, z.B. Niederalkylenamino, z.B. Pyrrolidino, 2,5-Dimethylpyrrolidino, Piperidino, 2-Methylpiperidino, Hexahydroazepino oder Octahydroazocino, Oxaniederalkylenamino, z.B. Morpholino, Thianiederalkylenamino, z.B. Thiomorpholino oder Azaniederalkylenamino, z.B. Piperazino, N-Methyl- oder N-Phenylpiperazino bedeuten, $R_3$ Wasserstoff oder Niederalkyl, $R_4$ Wasserstoff, Niederalkyl, Niederalkoxy, Niederalkylthio, Niederalkylamino, Diniederalkylamino, Halogen, Trifluormethyl, oder an einem beidseitig einfach gebundenen Kohlenstoffatom auch Oxo, oder gegebenenfalls durch Niederalkyl, Niederalkoxy oder Halogen substituiertes Phenyl, $R_5$ Wasserstoff oder Niederalkyl, und Het einen zweiwertigen Heteroalkylenrest mit 3 Kettengliedern, der die Gruppe C-N zu einem gesättigten oder einfach ungesättigten heterocyclischen Fünfring mit 2-3 Heteroatomen im Ring ergänzt und beispielsweise ein Imidazolin-, Imidazolidin-, Oxazolin-, Oxazolidin-, Thiazolin-, Thiazolidin-, Isoxazolin-, Isoxazolidin-, Isothiazolin-, Isothiazolidin-, Oxadiazolin-, Oxadiazolidin-, Thiadiazolin-, Thiadiazolidin-, Pyrazolin-, Pyrazolidin- oder auch Triazolinring sein kann, und Ph einen gegebenenfalls durch Niederalkyl, Niederalkoxy oder Halogen substituierten Phenylrest bedeuten, als auch ihre tautomeren Verbindungen und Salze, herstellt.

13. Verfahren nach Patentansprüchen 6 und 7, dadurch gekennzeichnet, dass man Verbindungen der Formel I, in der $R_1$ Niederalkyl oder Cycloalkyl, $R_2$ Wasserstoff oder Niederalkyl oder die Gruppe $-NR_1R_2$ beispielsweise Niederalkylenamino, in der die Niederalkylenkette gegebenenfalls durch ein Sauerstoff- oder Schwefelatom unterbrochen sein kann, bedeutet und beispielsweise Pyrrolidino, 2,5-Dimethylpyrrolidino, Piperidino, 2-Methylpiperidino, Morpholino oder Thiomorpholino sein kann, $R_3$ Wasserstoff oder Niederalkyl, $R_4$ Wasserstoff, Niederalkyl, Niederalkoxy, Niederalkylthio, Halogen, Trifluormethyl oder an einem beidseitig einfach gebundenen Kohlenstoffatom auch Oxo, oder gegebenenfalls durch Niederalkyl, Niederalkoxy oder Halogen substituiertes Phenyl, $R_5$ Wasserstoff oder Niederalkyl, und Het als zweiwertiger Heteroalkylenrest mit der Gruppe C-N zusammen einen gegebenenfalls einfach ungesättigten heterocyclischen Fünfring, wie z.B. Imidazolin-, Imidazolidin-, Oxazolin-, Oxazolidin-, Thiazolin-, Thiazolidin-, Oxadiazolin-, Oxadiazolidin-, Triazolin-, Thiadiazolin- oder Thiadiazolidinring bedeutet, und Ph einen gegebenenfalls durch Niederalkyl, Niederalkoxy oder Halogen substituierten Phenylrest bedeutet, als auch ihre tautomeren Verbindungen und Salze, herstellt.

14. Verfahren nach Patentansprüchen 6 und 7, dadurch gekennzeichnet, dass man Verbindungen der Formel I, in der $R_1$ Niederalkyl, z.B. Methyl oder Aethyl, $R_2$ Wasserstoff oder Niederalkyl, z.B. Methyl oder Aethyl bedeutet oder die Gruppe $-NR_1R_2$ als gegebenenfalls durch ein Sauerstoff- oder Schwefelatom unterbrochenes Niederalkylenamino, beispielsweise als Pyrrolidino, Piperidino, Morpholini oder Thiomorpholino vorliegt, $R_3$ Wasserstoff oder Niederalkyl, z.B. Methyl oder Aethyl, $R_4$ Wasserstoff, Niederalkyl, z.B. Methyl oder Aethyl, Niederalkoxy, z.B. Methoxy oder Aethoxy, Niederalkylthio, z.B. Methylthio oder Aethylthio, Halogen, z.B. Chlor oder Brom, Trifluormethyl oder gegebenenfalls durch

Niederalkyl, z.B. Methyl oder Aethyl, Halogen, z.B. Chlor oder Brom substituiertes Phenyl, $R_5$ Wasserstoff oder Niederalkyl, z.B. Methyl oder Aethyl und Het als zweiwertiger Heteroalkylenrest mit der Gruppe C-N einen gegebenenfalls einfach ungesättigten heterocyclischen Fünfring, wie z.B. den 4-Imidazolin-, 4-Oxazolin-, 4-Thiazolin-, 4-(1,3,4)-Thiadiazolin-, 4-(1,3,4)-Oxadiazolin- oder auch Isoxazolidinring bedeutet, und Ph einen gegebenenfalls durch Niederalkyl, wie z.B. Methyl oder Aethyl, Halogen, wie z.B. Chlor oder Brom oder Niederalkoxy, wie z.B. Methoxy oder Aethoxy, substituierten Phenylrest bedeutet, als auch ihre tautomeren Verbindungen und Salze, herstellt.

15. Verfahren nach Patentansprüchen 6 und 7, dadurch gekennzeichnet, dass man das N-(2,5-Dimethyl-3-isoxazolidinyliden)-N'-phenyl-4-morpholin-carboximidamid als auch ihre tautomeren Verbindungen und Salze herstellt.

16. Verfahren nach Patentansprüchen 6 und 7, dadurch gekennzeichnet, dass man das N-[3-Methyl-4-(1,3,4)-thiadiazolin-2-yliden]-N'-phenyl-4-morpholin-carboximidamid-hydrochlorid als auch ihre tautomeren Verbindungen und Salze herstellt.

17. Verfahren nach Patentansprüchen 8 und 9, dadurch gekennzeichnet, dass man Verbindungen der Formel Ib

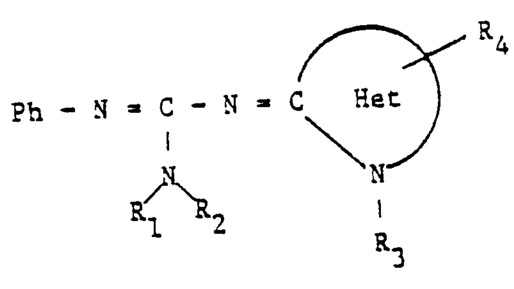

(Ib)

worin $R_1$ und $R_2$ einen gegebenenfalls substituierten aliphatischen oder cycloaliphatischen Kohlenwasserstoffrest bedeuten oder $R_1$ und $R_2$ zusammengenommen einen gegebenenfalls substituierten bivalenten Kohlenwasserstoffrest aliphatischen Charakters, in welchem die Kohlenstoffatome der Kette durch ein Heteroatom unterbrochen sein können, $R_3$ Wasserstoff, Niederalkyl, $R_4$ Wasserstoff, Niederalkyl, Niederalkoxy, Niederalkylamino, Diniederalkylamino, Halogen, Niederalkoxycarbonyl oder gegebenenfalls substituiertes Phenyl, oder an einem beidseitig einfach gebundenen Kohlenstoffatom auch Oxo, Het einen Heteroalkylenrest mit 4 bis 6 Kettengliedern, der die Gruppe C-N zu einem heterocyclischen Sechs- bis Achtring, der neben dem Stickstoffatom noch ein Heteroatom aus der Gruppe Sauerstoff, Schwefel oder Stickstoff im Ring enthält, ergänzt, und Ph einen gegebenenfalls substituierten Phenylrest bedeuten, ihre tautomeren Verbindungen und Salze herstellt.

18. Verfahren nach Patentansprüchen 8 und 9, dadurch gekennzeichnet, dass man Verbindungen der Formel Ib, in der $R_1$ und $R_2$ Niederalkyl oder Cycloalkyl, oder beide zusammengenommen eine gerade, verzweigte oder substituierte Niederalkylenkette, die gegebenenfalls durch ein Sauerstoff-, Schwefel- oder gegebenenfalls durch z.B. Niederalkyl, Phenyl oder Niederalkoxycarbonyl substituiertes Stickstoffatom unterbrochen sein kann, $R_3$ Wasserstoff oder Niederalkyl, $R_4$ Wasserstoff, Niederalkyl, Niederalkoxy, Niederalkylamino, Diniederalkylamino, Halogen, Niederalkoxycarbonyl, oder an einem beidseitig einfach gebundenen Kohlenstoffatom auch Oxo, oder gegebenenfalls durch Niederalkyl, Niederalkoxy oder Halogen substituiertes Phenyl, und Het einen Heteroalkylenrest mit 4 bis 6 Kettengliedern, der die Gruppe C-N zu einem heterocyclischen Sechs- bis Achtring, der neben dem Stickstoffatom noch ein Heteroatom aus der Gruppe Sauerstoff, Schwefel oder Stickstoff im Ring enthält, ergänzt, und Ph einen gegebenenfalls substituierten Phenylrest bedeuten, als auch ihre tautomeren Verbindungen und Salze, herstellt.

19. Verfahren nach Patentansprüchen 8 und 9, dadurch gekennzeichnet, dass man Verbindungen der Formel Ib, in der $R_1$ und $R_2$ Niederalkyl oder Cycloalkyl, oder beide zusammengenommen eine gerade, verzweigte oder substituierte Niederalkylenkette, die gegebenenfalls durch ein Sauerstoff- oder Schwefelatom oder gegebenenfalls durch z.B. Niederalkyl, Phenyl oder Niederalkoxycarbonyl substituiertes Stickstoffatom unterbrochen sein kann, als Niederalkylenamino beispielsweise Pyrrolidino, 2,5-Dimethylpyrrolidino, Piperidino, 2-Methylpiperidino, 4-Phenylpiperidino, Hexahydroazepino oder Octahydroazocino, als Oxaniederalkylenamino beispielsweise Morpholino, 2,6-Dimethylmorpholino, als Thianiederalkylenamino beispielsweise Thiomorpholino oder als Azaniederalkylenamino, beispielsweise Piperazino, N-Niederalkoxycarbonyl, N-Methyl- oder N-Phenylpiperazino bedeuten, $R_3$ Wasserstoff oder Niederalkyl, $R_4$ Wasserstoff, Niederalkyl, Niederalkoxy, Niederalkylamino, Diniederalkylamino, Halogen, Niederalkoxycarbonyl, oder gegebenenfalls durch Niederalkyl, Niederalkoxy oder Halogen substituiertes Phenyl, und Het einen zweiwertigen Heteroalkylenrest mit 4 bis 5 Kettengliedern, der die Gruppe C-N zu einem heterocyclischen Sechs- bis Siebenring, der neben dem Stickstoffatom noch ein Heteroatom aus der Gruppe Sauerstoff, Schwefel oder Stickstoff, im Ring enthält, ergänzt, und Ph einen gegebenenfalls durch Niederalkyl, Niederalkoxy oder Halogen substituierten Phenylrest bedeuten als auch ihre tautomeren Verbindungen und Salze, herstellt.

20. Verfahren nach Patentansprüchen 8 und 9, dadurch gekennzeichnet, dass man Verbindungen der Formel Ib, in der $R_1$ und $R_2$ Niederalkyl oder die Gruppe $-NR_1R_2$ gemeinsam ein Niederalkylamino, in der die gerade, verzweigte oder durch Phenyl substituierte Niederalkylenkette gegebenenfalls durch ein Sauerstoff-, Schwefel- oder gegebenenfalls

durch ein Sauerstoff-, Schwefel- oder gegebenenfalls durch z.B. Niederalkyl, Phenyl oder Niederalkoxycarbonyl substituiertes Stickstoffatom unterbrochen sein kann, bedeutet und beispielsweise als Niederalkylenamino Pyrrolidino, 2,5-Dimethylpyrrolidino, Piperidino, 2-Methylpiperidino, 4-Phenylpiperidino, Hexahydroazepino oder Octahydroazocino, als Oxaniederalkylenamino beispielsweise Morpholino, 2,6-Dimethylmorpholino, als Thianiederalkylenamino beispielsweise Thiomorpholino oder als Azaniederalkylenamino beispielsweise Piperazino, N-Aethoxycarbonyl-, N-Methyl-, N-Phenylpiperazino sein kann, $R_3$ Wasserstoff oder Niederalkyl, $R_4$ Wasserstoff, Niederalkyl, Niederalkoxy, Niederalkylamino, Halogen, Niederalkoxycarbonyl, oder gegebenenfalls durch Niederalkyl, Niederalkoxy oder Halogen substituiertes Phenyl und Het einen zweiwertigen Heteroalkylenrest mit 4 bis 5 Kettengliedern, der die Gruppe C-N zu einem heterocyclischen Sechs- bis Siebenring, der neben dem Stickstoffatom noch ein Heteroatom aus der Gruppe Sauerstoff, Schwefel oder Stickstoff im Ring enthält, ergänzt, und beispielsweise ein Morpholin-, Thiomorpholin-, 2H-3,4,5,6-Tetrahydro-1,3-thiazin-, 2H-3,4,5,6-Tetrahydropyrimidin-, Piperazin- oder auch Hexahydro-1,4-thiazepinring sein kann, und Ph einen gegebenenfalls durch Niederalkyl, Niederalkoxy, oder Halogen substituierten Phenylrest bedeutet, als auch ihre tautomeren Verbindungen und Salze, herstellt.

21. Verfahren nach Patentansprüchen 8 und 9, dadurch gekennzeichnet, dass man Verbindungen der Formel Ib, in der $R_1$ und $R_2$ Niederalkyl, z.B. Methyl oder Aethyl, oder die Gruppe $-NR_1R_2$ gemeinsam ein Niederalkylenamino, in der die gerade, verzweigte oder durch Phenyl substituierte Niederalkylenkette gegebenenfalls durch ein Sauerstoff-, Schwefel- oder gegebenenfalls durch Niederalkyl, wie z.B. Methyl oder Aethyl oder Niederalkoxycarbonyl, wie z.B. Methoxy- oder Aethoxycarbonyl substituiertes Stickstoffatom unterbrochen sein kann, bedeutet und beispielsweise Pyrrolidino, Piperidino, 4-Phenylpiperidino, Morpholino, 2,6-Dimethylmorpholino, Thiomorpholino, Piperazino, N-Aethoxycarbonylpiperazino sein kann, $R_3$ Wasserstoff oder Niederalkyl, z.B.Methyl oder Aethyl, $R_4$ Wasserstoff, Niederalkyl, z.B. Methyl oder Aethyl, oder Niederalkoxycarbonyl, z.B. Methoxy- oder Aethoxycarbonyl und Het

einen zweiwertigen Heteroalkylenrest, der mit der Gruppe C-N diese zu einem heterocyclischen Sechsring, der neben dem Stickstoffatom noch ein Heteroatom aus der Gruppe Sauerstoff, Schwefel oder Stickstoff im Ring enthält, ergänzt, und beispielsweise ein Morpholin-, Thiomorpholin-, oder auch Piperazinring sein kann, und Ph einen gegebenenfalls durch Niederalkyl, wie z.B. Methyl oder Aethyl, Halogen, wie z.B. Fluor, Chlor oder Brom oder Niederalkoxy, wie z.B. Methoxy oder Aethoxy, substituierten Phenylrest bedeutet, als auch ihre tautomeren Verbindungen und Salze, herstellt.

22. Verfahren nach Patentansprüchen 8 und 9, dadurch gekennzeichnet, dass man das N-(Thiomorpholin-3-yliden)-N'-phenyl-4-morpholincarboximidamid als auch ihre tautomeren Verbindungen und Salze herstellt.

23. Verfahren nach Patentansprüchen 8 und 9, dadurch gekennzeichnet, dass man das N-(Morpholin-3-yliden)-N'-phenyl-4-morpholincarboximidamid als auch ihre tautomeren Verbindungen und Salze herstellt.

24. Pharmazeutisch verwendbare Präparate enthaltend eine gemäss Anspruch 1 erhaltene Verbindung.

25. Pharmazeutisch verwendbare Präparate enthaltend eine der Verbindungen der Ansprüche 10-16.

26. Pharmazeutisch verwendbare Präparate enthaltend eine der Verbindungen der Ansprüche 17-23.

Europäisches Patentamt

# EUROPÄISCHER RECHERCHENBERICHT

0020304

Nummer der Anmeldung

EP 80 81 0172

| Kategorie | EINSCHLÄGIGE DOKUMENTE<br>Kennzeichnung des Dokuments mit Angabe, soweit erforderlich, der maßgeblichen Teile | betrifft Anspruch |
|---|---|---|
| | FR - M - 8382 (HOFFMANN-LA-ROCHE)<br>* Ansprüche *<br>-- | 1 |
| | BE - A - 852 565 (McNEIL LAB.)<br>* Ansprüche *<br>-- | 1 |
| | GB - A - 1 409 768 (DR. KARL THOMAE GmbH)<br>* Spalten 1,2 *<br>-- | 1 |
| A | DE - A - 2 539 381 (IN. FRANCAIS DU PETROL)<br>* Ansprüche *<br>----- | 1 |

KLASSIFIKATION DER ANMELDUNG (Int Cl. ³)

C 07 D 521/00
A 61 K 31/395//
(C 07 D 521/00
279/12
265/30
263/28
277/48
277/18
285/12
263/48
233/88
417/12
233/44
./.

RECHERCHIERTE SACHGEBIETE (Int. Cl. ³)

C 07 D 279/12
265/30
263/28
277/48
277/18
285/12
263/48
233/88
417/12
233/44
241/06
271/10
261/04
A 61 K 31/395

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 27-08-1980 | BRIGHENTI |

Europäisches Patentamt

# EUROPÄISCHER RECHERCHENBERICHT

0020304

Nummer der Anmeldung

EP 80 81 0172

-2-

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | | | C 07 D 241/06<br>271/10<br>261/04)<br>C 07 D 233/46<br>277/40<br>211/16<br>295/20 |
| | | | RECHERCHIERTE SACHGEBIETE (Int. Cl. 3) |